# EUROPEAN PATENT SPECIFICATION

(11) **EP 1 229 781 B1**
(45) Date of publication and mention of the grant of the patent: **02.01.2013**
(21) Application number: 00980417.0
(22) Date of filing: 14.11.2000
(51) Int. Cl.: C12N 15/82, C07K 14/415, A01H 5/00

(54) **Seed trait genes**
Saatmerkmal bestimmende Gene
Gènes de caractéristiques de semences de plantes

(30) Priority: 17.11.1999 US 166228 P; 17.04.2000 US 197899 P; 22.08.2000 US 227439 P
(43) Date of publication of application: 14.08.2002
(73) Proprietor: Mendel Biotechnology, Inc., Hayward, CA 94545 (US)
(72) Inventor: REUBER, Lynne, San Mateo, CA 94402 (US); CREELMAN, Robert, Castro Valley, CA 94546 (US); PILGRIM, Marsha, Phoenixville, PA 19460 (US); RIECHMANN, Jose Luis, Pasadena, CA 91101 (US); JIANG, Cai-Zhong, Fremont, CA 94555 (US); YU, Guo-Liang, Berkeley, CA 94705 (US); PINEDA, Omaira, Vero Beach, FL 32960 (US); HEARD, Jacqueline, San Mateo, CA 94402 (US)
(74) Representative: Brasnett, Adrian Hugh
(86) International application number: PCT/US2000/031457
(87) International publication number: WO 2001/035727

(56) References cited:
- EP-A- 0 803 572
- EP-A- 1 033 405
- WO-A-01/36598
- WO-A-98/07842
- WO-A-98/48007
- WO-A-99/24573
- WO-A-99/55840
- US-A- 5 939 601
- WANG ZHI-YONG ET AL: "A Myb-related transcription factor is involved in the phytochrome regulation of an Arabidopsis Lhcb gene" PLANT CELL, AMERICAN SOCIETY OF PLANT PHYSIOLOGISTS, ROCKVILLE, MD, US, vol. 9, no. 4, 1997, pages 491-507, XP002296200 ISSN: 1040-4651
- WANG Z-Y ET AL: "CONSTITUTIVE EXPRESSION OF THE CIRCADIAN CLOCK ASSOCIATED 1 (CCA1) GENE DISRUPTS CIRCADIAN RHYTHMS AND SUPPRESSES ITS OWN EXPRESSION" CELL, CELL PRESS, CAMBRIDGE, NA, US, vol. 93, no. 7, 1998, pages 1207-1217, XP000929262 ISSN: 0092-8674
- LI ZHONGSEN ET AL: "PEI1, an embryo-specific zinc finger protein gene required for heart-stage embryo formation in arabidopsis" PLANT CELL, vol. 10, no. 3, March 1998 (1998-03), pages 383-398, XP002302043 ISSN: 1040-4651
- SHEWMAKER C K ET AL: "Seed-specific overexpression of phytoene synthase: increase in carotenoids and other metabolic effects" PLANT JOURNAL, BLACKWELL SCIENTIFIC PUBLICATIONS, OXFORD, GB, vol. 20, no. 4, 1999, pages 401-412, XP002969263 ISSN: 0960-7412
- WADA T ET AL: "EPIDERMAL CELL DIFFERENTIATION IN ARABIDOPSIS DETERMINED BY A MYB HOMOLOG, CPC" SCIENCE, AMERICAN ASSOCIATION FOR THE ADVANCEMENT OF SCIENCE,, US, vol. 277, no. 5329, 1997, pages 1113-1116, XP000929264 ISSN: 0036-8075
- DATABASE EMBL [Online] 24 July 1997 (1997-07-24), "Arabidopsis thaliana chromosome 2 clone T9D9 map m283, complete sequence." XP002312931 retrieved from EBI accession no. EM_PRO:AC002338 Database accession no. AC002338
- DATABASE EMBL [Online] 9 September 1999 (1999-09-09), "701556714 A. thaliana, Columbia Col-0, rosette-3 Arabidopsis thaliana cDNA clone 701556714, mRNA sequence." XP002312932 retrieved from EBI accession no. EM_PRO:AI999616 Database accession no. AI999616
- MA Q-H ET AL: "SEED-SPECIFIC EXPRESSION OF THE ISOPENTENYL TRANSFERASE GENE (IPT) IN TRANSGENIC TOBACCO" AUSTRALIAN JOURNAL OF PLANT PHYSIOLOGY, CSIRO, MELBOURNE, AU, vol. 25, no. 1, 1998, pages 53-59, XP000938791 ISSN: 0310-7841
- LI S.F. ET AL.: 'A novel MYB-related gene from arabidopsis thaliana' FEBS LETTERS vol. 379, 1996, pages 117 - 121, XP002938163
- SCHAFFER R. ET AL.: 'The late elongated hypocotyl mutation of arabidopsis disrupts circadian rhythms and the photoperiodic control of flowering' CELL vol. 93, 1998, pages 1219 - 1229, XP002938164
- DATABASE NCBI NUCLEOTIDE [Online] U.S. NATIONAL LIBRARY OF MEDICINE, (BETHEDSA, MD, USA) 14 January 1997 WANG Z.: 'Direct submission, sequence', XP002938165 Database accession no. U28422
- SUZUKI A. ET AL.: 'Cloning and expression of five MYB-related genes from rice seed' GENE vol. 198, 1997, pages 393 - 398, XP002938166
- LOGUERCIO L.L. ET AL.: 'Differential regulation of six novel MYB-domain genes defines tow distinct expression in allotraploid cotton (gossypium hitsutum L.)' MOL. GEN. GENET. vol. 261, 1999, pages 660 - 671, XP002938167
- KIRIK V. ET AL.: 'Two novel MYB homologues with changed expression in late embryogenesis-defective arabidopsis mutants' PLANT MOL. BIOL. vol. 37, 1998, pages 819 - 827, XP002938168

### SEQUENCE LISTING

<110> Reuber, Lynne
   Creelman, Robert
   Pilgrim, Marsha
   Riechmann, Jose Luis
   Jiang, Cai-zhong
   Yu, Guo-Liang
   Pineda, Omaira
   Heard, Jacqueline
<120> Seed Trait Genes
<130> MBI-0017
<150> 60/166,228
   <151> 1999-11-17
<150> 60/197,899
   <151> 2000-04-17
<150> Plant Trait Modification III,
   <151> 2000-08-22
<160> 54
<170> PatentIn version 3.0
<210> 1
   <211> 2240
   <212> DNA
   <213> Arabidopsis thaliana
<220>
   <221> CDS
   <222> (238)..(2064)
   <223> G214
<400> 1
   tgagatttct ccatttccgt agcttctggt ctcttttctt tgtttcattg atcaaaagca     60
   aatcacttct tcttcttctt cttctcgatt tcttactgtt ttcttatcca acgaaatctg    120
   gaattaaaaa tggaatcttt atcgaatcca agctgatttt gtttctttca ttgaatcatc    180
   tctctaaagt ggaattttgt aaagagaaga tctgaagttg tgtagaggag cttagtg       237
   atg gag aca aat tcg tct gga gaa gat ctg gtt att aag act cgg aag      285
   Met Glu Thr Asn Ser Ser Gly Glu Asp Leu Val Ile Lys Thr Arg Lys
   1               5                   10                  15
   cca tat acg ata aca aag caa cgt gaa agg tgg act gag gaa gaa cat      333
   Pro Tyr Thr Ile Thr Lys Gln Arg Glu Arg Trp Thr Glu Glu Glu His
               20                  25                  30
   aat aga ttc att gaa gct ttg agg ctt tat ggt aga gca tgg cag aag      381
   Asn Arg Phe Ile Glu Ala Leu Arg Leu Tyr Gly Arg Ala Trp Gln Lys
           35                  40                  45
   att gaa gaa cat gta gca aca aaa act gct gtc cag ata aga agt cac      429
   Ile Glu Glu His Val Ala Thr Lys Thr Ala Val Gln Ile Arg Ser His
       50                  55                  60
   gct cag aaa ttt ttc tcc aag gta gag aaa gag gct gaa gct aaa ggt      477
   Ala Gln Lys Phe Phe Ser Lys Val Glu Lys Glu Ala Glu Ala Lys Gly
   65                  70                  75                  80
   gta gct atg ggt caa gcg cta gac ata gct att cct cct cca cgg cct      525
   Val Ala Met Gly Gln Ala Leu Asp Ile Ala Ile Pro Pro Pro Arg Pro
                   85                  90                  95
   aag cgt aaa cca aac aat cct tat cct cga aag acg gga agt gga acg      573
   Lys Arg Lys Pro Asn Asn Pro Tyr Pro Arg Lys Thr Gly Ser Gly Thr
               100                 105                 110
   atc ctt atg tca aaa acg ggt gtg aat gat gga aaa gag tcc ctt gga      621
   Ile Leu Met Ser Lys Thr Gly Val Asn Asp Gly Lys Glu Ser Leu Gly
           115                 120                 125
   tca gaa aaa gtg tcg cat cct gag atg gcc aat gaa gat cga caa caa      669
   Ser Glu Lys Val Ser His Pro Glu Met Ala Asn Glu Asp Arg Gln Gln
       130                 135                 140
   tca aag cct gaa gag aaa act ctg cag gaa gac aac tgt tca gat tgt      717
   Ser Lys Pro Glu Glu Lys Thr Leu Gln Glu Asp Asn Cys Ser Asp Cys
   145                 150                 155                 160
   ttc act cat cag tat ctc tct gct gca tcc tcc atg aat aaa agt tgt      765
   Phe Thr His Gln Tyr Leu Ser Ala Ala Ser Ser Met Asn Lys Ser Cys
                   165                 170                 175
   ata gag aca tca aac gca agc act ttc cgc gag ttc ttg cct tca cgg      813
   Ile Glu Thr Ser Asn Ala Ser Thr Phe Arg Glu Phe Leu Pro Ser Arg
               180                 185                 190
   gaa gag gga agt cag aat aac agg gta aga aag gag tca aac tca gat      861
   Glu Glu Gly Ser Gln Asn Asn Arg Val Arg Lys Glu Ser Asn Ser Asp
           195                 200                 205
   ttg aat gca aaa tct ctg gaa aac ggt aat gag caa gga cct cag act      909
   Leu Asn Ala Lys Ser Leu Glu Asn Gly Asn Glu Gln Gly Pro Gln Thr
       210                 215                 220
   tat ccg atg cat atc cct gtg cta gtg cca ttg ggg agc tca ata aca      957
   Tyr Pro Met His Ile Pro Val Leu Val Pro Leu Gly Ser Ser Ile Thr
   225                 230                 235                 240
   agt tct cta tca cat cct cct tca gag cca gat agt cat ccc cac aca     1005
   Ser Ser Leu Ser His Pro Pro Ser Glu Pro Asp Ser His Pro His Thr
                   245                 250                 255
   gtt gca gga gat tat cag tcg ttt cct aat cat ata atg tca acc ctt     1053
   Val Ala Gly Asp Tyr Gln Ser Phe Pro Asn His Ile Met Ser Thr Leu
               260                 265                 270
   tta caa aca ccg gct ctt tat act gcc gca act ttc gcc tca tca ttt     1101
   Leu Gln Thr Pro Ala Leu Tyr Thr Ala Ala Thr Phe Ala Ser Ser Phe
           275                 280                 285
   tgg cct ccc gat tct agt ggt ggc tca cct gtt cca ggg aac tca cct     1149
   Trp Pro Pro Asp Ser Ser Gly Gly Ser Pro Val Pro Gly Asn Ser Pro
       290                 295                 300
   ccg aat ctg gct gcc atg gcc gca gcc act gtt gca gct gct agt gct     1197
   Pro Asn Leu Ala Ala Met Ala Ala Ala Thr Val Ala Ala Ala Ser Ala
   305                 310                 315                 320
   tgg tgg gct gcc aat gga tta tta cct tta tgt gct cct ctt agt tca     1245
   Trp Trp Ala Ala Asn Gly Leu Leu Pro Leu Cys Ala Pro Leu Ser Ser
                   325                 330                 335
   ggt ggt ttc act agt cat cct cca tct act ttt gga cca tca tgt gat     1293
   Gly Gly Phe Thr Ser His Pro Pro Ser Thr Phe Gly Pro Ser Cys Asp
               340                 345                 350
   gta gag tac aca aaa gca agc act tta caa cat ggt tct gtg cag agc     1341
   Val Glu Tyr Thr Lys Ala Ser Thr Leu Gln His Gly Ser Val Gln Ser
           355                 360                 365
   cga gag caa gaa cac tcc gag gca tca aag gct cga tct tca ctg gac     1389
   Arg Glu Gln Glu His Ser Glu Ala Ser Lys Ala Arg Ser Ser Leu Asp
       370                 375                 380
   tca gag gat gtt gaa aat aag agt aaa cca gtt tgt cat gag cag cct     1437
   Ser Glu Asp Val Glu Asn Lys Ser Lys Pro Val Cys His Glu Gln Pro
   385                 390                 395                 400
   tct gca aca cct gag agt gat gca aag ggt tca gat gga gca gga gac     1485
   Ser Ala Thr Pro Glu Ser Asp Ala Lys Gly Ser Asp Gly Ala Gly Asp
                       405                 410                 415
   aga aaa caa gtt gac cgg tcc tcg tgt ggc tca aac act ccg tcg agt      1533
   Arg Lys Gln Val Asp Arg Ser Ser Cys Gly Ser Asn Thr Pro Ser Ser
                   420                 425                 430
   agt gat gat gtt gag gcg gat gca tca gaa agg caa gag gat ggc acc      1581
   Ser Asp Asp Val Glu Ala Asp Ala Ser Glu Arg Gln Glu Asp Gly Thr
               435                 440                 445
   aat ggt gag gtg aaa gaa acg aat gaa gac act aat aaa cct caa act      1629
   Asn Gly Glu Val Lys Glu Thr Asn Glu Asp Thr Asn Lys Pro Gln Thr
       450                 455                 460
   tca gag tcc aat gca cgc cgc agt aga atc agc tcc aat ata acc gat      1677
   Ser Glu Ser Asn Ala Arg Arg Ser Arg Ile Ser Ser Asn Ile Thr Asp
   465                 470                 475                 480
   cca tgg aag tct gtg tct gac gag ggt cga att gcc ttc caa gct ctc      1725
   Pro Trp Lys Ser Val Ser Asp Glu Gly Arg Ile Ala Phe Gln Ala Leu
                   485                 490                 495
   ttc tcc aga gag gta ttg ccg caa agt ttt aca tat cga gaa gaa cac      1773
   Phe Ser Arg Glu Val Leu Pro Gln Ser Phe Thr Tyr Arg Glu Glu His
               500                 505                 510
   aga gag gaa gaa caa caa caa caa gaa caa aga tat cca atg gca ctt      1821
   Arg Glu Glu Glu Gln Gln Gln Gln Glu Gln Arg Tyr Pro Met Ala Leu
           515                 520                 525
   gat ctt aac ttc aca gct cag tta aca cca gtt gat gat caa gag gag      1869
   Asp Leu Asn Phe Thr Ala Gln Leu Thr Pro Val Asp Asp Gln Glu Glu
       530                 535                 540
   aag aga aac aca gga ttt ctt gga atc gga tta gat gct tca aag cta      1917
   Lys Arg Asn Thr Gly Phe Leu Gly Ile Gly Leu Asp Ala Ser Lys Leu
   545                 550                 555                 560
   atg agt aga gga aga aca ggt ttt aaa cca tac aaa aga tgt tcc atg      1965
   Met Ser Arg Gly Arg Thr Gly Phe Lys Pro Tyr Lys Arg Cys Ser Met
                   565                 570                 575
   gaa gcc aaa gaa agt aga atc ctc aac aac aat cct atc att cat gtg      2013
   Glu Ala Lys Glu Ser Arg Ile Leu Asn Asn Asn Pro Ile Ile His Val
               580                 585                 590
   gaa cag aaa gat ccc aaa cgg atg cgg ttg gaa act caa gct tcc aca      2061
   Glu Gln Lys Asp Pro Lys Arg Met Arg Leu Glu Thr Gln Ala Ser Thr
           595                 600                 605
   tga gactctattt tcatctgatc tgttgtttgt actctgtttt taagttttca          2114
   agaccactgc tacattttct ttttcttttg aggcctttgt atttgtttcc ttgtccatag   2174
   tcttcctgta acatttgact ctgtattatt caacaaatca taaactgttt aatctttttt   2234
   tttcca                                                               2240
<210> 2
   <211> 608
   <212> PRT
   <213> Arabidopsis thaliana
<400> 2
   Met Glu Thr Asn Ser Ser Gly Glu Asp Leu Val Ile Lys Thr Arg Lys
   1 5 10 15
   Pro Tyr Thr Ile Thr Lys Gln Arg Glu Arg Trp Thr Glu Glu Glu His
   20 25 30
   Asn Arg Phe Ile Glu Ala Leu Arg Leu Tyr Gly Arg Ala Trp Gln Lys
   35 40 45
   Ile Glu Glu His Val Ala Thr Lys Thr Ala Val Gln Ile Arg Ser His
   50 55 60
   Ala Gln Lys Phe Phe Ser Lys Val Glu Lys Glu Ala Glu Ala Lys Gly
   65 70 75 80
   Val Ala Met Gly Gln Ala Leu Asp Ile Ala Ile Pro Pro Pro Arg Pro
   85 90 95
   Lys Arg Lys Pro Asn Asn Pro Tyr Pro Arg Lys Thr Gly Ser Gly Thr
   100 105 110
   Ile Leu Met Ser Lys Thr Gly Val Asn Asp Gly Lys Glu Ser Leu Gly
   115 120 125
   Ser Glu Lys Val Ser His Pro Glu Met Ala Asn Glu Asp Arg Gln Gln
   130 135 140
   Ser Lys Pro Glu Glu Lys Thr Leu Gln Glu Asp Asn Cys Ser Asp Cys
   145 150 155 160
   Phe Thr His Gln Tyr Leu Ser Ala Ala Ser Ser Met Asn Lys Ser Cys
   165 170 175
   Ile Glu Thr Ser Asn Ala Ser Thr Phe Arg Glu Phe Leu Pro Ser Arg
   180 185 190
   Glu Glu Gly Ser Gln Asn Asn Arg Val Arg Lys Glu Ser Asn Ser Asp
   195 200 205
   Leu Asn Ala Lys Ser Leu Glu Asn Gly Asn Glu Gln Gly Pro Gln Thr
   210 215 220
   Tyr Pro Met His Ile Pro Val Leu Val Pro Leu Gly Ser Ser Ile Thr
   225 230 235 240
   Ser Ser Leu Ser His Pro Pro Ser Glu Pro Asp Ser His Pro His Thr
   245 250 255
   Val Ala Gly Asp Tyr Gln Ser Phe Pro Asn His Ile Met Ser Thr Leu
   260 265 270
   Leu Gln Thr Pro Ala Leu Tyr Thr Ala Ala Thr Phe Ala Ser Ser Phe
   275 280 285
   Trp Pro Pro Asp Ser Ser Gly Gly Ser Pro Val Pro Gly Asn Ser Pro
   290 295 300
   Pro Asn Leu Ala Ala Met Ala Ala Ala Thr Val Ala Ala Ala Ser Ala
   305 310 315 320
   Trp Trp Ala Ala Asn Gly Leu Leu Pro Leu Cys Ala Pro Leu Ser Ser
   325 330 335
   Gly Gly Phe Thr Ser His Pro Pro Ser Thr Phe Gly Pro Ser Cys Asp
               340                 345                 350
   Val Glu Tyr Thr Lys Ala Ser Thr Leu Gln His Gly Ser Val Gln Ser
           355                 360                 365
   Arg Glu Gln Glu His Ser Glu Ala Ser Lys Ala Arg Ser Ser Leu Asp
       370                 375                 380
   Ser Glu Asp Val Glu Asn Lys Ser Lys Pro Val Cys His Glu Gln Pro
   385                 390                 395                 400
   Ser Ala Thr Pro Glu Ser Asp Ala Lys Gly Ser Asp Gly Ala Gly Asp
                   405                 410                 415
   Arg Lys Gln Val Asp Arg Ser Ser Cys Gly Ser Asn Thr Pro Ser Ser
               420                 425                 430
   Ser Asp Asp Val Glu Ala Asp Ala Ser Glu Arg Gln Glu Asp Gly Thr
           435                 440                 445
   Asn Gly Glu Val Lys Glu Thr Asn Glu Asp Thr Asn Lys Pro Gln Thr
       450                 455                 460
   Ser Glu Ser Asn Ala Arg Arg Ser Arg Ile Ser Ser Asn Ile Thr Asp
   465                 470                 475                 480
   Pro Trp Lys Ser Val Ser Asp Glu Gly Arg Ile Ala Phe Gln Ala Leu
                   485                 490                 495
   Phe Ser Arg Glu Val Leu Pro Gln Ser Phe Thr Tyr Arg Glu Glu His
               500                 505                 510
   Arg Glu Glu Glu Gln Gln Gln Gln Glu Gln Arg Tyr Pro Met Ala Leu
           515                 520                 525
   Asp Leu Asn Phe Thr Ala Gln Leu Thr Pro Val Asp Asp Gln Glu Glu
       530                 535                 540
   Lys Arg Asn Thr Gly Phe Leu Gly Ile Gly Leu Asp Ala Ser Lys Leu
   545                 550                 555                 560
   Met Ser Arg Gly Arg Thr Gly Phe Lys Pro Tyr Lys Arg Cys Ser Met
                   565                 570                 575
   Glu Ala Lys Glu Ser Arg Ile Leu Asn Asn Asn Pro Ile Ile His Val
               580                 585                 590
   Glu Gln Lys Asp Pro Lys Arg Met Arg Leu Glu Thr Gln Ala Ser Thr
           595                 600                 605
<210> 3
   <211> 407
   <212> DNA
   <213> Arabidopsis thaliana
<220>
   <221> CDS
   <222> (10)..(348)
   <223> G226
<400> 3
   ccagtagtt atg gat aat acc aac cgt ctt cgt ctt cgt cgc ggt ccc agt      51
             Met Asp Asn Thr Asn Arg Leu Arg Leu Arg Arg Gly Pro Ser
             1               5                   10
   ctt agg caa act aag ttc act cga tcc cga tat gac tct gaa gaa gtg        99
   Leu Arg Gln Thr Lys Phe Thr Arg Ser Arg Tyr Asp Ser Glu Glu Val
   15                  20                  25                  30
   agt agc atc gaa tgg gag ttt atc agt atg acc gaa caa gaa gaa gat       147
   Ser Ser Ile Glu Trp Glu Phe Ile Ser Met Thr Glu Gln Glu Glu Asp
                   35                  40                  45
   ctc atc tct cga atg tac aga ctt gtc ggt aat agg tgg gat tta ata       195
   Leu Ile Ser Arg Met Tyr Arg Leu Val Gly Asn Arg Trp Asp Leu Ile
               50                  55                  60
   gca gga aga gtc gta gga aga aag gca aat gag att gag aga tac tgg       243
   Ala Gly Arg Val Val Gly Arg Lys Ala Asn Glu Ile Glu Arg Tyr Trp
           65                  70                  75
   att atg aga aac tct gac tat ttt tct cac aaa cga cga cgt ctt aat       291
   Ile Met Arg Asn Ser Asp Tyr Phe Ser His Lys Arg Arg Arg Leu Asn
       80                  85                  90
   aat tct ccc ttt ttt tct act tct cct ctt aat ctc caa gaa aat cta       339
   Asn Ser Pro Phe Phe Ser Thr Ser Pro Leu Asn Leu Gln Glu Asn Leu
   95                  100                 105                 110
   aaa ttg taa agaaatcaaa ataaaagctt tcaatcataa aagtagaaca               388
   Lys Leu
   aatcttgaat gtcttctca                                                  407
<210> 4
   <211> 112
   <212> PRT
   <213> Arabidopsis thaliana
<400> 4
   Met Asp Asn Thr Asn Arg Leu Arg Leu Arg Arg Gly Pro Ser Leu Arg
   1               5                   10                  15
   Gln Thr Lys Phe Thr Arg Ser Arg Tyr Asp Ser Glu Glu Val Ser Ser
               20                  25                  30
   Ile Glu Trp Glu Phe Ile Ser Met Thr Glu Gln Glu Glu Asp Leu Ile
           35                  40                  45
   Ser Arg Met Tyr Arg Leu Val Gly Asn Arg Trp Asp Leu Ile Ala Gly
       50                  55                  60
   Arg Val Val Gly Arg Lys Ala Asn Glu Ile Glu Arg Tyr Trp Ile Met
   65                  70                  75                  80
   Arg Asn Ser Asp Tyr Phe Ser His Lys Arg Arg Arg Leu Asn Asn Ser
                   85                  90                  95
   Pro Phe Phe Ser Thr Ser Pro Leu Asn Leu Gln Glu Asn Leu Lys Leu
               100                 105                 110
<210> 5
   <211> 1209
   <212> DNA
   <213> Arabidopsis thaliana
<220>
   <221> CDS
   <222> (41)..(1156)
   <223> G229
<400> 5
   ttgtggtcag tggaataaac acatataacc gccggagaaa atg gga aga gcg cca       55
                                               Met Gly Arg Ala Pro
                                               1               5
   tgt tgc gag aag gtc ggt atc aag aga ggg cgg tgg acg gcg gag gag      103
   Cys Cys Glu Lys Val Gly Ile Lys Arg Gly Arg Trp Thr Ala Glu Glu
                   10                  15                  20
   gac cag att ctc tcc aac tac att caa tcc aat ggt gaa ggt tct tgg      151
   Asp Gln Ile Leu Ser Asn Tyr Ile Gln Ser Asn Gly Glu Gly Ser Trp
               25                  30                  35
   aga tct ctc ccc aaa aat gcc gga tta aaa agg tgt gga aag agc tgt      199
   Arg Ser Leu Pro Lys Asn Ala Gly Leu Lys Arg Cys Gly Lys Ser Cys
           40                  45                  50
   aga ttg aga tgg ata aac tat cta aga tca gac ctc aag cgt gga aac      247
   Arg Leu Arg Trp Ile Asn Tyr Leu Arg Ser Asp Leu Lys Arg Gly Asn
       55                  60                  65
   ata act cca gaa gaa gaa gaa ctc gtt gtt aaa ttg cat tcc act ttg      295
   Ile Thr Pro Glu Glu Glu Glu Leu Val Val Lys Leu His Ser Thr Leu
   70                  75                  80                  85
   gga aac agg tgg tca cta atc gcg ggt cat cta cca ggg aga aca gac      343
   Gly Asn Arg Trp Ser Leu Ile Ala Gly His Leu Pro Gly Arg Thr Asp
                   90                  95                  100
   aac gaa ata aaa aat tat tgg aac tct cat ctc agc cgt aaa ctc cac      391
   Asn Glu Ile Lys Asn Tyr Trp Asn Ser His Leu Ser Arg Lys Leu His
               105                 110                 115
   aac ttc att agg aag cca tcc atc tct caa gac gtc tcc gcc gta atc      439
   Asn Phe Ile Arg Lys Pro Ser Ile Ser Gln Asp Val Ser Ala Val Ile
           120                 125                 130
   atg gcg aac gct tct tca gcg cca ccg ccg ccg cag gca aaa cgc aga      487
   Met Ala Asn Ala Ser Ser Ala Pro Pro Pro Pro Gln Ala Lys Arg Arg
       135                 140                 145
   ctt ggg aga acg agt agg tcc gct atg aaa cca aaa atc cgc aga aca      535
   Leu Gly Arg Thr Ser Arg Ser Ala Met Lys Pro Lys Ile Arg Arg Thr
   150                 155                 160                 165
   aaa act cgt aaa acg aag aaa acg tct gca cca ccg gag cct aac gcc      583
   Lys Thr Arg Lys Thr Lys Lys Thr Ser Ala Pro Pro Glu Pro Asn Ala
                   170                 175                 180
   gat gta gct ggg gct gat aaa gaa gca tta atg gtg gag tca agt gga      631
   Asp Val Ala Gly Ala Asp Lys Glu Ala Leu Met Val Glu Ser Ser Gly
               185                 190                 195
   gcc gag gct gag cta gga cga cca tgt gac tac tat gga gat gat tgt      679
   Ala Glu Ala Glu Leu Gly Arg Pro Cys Asp Tyr Tyr Gly Asp Asp Cys
           200                 205                 210
   aac aaa aat ctc atg agc att aat ggc gat aat gga gtt tta acg ttt      727
   Asn Lys Asn Leu Met Ser Ile Asn Gly Asp Asn Gly Val Leu Thr Phe
       215                 220                 225
   gat gat gat atc atc gat ctt ttg ttg gac gag tca gat cct ggc cac      775
   Asp Asp Asp Ile Ile Asp Leu Leu Leu Asp Glu Ser Asp Pro Gly His
   230                 235                 240                 245
   ttg tac aca aac aca acg tgc ggt ggt ggt ggg gag ttg cat aac ata      823
   Leu Tyr Thr Asn Thr Thr Cys Gly Gly Gly Gly Glu Leu His Asn Ile
                   250                 255                 260
   aga gac tct gaa gga gcc aga ggg ttc tcg gat act tgg aac caa ggg      871
   Arg Asp Ser Glu Gly Ala Arg Gly Phe Ser Asp Thr Trp Asn Gln Gly
               265                 270                 275
   aat ctc gac tgt ctt ctt cag tct tgt cca tct gtg gag tcg ttt ctc      919
   Asn Leu Asp Cys Leu Leu Gln Ser Cys Pro Ser Val Glu Ser Phe Leu
           280                 285                 290
   aac tac gac cac caa gtt aac gac gcg tcg acg gat gag ttt atc gat      967
   Asn Tyr Asp His Gln Val Asn Asp Ala Ser Thr Asp Glu Phe Ile Asp
       295                 300                 305
   tgg gat tgt gtt tgg caa gaa ggt agt gat aat aat ctt tgg cat gag     1015
   Trp Asp Cys Val Trp Gln Glu Gly Ser Asp Asn Asn Leu Trp His Glu
   310                 315                 320                 325
   aaa gag aat ccc gac tca atg gtc tcg tgg ctt tta gac ggt gat gat     1063
   Lys Glu Asn Pro Asp Ser Met Val Ser Trp Leu Leu Asp Gly Asp Asp
                   330                 335                 340
   gag gcc acg atc ggg aat agt aat tgt gag aac ttt gga gaa ccg tta     1111
   Glu Ala Thr Ile Gly Asn Ser Asn Cys Glu Asn Phe Gly Glu Pro Leu
               345                 350                 355
   gat cat gac gac gaa agc gct ttg gtc gct tgg ctt ctg tca tga         1156
   Asp His Asp Asp Glu Ser Ala Leu Val Ala Trp Leu Leu Ser
           360                 365                 370
   tgatattgat tgatccgtta tgtaatcttt tttgtgcatt cacagtttga atc         1209
<210> 6
   <211> 371
   <212> PRT
   <213> Arabidopsis thaliana
<400> 6
   Met Gly Arg Ala Pro Cys Cys Glu Lys Val Gly Ile Lys Arg Gly Arg
   1               5                   10                  15
   Trp Thr Ala Glu Glu Asp Gln Ile Leu Ser Asn Tyr Ile Gln Ser Asn
               20                  25                  30
   Gly Glu Gly Ser Trp Arg Ser Leu Pro Lys Asn Ala Gly Leu Lys Arg
           35                  40                  45
   Cys Gly Lys Ser Cys Arg Leu Arg Trp Ile Asn Tyr Leu Arg Ser Asp
       50                  55                  60
   Leu Lys Arg Gly Asn Ile Thr Pro Glu Glu Glu Glu Leu Val Val Lys
   65                  70                  75                  80
   Leu His Ser Thr Leu Gly Asn Arg Trp Ser Leu Ile Ala Gly His Leu
                   85                  90                  95
   Pro Gly Arg Thr Asp Asn Glu Ile Lys Asn Tyr Trp Asn Ser His Leu
               100                 105                 110
   Ser Arg Lys Leu His Asn Phe Ile Arg Lys Pro Ser Ile Ser Gln Asp
           115                 120                 125
   Val Ser Ala Val Ile Met Ala Asn Ala Ser Ser Ala Pro Pro Pro Pro
       130                 135                 140
   Gln Ala Lys Arg Arg Leu Gly Arg Thr Ser Arg Ser Ala Met Lys Pro
   145                 150                 155                 160
   Lys Ile Arg Arg Thr Lys Thr Arg Lys Thr Lys Lys Thr Ser Ala Pro
                   165                 170                 175
   Pro Glu Pro Asn Ala Asp Val Ala Gly Ala Asp Lys Glu Ala Leu Met
               180                 185                 190
   Val Glu Ser Ser Gly Ala Glu Ala Glu Leu Gly Arg Pro Cys Asp Tyr
           195                 200                 205
   Tyr Gly Asp Asp Cys Asn Lys Asn Leu Met Ser Ile Asn Gly Asp Asn
       210                 215                 220
   Gly Val Leu Thr Phe Asp Asp Asp Ile Ile Asp Leu Leu Leu Asp Glu
   225                 230                 235                 240
   Ser Asp Pro Gly His Leu Tyr Thr Asn Thr Thr Cys Gly Gly Gly Gly
                   245                 250                 255
   Glu Leu His Asn Ile Arg Asp Ser Glu Gly Ala Arg Gly Phe Ser Asp
               260                 265                 270
   Thr Trp Asn Gln Gly Asn Leu Asp Cys Leu Leu Gln Ser Cys Pro Ser
           275                 280                 285
   Val Glu Ser Phe Leu Asn Tyr Asp His Gln Val Asn Asp Ala Ser Thr
       290                 295                 300
   Asp Glu Phe Ile Asp Trp Asp Cys Val Trp Gln Glu Gly Ser Asp Asn
   305                 310                 315                 320
   Asn Leu Trp His Glu Lys Glu Asn Pro Asp Ser Met Val Ser Trp Leu
                   325                 330                 335
   Leu Asp Gly Asp Asp Glu Ala Thr Ile Gly Asn Ser Asn Cys Glu Asn
               340                 345                 350
   Phe Gly Glu Pro Leu Asp His Asp Asp Glu Ser Ala Leu Val Ala Trp
           355                 360                 365
   Leu Leu Ser
       370
<210> 7
   <211> 1046
   <212> DNA
   <213> Arabidopsis thaliana
<220>
   <221> CDS
   <222> (46)..(867)
   <223> G241
<400> 7
   gaaaaacatt tcaacttctt ttatcagcaa tcacaaatca aagag atg gga aga gct      57
                                                     Met Gly Arg Ala
                                                     1
   cca tgc tgt gag aag atg ggg ttg aag aga gga cca tgg aca cct gaa       105
   Pro Cys Cys Glu Lys Met Gly Leu Lys Arg Gly Pro Trp Thr Pro Glu
   5                   10                  15                  20
   gaa gat caa atc ttg gtc tct ttt atc ctc aac cat gga cat agt aac       153
   Glu Asp Gln Ile Leu Val Ser Phe Ile Leu Asn His Gly His Ser Asn
                   25                  30                  35
   tgg cga gcc ctc cct aag caa gct ggt ctt ttg aga tgt gga aaa agc       201
   Trp Arg Ala Leu Pro Lys Gln Ala Gly Leu Leu Arg Cys Gly Lys Ser
               40                  45                  50
   tgt aga ctt agg tgg atg aac tat tta aag cct gat att aaa cgt ggc       249
   Cys Arg Leu Arg Trp Met Asn Tyr Leu Lys Pro Asp Ile Lys Arg Gly
           55                  60                  65
   aat ttc acc aaa gaa gag gaa gat gct atc atc agc tta cac caa ata       297
   Asn Phe Thr Lys Glu Glu Glu Asp Ala Ile Ile Ser Leu His Gln Ile
       70                  75                  80
   ctt ggc aat aga tgg tca gcg att gca gca aaa ctg cct gga aga acc       345
   Leu Gly Asn Arg Trp Ser Ala Ile Ala Ala Lys Leu Pro Gly Arg Thr
   85                  90                  95                  100
   gat aac gag atc aag aac gta tgg cac act cac ttg aag aag aga ctc       393
   Asp Asn Glu Ile Lys Asn Val Trp His Thr His Leu Lys Lys Arg Leu
                   105                 110                 115
   gaa gat tat caa cca gct aaa cct aag acc agc aac aaa aag aag ggt       441
   Glu Asp Tyr Gln Pro Ala Lys Pro Lys Thr Ser Asn Lys Lys Lys Gly
               120                 125                 130
   act aaa cca aaa tct gaa tcc gta ata acg agc tcg aac agt act aga       489
   Thr Lys Pro Lys Ser Glu Ser Val Ile Thr Ser Ser Asn Ser Thr Arg
           135                 140                 145
   agc gaa tcg gag cta gca gat tca tca aac cct tct gga gaa agc tta       537
   Ser Glu Ser Glu Leu Ala Asp Ser Ser Asn Pro Ser Gly Glu Ser Leu
       150                 155                 160
   ttt tcg aca tcg cct tcg aca agt gag gtt tct tcg atg aca ctc ata       585
   Phe Ser Thr Ser Pro Ser Thr Ser Glu Val Ser Ser Met Thr Leu Ile
   165                 170                 175                 180
   agc cac gac ggc tat agc aac gag att aat atg gat aac aaa ccg gga       633
   Ser His Asp Gly Tyr Ser Asn Glu Ile Asn Met Asp Asn Lys Pro Gly
                   185                 190                 195
   gat atc agt act atc gat caa gaa tgt gtt tct ttc gaa act ttt ggt       681
   Asp Ile Ser Thr Ile Asp Gln Glu Cys Val Ser Phe Glu Thr Phe Gly
               200                 205                 210
   gcg gat atc gat gaa agc ttc tgg aaa gag aca ctg tat agc caa gat       729
   Ala Asp Ile Asp Glu Ser Phe Trp Lys Glu Thr Leu Tyr Ser Gln Asp
           215                 220                 225
   gaa cac aac tac gta tcg aat gac cta gaa gtc gct ggt tta gtt gag       777
   Glu His Asn Tyr Val Ser Asn Asp Leu Glu Val Ala Gly Leu Val Glu
       230                 235                 240
   ata caa caa gag ttt caa aac ttg ggc tcc gct aat aat gag atg att       825
   Ile Gln Gln Glu Phe Gln Asn Leu Gly Ser Ala Asn Asn Glu Met Ile
   245                 250                 255                 260
   ttt gac agt gag atg gaa ctt ctg gtt cga tgt att ggc tag               867
   Phe Asp Ser Glu Met Glu Leu Leu Val Arg Cys Ile Gly
                   265                 270
   aaccggcggg gaacaagatc tcttagccgg gctctagtta acatgtttga ggagtaaagt     927
   gaaatggtgc aaattagtta aggctaagaa attcaaaagc ttttgtttac cgagaaaaaa 987
   acacactcta actcttgatg tgatgtagtt agtgtattaa ttagaggctg cgttttcaa 1046
<210> 8
   <211> 273
   <212> PRT
   <213> Arabidopsis thaliana
<400> 8
   Met Gly Arg Ala Pro Cys Cys Glu Lys Met Gly Leu Lys Arg Gly Pro
   1 5 10 15
   Trp Thr Pro Glu Glu Asp Gln Ile Leu Val Ser Phe Ile Leu Asn His
   20 25 30
   Gly His Ser Asn Trp Arg Ala Leu Pro Lys Gln Ala Gly Leu Leu Arg
   35 40 45
   Cys Gly Lys Ser Cys Arg Leu Arg Trp Met Asn Tyr Leu Lys Pro Asp
   50 55 60
   Ile Lys Arg Gly Asn Phe Thr Lys Glu Glu Glu Asp Ala Ile Ile Ser
   65 70 75 80
   Leu His Gln Ile Leu Gly Asn Arg Trp Ser Ala Ile Ala Ala Lys Leu
   85 90 95
   Pro Gly Arg Thr Asp Asn Glu Ile Lys Asn Val Trp His Thr His Leu
   100 105 110
   Lys Lys Arg Leu Glu Asp Tyr Gln Pro Ala Lys Pro Lys Thr Ser Asn
   115 120 125
   Lys Lys Lys Gly Thr Lys Pro Lys Ser Glu Ser Val Ile Thr Ser Ser
   130 135 140
   Asn Ser Thr Arg Ser Glu Ser Glu Leu Ala Asp Ser Ser Asn Pro Ser
   145 150 155 160
   Gly Glu Ser Leu Phe Ser Thr Ser Pro Ser Thr Ser Glu Val Ser Ser
   165 170 175
   Met Thr Leu Ile Ser His Asp Gly Tyr Ser Asn Glu Ile Asn Met Asp
   180 185 190
   Asn Lys Pro Gly Asp Ile Ser Thr Ile Asp Gln Glu Cys Val Ser Phe
   195 200 205
   Glu Thr Phe Gly Ala Asp Ile Asp Glu Ser Phe Trp Lys Glu Thr Leu
   210 215 220
   Tyr Ser Gln Asp Glu His Asn Tyr Val Ser Asn Asp Leu Glu Val Ala
   225 230 235 240
   Gly Leu Val Glu Ile Gln Gln Glu Phe Gln Asn Leu Gly Ser Ala Asn
   245 250 255
   Asn Glu Met Ile Phe Asp Ser Glu Met Glu Leu Leu Val Arg Cys Ile
   260 265 270
   Gly
<210> 9
   <211> 989
   <212> DNA
   <213> Arabidopsis thaliana
<220>
   <221> CDS
   <222> (41)..(664)
   <223> G464
<400> 9
   ctctgctggt atcattggag tctagggttt tgttattgac atg cgt ggt gtg tca      55
                                               Met Arg Gly Val Ser
                                               1               5
   gaa ttg gag gtg ggg aag agt aat ctt ccg gcg gag agt gag ctg gaa     103
   Glu Leu Glu Val Gly Lys Ser Asn Leu Pro Ala Glu Ser Glu Leu Glu
                   10                  15                  20
   ttg gga tta ggg ctc agc ctc ggt ggt ggc gcg tgg aaa gag cgt ggg     151
   Leu Gly Leu Gly Leu Ser Leu Gly Gly Gly Ala Trp Lys Glu Arg Gly
               25                  30                  35
   agg att ctt act gct aag gat ttt cct tcc gtt ggg tct aaa cgc tct     199
   Arg Ile Leu Thr Ala Lys Asp Phe Pro Ser Val Gly Ser Lys Arg Ser
           40                  45                  50
   gct gaa tct tcc tct cac caa gga gct tct cct cct cgt tca agt caa     247
   Ala Glu Ser Ser Ser His Gln Gly Ala Ser Pro Pro Arg Ser Ser Gln
       55                  60                  65
   gtg gta gga tgg cca cca att ggg tta cac agg atg aac agt ttg gtt     295
   Val Val Gly Trp Pro Pro Ile Gly Leu His Arg Met Asn Ser Leu Val
   70                  75                  80                  85
   aat aac caa gct atg aag gca gca aga gcg gaa gaa gga gac ggg gag     343
   Asn Asn Gln Ala Met Lys Ala Ala Arg Ala Glu Glu Gly Asp Gly Glu
                   90                  95                  100
   aag aaa gtt gtg aag aat ggt gag ctc aaa gat gtg tca atg aag gtg     391
   Lys Lys Val Val Lys Asn Gly Glu Leu Lys Asp Val Ser Met Lys Val
               105                 110                 115
   aat ccg aaa gtt cag ggc tta ggg ttt gtt aag gtg aat atg gat gga     439
   Asn Pro Lys Val Gln Gly Leu Gly Phe Val Lys Val Asn Met Asp Gly
           120                 125                 130
   gtt ggt ata ggc aga aaa gtg gat atg aga gct cat tcg tct tac gaa     487
   Val Gly Ile Gly Arg Lys Val Asp Met Arg Ala His Ser Ser Tyr Glu
       135                 140                 145
   aac ttg gct cag acg ctt gag gaa atg ttc ttt gga atg aca ggt act     535
   Asn Leu Ala Gln Thr Leu Glu Glu Met Phe Phe Gly Met Thr Gly Thr
   150                 155                 160                 165
   act tgt cga gaa acg gtt aaa cct tta agg ctt tta gat gga tca tca     583
   Thr Cys Arg Glu Thr Val Lys Pro Leu Arg Leu Leu Asp Gly Ser Ser
                   170                 175                 180
   gac ttt gta ctc act tat gaa gat aag ggg att gga tgc ttg ttg gag     631
   Asp Phe Val Leu Thr Tyr Glu Asp Lys Gly Ile Gly Cys Leu Leu Glu
               185                 190                 195
   atg ttc cat gga gaa tgt tta tca act cgg tga aaaggcttcg gatcatggga   684
   Met Phe His Gly Glu Cys Leu Ser Thr Arg
                   200                 205
   acctcagaag ctagtggact agctccaaga cgtcaagagc agaaggatag acaaagaaac     744
   aaccctgttt agcttccctt ccaaagctgg cattgtttat gtattgtttg aggtttgcaa     804
   tttactcgat actttttgaa gaaagtattt tggagaatat ggataaaagc atgcagaagc     864
   ttagatatga tttgaatccg gttttcggat atggttttgc ttaggtcatt caattcgtag     924
   ttttccagtt tgtttcttct ttggctgtgt accaattatc tatgttctgt gagagaaagc     984
   tcttg                                                                 989
<210> 10
   <211> 207
   <212> PRT
   <213> Arabidopsis thaliana
<400> 10
   Met Arg Gly Val Ser Glu Leu Glu Val Gly Lys Ser Asn Leu Pro Ala
   1               5                   10                  15
   Glu Ser Glu Leu Glu Leu Gly Leu Gly Leu Ser Leu Gly Gly Gly Ala
               20                  25                  30
   Trp Lys Glu Arg Gly Arg Ile Leu Thr Ala Lys Asp Phe Pro Ser Val
           35                  40                  45
   Gly Ser Lys Arg Ser Ala Glu Ser Ser Ser His Gln Gly Ala Ser Pro
       50                  55                  60
   Pro Arg Ser Ser Gln Val Val Gly Trp Pro Pro Ile Gly Leu His Arg
   65                  70                  75                  80
   Met Asn Ser Leu Val Asn Asn Gln Ala Met Lys Ala Ala Arg Ala Glu
                   85                  90                  95
   Glu Gly Asp Gly Glu Lys Lys Val Val Lys Asn Gly Glu Leu Lys Asp
               100                 105                 110
   Val Ser Met Lys Val Asn Pro Lys Val Gln Gly Leu Gly Phe Val Lys
           115                 120                 125
   Val Asn Met Asp Gly Val Gly Ile Gly Arg Lys Val Asp Met Arg Ala
       130                 135                 140
   His Ser Ser Tyr Glu Asn Leu Ala Gln Thr Leu Glu Glu Met Phe Phe
   145                 150                 155                 160
   Gly Met Thr Gly Thr Thr Cys Arg Glu Thr Val Lys Pro Leu Arg Leu
                   165                 170                 175
   Leu Asp Gly Ser Ser Asp Phe Val Leu Thr Tyr Glu Asp Lys Gly Ile
               180                 185                 190
   Gly Cys Leu Leu Glu Met Phe His Gly Glu Cys Leu Ser Thr Arg
           195                 200                 205
<210> 11
   <211> 1033
   <212> DNA
   <213> Arabidopsis thaliana
<220>
   <221> CDS
   <222> (113)..(862)
   <223> G663
<400> 11
   gtcgacccac gcgtccgtgg gaagccacaa taacccccta ttcctcggcc ttttttaaaa      60
   aagttttaga ataatccgat aaaatacttt tatattaatt tttctttggt cc atg gag     118
                                                             Met Glu
                                                             1
   ggt tcg tcc aaa ggg ttg agg aaa ggt gca tgg act gct gaa gaa gat      166
   Gly Ser Ser Lys Gly Leu Arg Lys Gly Ala Trp Thr Ala Glu Glu Asp
           5                   10                  15
   agt ctc ttg agg cta tgt att gat aag tat gga gaa ggc aaa tgg cat      214
   Ser Leu Leu Arg Leu Cys Ile Asp Lys Tyr Gly Glu Gly Lys Trp His
       20                  25                  30
   caa gtt cct ttg aga gct ggg cta aat cga tgc aga aag agt tgt aga      262
   Gln Val Pro Leu Arg Ala Gly Leu Asn Arg Cys Arg Lys Ser Cys Arg
   35                  40                  45                  50
   cta aga tgg ttg aac tat ttg aag cca agt atc aag aga gga aga ctt      310
   Leu Arg Trp Leu Asn Tyr Leu Lys Pro Ser Ile Lys Arg Gly Arg Leu
                   55                  60                  65
   agc aat gat gaa gtt gat ctt ctt ctt cgc ctt cat aag ctt cta gga      358
   Ser Asn Asp Glu Val Asp Leu Leu Leu Arg Leu His Lys Leu Leu Gly
               70                  75                  80
   aat agg tgg tcc ttg att gct ggt cga ttg cct ggt cgg acc gct aat      406
   Asn Arg Trp Ser Leu Ile Ala Gly Arg Leu Pro Gly Arg Thr Ala Asn
           85                  90                  95
   gat gtc aaa aat tac tgg aac acc cat ctg agt aaa aaa cat gag tct      454
   Asp Val Lys Asn Tyr Trp Asn Thr His Leu Ser Lys Lys His Glu Ser
       100                 105                 110
   tcg tgt tgt aag tct aaa atg aaa aag aaa aac att att tcc cct cct      502
   Ser Cys Cys Lys Ser Lys Met Lys Lys Lys Asn Ile Ile Ser Pro Pro
   115                 120                 125                 130
   aca aca ccg gtc caa aaa atc ggt gtt ttt aag cct cga cct cga tcc      550
   Thr Thr Pro Val Gln Lys Ile Gly Val Phe Lys Pro Arg Pro Arg Ser
                   135                 140                 145
   ttc tct gtt aac aat ggt tgc agc cat ctc aat ggt ctg cca gaa gtt      598
   Phe Ser Val Asn Asn Gly Cys Ser His Leu Asn Gly Leu Pro Glu Val
               150                 155                 160
   gat tta att cct tca tgc ctt gga ctc aag aaa aat aat gtt tgt gaa      646
   Asp Leu Ile Pro Ser Cys Leu Gly Leu Lys Lys Asn Asn Val Cys Glu
           165                 170                 175
   aat agt atc aca tgt aac aaa gat gat gag aaa gat gat ttt gtg aat      694
   Asn Ser Ile Thr Cys Asn Lys Asp Asp Glu Lys Asp Asp Phe Val Asn
       180                 185                 190
   aat cta atg aat gga gat aat atg tgg ttg gag aat tta ctg ggg gaa      742
   Asn Leu Met Asn Gly Asp Asn Met Trp Leu Glu Asn Leu Leu Gly Glu
   195                 200                 205                 210
   aac caa gaa gct gat gcg att gtt cct gaa gcg acg aca gct gaa cat      790
   Asn Gln Glu Ala Asp Ala Ile Val Pro Glu Ala Thr Thr Ala Glu His
                   215                 220                 225
   ggg gcc act ttg gcg ttt gac gtt gag caa ctt tgg agt ctg ttt gat      838
   Gly Ala Thr Leu Ala Phe Asp Val Glu Gln Leu Trp Ser Leu Phe Asp
                   230                 235                 240
   gga gag act gtt gaa ctt gat tag tgtttctcac cgtttgttta agattgtggg      892
   Gly Glu Thr Val Glu Leu Asp
           245
   tggcttttct ttcgtatttt agtaatgtat ttttctgtat gaagtaaaga atttcagcat     952
   tttaagaaaa atggttatgt ttctacgtaa taaaaaaaaa cgttatttat aaaaaaaaaa    1012
   aaaaaaaaaa aaaaaaaaaa a                                              1033
<210> 12
   <211> 249
   <212> PRT
   <213> Arabidopsis thaliana
<400> 12
   Met Glu Gly Ser Ser Lys Gly Leu Arg Lys Gly Ala Trp Thr Ala Glu
   1               5                   10                  15
   Glu Asp Ser Leu Leu Arg Leu Cys Ile Asp Lys Tyr Gly Glu Gly Lys
               20                  25                  30
   Trp His Gln Val Pro Leu Arg Ala Gly Leu Asn Arg Cys Arg Lys Ser
           35                  40                  45
   Cys Arg Leu Arg Trp Leu Asn Tyr Leu Lys Pro Ser Ile Lys Arg Gly
       50                  55                  60
   Arg Leu Ser Asn Asp Glu Val Asp Leu Leu Leu Arg Leu His Lys Leu
   65                  70                  75                  80
   Leu Gly Asn Arg Trp Ser Leu Ile Ala Gly Arg Leu Pro Gly Arg Thr
                   85                  90                  95
   Ala Asn Asp Val Lys Asn Tyr Trp Asn Thr His Leu Ser Lys Lys His
               100                 105                 110
   Glu Ser Ser Cys Cys Lys Ser Lys Met Lys Lys Lys Asn Ile Ile Ser
           115                 120                 125
   Pro Pro Thr Thr Pro Val Gln Lys Ile Gly Val Phe Lys Pro Arg Pro
       130                 135                 140
   Arg Ser Phe Ser Val Asn Asn Gly Cys Ser His Leu Asn Gly Leu Pro
   145                 150                 155                 160
   Glu Val Asp Leu Ile Pro Ser Cys Leu Gly Leu Lys Lys Asn Asn Val
                   165                 170                 175
   Cys Glu Asn Ser Ile Thr Cys Asn Lys Asp Asp Glu Lys Asp Asp Phe
               180                 185                 190
   Val Asn Asn Leu Met Asn Gly Asp Asn Met Trp Leu Glu Asn Leu Leu
           195                 200                 205
   Gly Glu Asn Gln Glu Ala Asp Ala Ile Val Pro Glu Ala Thr Thr Ala
       210                 215                 220
   Glu His Gly Ala Thr Leu Ala Phe Asp Val Glu Gln Leu Trp Ser Leu
   225                 230                 235                 240
   Phe Asp Gly Glu Thr Val Glu Leu Asp
                   245
<210> 13
   <211> 1640
   <212> DNA
   <213> Arabidopsis thaliana
<220>
   <221> CDS
   <222> (76)..(1431)
   <223> G776
<400> 13
   tgcaattgaa ggtgaggttt ggtgaaaggg aaattgagaa aaccctagaa caagtacggt      60
   ctctattttg cttta atg ggt cgc gaa tct gtg gct gtt gtg act gcg ccg      111
                    Met Gly Arg Glu Ser Val Ala Val Val Thr Ala Pro
                    1               5                   10
   ccc tcg gcg act gct ccg ggt act gct tcg gtg gcg acc tcg ctt gct       159
   Pro Ser Ala Thr Ala Pro Gly Thr Ala Ser Val Ala Thr Ser Leu Ala
           15                  20                  25
   cct ggc ttc cga ttt cat ccg act gat gag gaa ctc gtg agc tat tac       207
   Pro Gly Phe Arg Phe His Pro Thr Asp Glu Glu Leu Val Ser Tyr Tyr
       30                  35                  40
   ttg aag agg aag gtt ctg ggc caa cct gta cgc ttc gat gcg att gga       255
   Leu Lys Arg Lys Val Leu Gly Gln Pro Val Arg Phe Asp Ala Ile Gly
   45                  50                  55                  60
   gag gtc gat ata tac aag cat gag ccc tgg gat tta gca gtg ttt tcg       303
   Glu Val Asp Ile Tyr Lys His Glu Pro Trp Asp Leu Ala Val Phe Ser
                   65                  70                  75
   aga ttg aag aca agg gac caa gaa tgg tac ttc tac agt gca tta gat       351
   Arg Leu Lys Thr Arg Asp Gln Glu Trp Tyr Phe Tyr Ser Ala Leu Asp
               80                  85                  90
   aag aag tat gga aac ggt gct agg atg aac cga gca act aac aga ggg       399
   Lys Lys Tyr Gly Asn Gly Ala Arg Met Asn Arg Ala Thr Asn Arg Gly
           95                  100                 105
   tac tgg aaa gct act gga aaa gac aga gaa atc cgc cgt gac att ctg       447
   Tyr Trp Lys Ala Thr Gly Lys Asp Arg Glu Ile Arg Arg Asp Ile Leu
       110                 115                 120
   ctt ctc ggt atg aaa aag aca ctt gtt ttc cac agt ggg cgt gca cca       495
   Leu Leu Gly Met Lys Lys Thr Leu Val Phe His Ser Gly Arg Ala Pro
   125                 130                 135                 140
   gac ggg ctt cgg act aat tgg gtt atg cat gag tat cgc ctt gtg gaa       543
   Asp Gly Leu Arg Thr Asn Trp Val Met His Glu Tyr Arg Leu Val Glu
                   145                 150                 155
   tat gaa acc gag aaa aac gga aac ctg gtg caa gat gca tat gtg ttg       591
   Tyr Glu Thr Glu Lys Asn Gly Asn Leu Val Gln Asp Ala Tyr Val Leu
               160                 165                 170
   tgt aga gtc ttc cac aag aat aac att ggg cca cca agt ggg aac aga       639
   Cys Arg Val Phe His Lys Asn Asn Ile Gly Pro Pro Ser Gly Asn Arg
           175                 180                 185
   tat gct ccg ttc atg gaa gag gaa tgg gct gat gat gaa gga gct ctg       687
   Tyr Ala Pro Phe Met Glu Glu Glu Trp Ala Asp Asp Glu Gly Ala Leu
       190                 195                 200
   att cca gga ata gac gtt aag ctc agg cta gag ccg ccg cca gta gcc      735
   Ile Pro Gly Ile Asp Val Lys Leu Arg Leu Glu Pro Pro Pro Val Ala
   205                 210                 215                 220
   aat gga aac gac cag atg gac cag gaa atc cag tca gcc agc aag agt      783
   Asn Gly Asn Asp Gln Met Asp Gln Glu Ile Gln Ser Ala Ser Lys Ser
                   225                 230                 235
   ctc atc aac atc aat gag cca ccg aga gag aca gct cca ctg gat atc      831
   Leu Ile Asn Ile Asn Glu Pro Pro Arg Glu Thr Ala Pro Leu Asp Ile
               240                 245                 250
   gaa tcg gac caa cag aat cat cat gag aat gac ctc aag ccg gag gag      879
   Glu Ser Asp Gln Gln Asn His His Glu Asn Asp Leu Lys Pro Glu Glu
           255                 260                 265
   cat aac aac aat aat aat tat gat gaa aac gag gaa aca ctc aaa cgc      927
   His Asn Asn Asn Asn Asn Tyr Asp Glu Asn Glu Glu Thr Leu Lys Arg
       270                 275                 280
   gag cag atg gaa gaa gag gag cgt cct cct cga cct gta tgc gtt ctc      975
   Glu Gln Met Glu Glu Glu Glu Arg Pro Pro Arg Pro Val Cys Val Leu
   285                 290                 295                 300
   aac aaa gaa gct cca tta cct ctt ctg caa tac aaa cgt aga cgc caa     1023
   Asn Lys Glu Ala Pro Leu Pro Leu Leu Gln Tyr Lys Arg Arg Arg Gln
                   305                 310                 315
   agc gag tcc aac aac aac tca agc agg aac aca cag gac cat tgt tcg     1071
   Ser Glu Ser Asn Asn Asn Ser Ser Arg Asn Thr Gln Asp His Cys Ser
               320                 325                 330
   tcc aca aca aca act gtc gac aat aca acc act tta atc tca tca tct     1119
   Ser Thr Thr Thr Thr Val Asp Asn Thr Thr Thr Leu Ile Ser Ser Ser
           335                 340                 345
   gcc gct gcc acc aac act gcc atc tct gca ttg ctt gag ttc tca ctc     1167
   Ala Ala Ala Thr Asn Thr Ala Ile Ser Ala Leu Leu Glu Phe Ser Leu
       350                 355                 360
   atg ggt atc tcc gac aag aaa gaa aag ccg cag caa ccg cta cgt cct     1215
   Met Gly Ile Ser Asp Lys Lys Glu Lys Pro Gln Gln Pro Leu Arg Pro
   365                 370                 375                 380
   cac aag gaa cct ttg cct cct caa act cca ctt gca tct cct gaa gag     1263
   His Lys Glu Pro Leu Pro Pro Gln Thr Pro Leu Ala Ser Pro Glu Glu
                   385                 390                 395
   aag gtt aat gat ctc cag aag gag att cac cag atg tct gtt gaa aga     1311
   Lys Val Asn Asp Leu Gln Lys Glu Ile His Gln Met Ser Val Glu Arg
               400                 405                 410
   gaa act ttc aag ctt gaa atg atg agt gca gaa gct atg atc agt att     1359
   Glu Thr Phe Lys Leu Glu Met Met Ser Ala Glu Ala Met Ile Ser Ile
           415                 420                 425
   ctc cag tca agg atc gat gcg ctg cgt cag gag aac gag gaa ctc aag     1407
   Leu Gln Ser Arg Ile Asp Ala Leu Arg Gln Glu Asn Glu Glu Leu Lys
       430                 435                 440
   aag aac aat gct aat gga caa taa aggctctaaa aacatctctc caggttactt    1461
   Lys Asn Asn Ala Asn Gly Gln
   445                 450
   cttattgccc ttcgcctttt atttagcttt aatctcccta atactatgac ccatctacat   1521
   agctcctcta gacagattgc gaactgtgtg aatctctgtt gtaacatagg ataaaacgga   1581
   ttcgagcccc tgagctgagt gttttatcct tcttctttta aaaaaaaaaa aaaaaaaaa    1640
<210> 14
   <211> 451
   <212> PRT
   <213> Arabidopsis thaliana
<400> 14
   Met Gly Arg Glu Ser Val Ala Val Val Thr Ala Pro Pro Ser Ala Thr
   1               5                   10                  15
   Ala Pro Gly Thr Ala Ser Val Ala Thr Ser Leu Ala Pro Gly Phe Arg
               20                  25                  30
   Phe His Pro Thr Asp Glu Glu Leu Val Ser Tyr Tyr Leu Lys Arg Lys
           35                  40                  45
   Val Leu Gly Gln Pro Val Arg Phe Asp Ala Ile Gly Glu Val Asp Ile
       50                  55                  60
   Tyr Lys His Glu Pro Trp Asp Leu Ala Val Phe Ser Arg Leu Lys Thr
   65                  70                  75                  80
   Arg Asp Gln Glu Trp Tyr Phe Tyr Ser Ala Leu Asp Lys Lys Tyr Gly
                   85                  90                  95
   Asn Gly Ala Arg Met Asn Arg Ala Thr Asn Arg Gly Tyr Trp Lys Ala
               100                 105                 110
   Thr Gly Lys Asp Arg Glu Ile Arg Arg Asp Ile Leu Leu Leu Gly Met
           115                 120                 125
   Lys Lys Thr Leu Val Phe His Ser Gly Arg Ala Pro Asp Gly Leu Arg
       130                 135                 140
   Thr Asn Trp Val Met His Glu Tyr Arg Leu Val Glu Tyr Glu Thr Glu
   145                 150                 155                 160
   Lys Asn Gly Asn Leu Val Gln Asp Ala Tyr Val Leu Cys Arg Val Phe
                   165                 170                 175
   His Lys Asn Asn Ile Gly Pro Pro Ser Gly Asn Arg Tyr Ala Pro Phe
               180                 185                 190
   Met Glu Glu Glu Trp Ala Asp Asp Glu Gly Ala Leu Ile Pro Gly Ile
           195                 200                 205
   Asp Val Lys Leu Arg Leu Glu Pro Pro Pro Val Ala Asn Gly Asn Asp
       210                 215                 220
   Gln Met Asp Gln Glu Ile Gln Ser Ala Ser Lys Ser Leu Ile Asn Ile
   225                 230                 235                 240
   Asn Glu Pro Pro Arg Glu Thr Ala Pro Leu Asp Ile Glu Ser Asp Gln
                   245                 250                 255
   Gln Asn His His Glu Asn Asp Leu Lys Pro Glu Glu His Asn Asn Asn
               260                 265                 270
   Asn Asn Tyr Asp Glu Asn Glu Glu Thr Leu Lys Arg Glu Gln Met Glu
           275                 280                 285
   Glu Glu Glu Arg Pro Pro Arg Pro Val Cys Val Leu Asn Lys Glu Ala
   290 295 300
   Pro Leu Pro Leu Leu Gln Tyr Lys Arg Arg Arg Gln Ser Glu Ser Asn
   305 310 315 320
   Asn Asn Ser Ser Arg Asn Thr Gln Asp His Cys Ser Ser Thr Thr Thr
   325 330 335
   Thr Val Asp Asn Thr Thr Thr Leu Ile Ser Ser Ser Ala Ala Ala Thr
   340 345 350
   Asn Thr Ala Ile Ser Ala Leu Leu Glu Phe Ser Leu Met Gly Ile Ser
   355 360 365
   Asp Lys Lys Glu Lys Pro Gln Gln Pro Leu Arg Pro His Lys Glu Pro
   370 375 380
   Leu Pro Pro Gln Thr Pro Leu Ala Ser Pro Glu Glu Lys Val Asn Asp
   385 390 395 400
   Leu Gln Lys Glu Ile His Gln Met Ser Val Glu Arg Glu Thr Phe Lys
   405 410 415
   Leu Glu Met Met Ser Ala Glu Ala Met Ile Ser Ile Leu Gln Ser Arg
   420 425 430
   Ile Asp Ala Leu Arg Gln Glu Asn Glu Glu Leu Lys Lys Asn Asn Ala
   435 440 445
   Asn Gly Gln
   450
<210> 15
   <211> 1389
   <212> DNA
   <213> Arabidopsis thaliana
<220>
   <221> CDS
   <222> (50)..(1249)
   <223> G778
<400> 15
   tctcaataac acaaaacctt ttaaactagt aaaatacaca gattttagg atg agc caa      58
                                                         Met Ser Gln
                                                         1
   tgt gtt cca aac tgt cac atc gat gat act ccg gca gca gcc acc acc       106
   Cys Val Pro Asn Cys His Ile Asp Asp Thr Pro Ala Ala Ala Thr Thr
       5                   10                  15
   acc gtc cgc tcc acc aca gcc gca gac atc ccc ata tta gac tac gag       154
   Thr Val Arg Ser Thr Thr Ala Ala Asp Ile Pro Ile Leu Asp Tyr Glu
   20                  25                  30                  35
   gta gcc gag ctg acg tgg gag aac ggg caa cta ggc ttg cac ggc tta       202
   Val Ala Glu Leu Thr Trp Glu Asn Gly Gln Leu Gly Leu His Gly Leu
                   40                  45                  50
   ggt cca ccg cga gtg acg gct tcg tcg acc aag tac tcc aca ggc gcc       250
   Gly Pro Pro Arg Val Thr Ala Ser Ser Thr Lys Tyr Ser Thr Gly Ala
               55                  60                  65
   ggt gga acg ttg gag tcg ata gtg gac caa gct act cgc ctc cct aac      298
   Gly Gly Thr Leu Glu Ser Ile Val Asp Gln Ala Thr Arg Leu Pro Asn
           70                  75                  80
   cct aag ccc acg gat gag ctc gtc ccg tgg ttc cat cat cgc tcc tcc      346
   Pro Lys Pro Thr Asp Glu Leu Val Pro Trp Phe His His Arg Ser Ser
       85                  90                  95
   agg gcc gcg atg gca atg gac gcg ctt gtc cct tgc tcc aac cta gta      394
   Arg Ala Ala Met Ala Met Asp Ala Leu Val Pro Cys Ser Asn Leu Val
   100                 105                 110                 115
   cac gag cag cag agc aag cct ggt ggc gtt ggc tcc acc cgg gtg ggg      442
   His Glu Gln Gln Ser Lys Pro Gly Gly Val Gly Ser Thr Arg Val Gly
                   120                 125                 130
   tca tgt agc gat ggt cgt acc atg ggc ggt gga aaa cga gca aga gtg      490
   Ser Cys Ser Asp Gly Arg Thr Met Gly Gly Gly Lys Arg Ala Arg Val
               135                 140                 145
   gca ccg gag tgg agc ggc ggc ggg agt cag cgg ctg acc atg gac act      538
   Ala Pro Glu Trp Ser Gly Gly Gly Ser Gln Arg Leu Thr Met Asp Thr
           150                 155                 160
   tac gac gta ggt ttc acc tca aca tca atg ggc tcg cac gat aac aca      586
   Tyr Asp Val Gly Phe Thr Ser Thr Ser Met Gly Ser His Asp Asn Thr
       165                 170                 175
   atc gac gat cat gac tcc gtc tgc cac agc cgc cca cag atg gag gac      634
   Ile Asp Asp His Asp Ser Val Cys His Ser Arg Pro Gln Met Glu Asp
   180                 185                 190                 195
   gaa gaa gag aag aaa gcc gga gga aaa tca tca gtt tca acc aag aga      682
   Glu Glu Glu Lys Lys Ala Gly Gly Lys Ser Ser Val Ser Thr Lys Arg
                   200                 205                 210
   agc aga gct gct gct att cat aac caa tcc gaa cgt aag agg aga gat      730
   Ser Arg Ala Ala Ala Ile His Asn Gln Ser Glu Arg Lys Arg Arg Asp
               215                 220                 225
   aaa atc aat caa agg atg aag act ttg caa aaa ctg gtt ccc aat tcc      778
   Lys Ile Asn Gln Arg Met Lys Thr Leu Gln Lys Leu Val Pro Asn Ser
           230                 235                 240
   agc aag acg gat aaa gca tct atg ttg gat gaa gtg ata gag tat ttg      826
   Ser Lys Thr Asp Lys Ala Ser Met Leu Asp Glu Val Ile Glu Tyr Leu
       245                 250                 255
   aag caa ctt caa gca caa gtg agc atg atg agc aga atg aat atg cct      874
   Lys Gln Leu Gln Ala Gln Val Ser Met Met Ser Arg Met Asn Met Pro
   260                 265                 270                 275
   tct atg atg ctt cct atg gcc atg cag caa caa caa caa cta caa atg      922
   Ser Met Met Leu Pro Met Ala Met Gln Gln Gln Gln Gln Leu Gln Met
                   280                 285                 290
   tct ctc atg tcc aat ccc atg ggt tta ggg atg ggc atg ggg atg ccc      970
   Ser Leu Met Ser Asn Pro Met Gly Leu Gly Met Gly Met Gly Met Pro
               295                 300                 305
   ggt ctc ggt ctc ctc gac ctt aat tct atg aac cga gct gct gca agc     1018
   Gly Leu Gly Leu Leu Asp Leu Asn Ser Met Asn Arg Ala Ala Ala Ser
           310                 315                 320
   gct cct aat atc cat gcc aac atg atg cca aac cca ttt ttg ccc atg     1066
   Ala Pro Asn Ile His Ala Asn Met Met Pro Asn Pro Phe Leu Pro Met
       325                 330                 335
   aat tgt cca tcg tgg gat gct tct tcc aat gac tct cga ttt cag tct     1114
   Asn Cys Pro Ser Trp Asp Ala Ser Ser Asn Asp Ser Arg Phe Gln Ser
   340                 345                 350                 355
   cct ctc atc ccg gat cct atg tct gcc ttt ctt gca tgc tct act cag     1162
   Pro Leu Ile Pro Asp Pro Met Ser Ala Phe Leu Ala Cys Ser Thr Gln
                   360                 365                 370
   cca acg acg atg gaa gcg tat agc agg atg gct aca tta tat cag caa      1210
   Pro Thr Thr Met Glu Ala Tyr Ser Arg Met Ala Thr Leu Tyr Gln Gln
               375                 380                 385
   atg caa caa caa ctt cct cct cct tcg aat cca aaa tga ttattactca       1259
   Met Gln Gln Gln Leu Pro Pro Pro Ser Asn Pro Lys
           390                 395
   aacacctcta tatagtttac gtctatatat gtgttagtca catacataca tatatatatt     1319
   ccatcataat tatttattta tatgtatagg cttctcatga attatgatat tatacgtatt     1379
   acgtaaaaaa                                                            1389
<210> 16
   <211> 399
   <212> PRT
   <213> Arabidopsis thaliana
<400> 16
   Met Ser Gln Cys Val Pro Asn Cys His Ile Asp Asp Thr Pro Ala Ala
   1 5 10 15
   Ala Thr Thr Thr Val Arg Ser Thr Thr Ala Ala Asp Ile Pro Ile Leu
   20 25 30
   Asp Tyr Glu Val Ala Glu Leu Thr Trp Glu Asn Gly Gln Leu Gly Leu
   35 40 45
   His Gly Leu Gly Pro Pro Arg Val Thr Ala Ser Ser Thr Lys Tyr Ser
   50 55 60
   Thr Gly Ala Gly Gly Thr Leu Glu Ser Ile Val Asp Gln Ala Thr Arg
   65 70 75 80
   Leu Pro Asn Pro Lys Pro Thr Asp Glu Leu Val Pro Trp Phe His His
   85 90 95
   Arg Ser Ser Arg Ala Ala Met Ala Met Asp Ala Leu Val Pro Cys Ser
   100 105 110
   Asn Leu Val His Glu Gln Gln Ser Lys Pro Gly Gly Val Gly Ser Thr
   115 120 125
   Arg Val Gly Ser Cys Ser Asp Gly Arg Thr Met Gly Gly Gly Lys Arg
   130 135 140
   Ala Arg Val Ala Pro Glu Trp Ser Gly Gly Gly Ser Gln Arg Leu Thr
   145 150 155 160
   Met Asp Thr Tyr Asp Val Gly Phe Thr Ser Thr Ser Met Gly Ser His
   165 170 175
   Asp Asn Thr Ile Asp Asp His Asp Ser Val Cys His Ser Arg Pro Gln
   180 185 190
   Met Glu Asp Glu Glu Glu Lys Lys Ala Gly Gly Lys Ser Ser Val Ser
   195 200 205
   Thr Lys Arg Ser Arg Ala Ala Ala Ile His Asn Gln Ser Glu Arg Lys
   210 215 220
   Arg Arg Asp Lys Ile Asn Gln Arg Met Lys Thr Leu Gln Lys Leu Val
   225 230 235 240
   Pro Asn Ser Ser Lys Thr Asp Lys Ala Ser Met Leu Asp Glu Val Ile
   245 250 255
   Glu Tyr Leu Lys Gln Leu Gln Ala Gln Val Ser Met Met Ser Arg Met
   260 265 270
   Asn Met Pro Ser Met Met Leu Pro Met Ala Met Gln Gln Gln Gln Gln
   275 280 285
   Leu Gln Met Ser Leu Met Ser Asn Pro Met Gly Leu Gly Met Gly Met
   290 295 300
   Gly Met Pro Gly Leu Gly Leu Leu Asp Leu Asn Ser Met Asn Arg Ala
   305 310 315 320
   Ala Ala Ser Ala Pro Asn Ile His Ala Asn Met Met Pro Asn Pro Phe
   325 330 335
   Leu Pro Met Asn Cys Pro Ser Trp Asp Ala Ser Ser Asn Asp Ser Arg
   340 345 350
   Phe Gln Ser Pro Leu Ile Pro Asp Pro Met Ser Ala Phe Leu Ala Cys
   355 360 365
   Ser Thr Gln Pro Thr Thr Met Glu Ala Tyr Ser Arg Met Ala Thr Leu
   370 375 380
   Tyr Gln Gln Met Gln Gln Gln Leu Pro Pro Pro Ser Asn Pro Lys
   385 390 395
<210> 17
   <211> 1126
   <212> DNA
   <213> Arabidopsis thaliana
<220>
   <221> CDS
   <222> (282)..(920)
   <223> G865
<400> 17
   atccccactt gttgttcatc accaagccaa gctccatgtc ctagtcactc cacagattcc     60
   ctatcatcat caattcgttt caaacttagt tcctttcaaa gtcttgtaca tatatacaca    120
   cacacctatt attctcttgg tgtgtttgtg tgttacatat acgtgtgagt acatactttg    180
   ttgtaaaagt ggatcggagg tatggaaagg gaccggttcc accggaaaca tcggcggcgg    240
   cggatgataa ttcgtcttgg aacgagactg atgtcaccgc c atg gtc tcc gct ctc    296
                                                 Met Val Ser Ala Leu
                                                 1               5
   agc cgt gtc ata gag aat ccg aca gac ccg ccg gtc aaa caa gag ctt      344
   Ser Arg Val Ile Glu Asn Pro Thr Asp Pro Pro Val Lys Gln Glu Leu
                   10                  15                  20
   gat aaa tcg gat caa cat caa cca gac caa gat caa cca aga aga aga      392
   Asp Lys Ser Asp Gln His Gln Pro Asp Gln Asp Gln Pro Arg Arg Arg
               25                  30                  35
   cac tat aga ggc gta agg cag aga cca tgg ggt aaa tgg gcg gca gaa      440
   His Tyr Arg Gly Val Arg Gln Arg Pro Trp Gly Lys Trp Ala Ala Glu
           40                  45                  50
   atc cgc gat cca aag aaa gca gcc cgt gtc tgg ctc ggg act ttc gag      488
   Ile Arg Asp Pro Lys Lys Ala Ala Arg Val Trp Leu Gly Thr Phe Glu
       55                  60                  65
   acg gca gag gaa gct gct tta gcc tat gac cga gct gcc ctc aaa ttc      536
   Thr Ala Glu Glu Ala Ala Leu Ala Tyr Asp Arg Ala Ala Leu Lys Phe
   70                  75                  80                  85
   aaa ggc acc aag gct aaa ctg aac ttc cct gaa cgg gtc caa ggc cct      584
   Lys Gly Thr Lys Ala Lys Leu Asn Phe Pro Glu Arg Val Gln Gly Pro
                   90                  95                  100
   act acc acc aca acc att tct cat gca cca aga gga gtt agt gaa tcc      632
   Thr Thr Thr Thr Thr Ile Ser His Ala Pro Arg Gly Val Ser Glu Ser
               105                 110                 115
   atg aac tca cct cct cct cga cct ggt cca cct tca act act act act      680
   Met Asn Ser Pro Pro Pro Arg Pro Gly Pro Pro Ser Thr Thr Thr Thr
           120                 125                 130
   tcg tgg cca atg act tat aac cag gac ata ctt caa tac gct cag ttg      728
   Ser Trp Pro Met Thr Tyr Asn Gln Asp Ile Leu Gln Tyr Ala Gln Leu
       135                 140                 145
   ctt acg agt aac aat gag gtt gat tta tca tac tac acg tcg act ctc      776
   Leu Thr Ser Asn Asn Glu Val Asp Leu Ser Tyr Tyr Thr Ser Thr Leu
   150                 155                 160                 165
   ttc agt caa cct ttt tca acg cct tct tca tct tct tct tcc tcc caa      824
   Phe Ser Gln Pro Phe Ser Thr Pro Ser Ser Ser Ser Ser Ser Ser Gln
                   170                 175                 180
   cag acg cag caa cag cag cta caa caa caa caa cag cag cgt gaa gaa      872
   Gln Thr Gln Gln Gln Gln Leu Gln Gln Gln Gln Gln Gln Arg Glu Glu
               185                 190                 195
   gaa gag aag aat tat ggt tac aat tat tat aac tac cca aga gaa taa      920
   Glu Glu Lys Asn Tyr Gly Tyr Asn Tyr Tyr Asn Tyr Pro Arg Glu
           200                 205                 210
   tctaattatt attgttggtc gaatcagttt tataaatagc tatcatagtt tcatttttgg    980
   tttccgtaac ctttgttgca tggaaaatat gaatgaacga gggacatgtg taacaatttg   1040
   tttgtgtttc gtaaatgtta gttgtatttg gatttgctga agtttgattt tctgagcata   1100
   aatcatttga cggtcaaaaa aaaaaa                                        1126
<210> 18
   <211> 212
   <212> PRT
   <213> Arabidopsis thaliana
<400> 18
   Met Val Ser Ala Leu Ser Arg Val Ile Glu Asn Pro Thr Asp Pro Pro
   1               5                   10                  15
   Val Lys Gln Glu Leu Asp Lys Ser Asp Gln His Gln Pro Asp Gln Asp
               20                  25                  30
   Gln Pro Arg Arg Arg His Tyr Arg Gly Val Arg Gln Arg Pro Trp Gly
           35                  40                  45
   Lys Trp Ala Ala Glu Ile Arg Asp Pro Lys Lys Ala Ala Arg Val Trp
       50                  55                  60
   Leu Gly Thr Phe Glu Thr Ala Glu Glu Ala Ala Leu Ala Tyr Asp Arg
   65                  70                  75                  80
   Ala Ala Leu Lys Phe Lys Gly Thr Lys Ala Lys Leu Asn Phe Pro Glu
                   85                  90                  95
   Arg Val Gln Gly Pro Thr Thr Thr Thr Thr Ile Ser His Ala Pro Arg
               100                 105                 110
   Gly Val Ser Glu Ser Met Asn Ser Pro Pro Pro Arg Pro Gly Pro Pro
           115                 120                 125
   Ser Thr Thr Thr Thr Ser Trp Pro Met Thr Tyr Asn Gln Asp Ile Leu
       130                 135                 140
   Gln Tyr Ala Gln Leu Leu Thr Ser Asn Asn Glu Val Asp Leu Ser Tyr
   145                 150                 155                 160
   Tyr Thr Ser Thr Leu Phe Ser Gln Pro Phe Ser Thr Pro Ser Ser Ser
                   165                 170                 175
   Ser Ser Ser Ser Gln Gln Thr Gln Gln Gln Gln Leu Gln Gln Gln Gln
               180                 185                 190
   Gln Gln Arg Glu Glu Glu Glu Lys Asn Tyr Gly Tyr Asn Tyr Tyr Asn
           195                 200                 205
   Tyr Pro Arg Glu
       210
<210> 19
   <211> 1571
   <212> DNA
   <213> Arabidopsis thaliana
<220>
   <221> CDS
   <222> (428)..(1402)
   <223> G869
<400> 19
   aggaacagtg aaaggttcgg ttttttgggt ttcgatctga taatcaacaa gaaaaaaggg     60
   tttgatttat gtcggctggg tttgaatcga ctgtgatttt gtctttgatt catatctctt    120
   ctccgatttc atcatcatct tccccatcat cgtcgtcttt gaaatcttgt cttctcaacg    180
   ctcttcactt ctgctgtaat aagcagaggc ttgttctgga gactccttct ctttccatgc    240
   gcttaagacc caaaaggact tgttctagtg ttgaagtctt tgggggtttt cacataaagc    300
   agcaaaagtt ttcttttttc atagttcgct gagagttttg agttttgata ccaaaaaagt    360
   tttgaccttt tagagtgatt ttttgttctt tctgttttct gggtattttt gaggagtggg    420
   tttaaca atg gtt gcg att aga aag gaa cag tct ttg agt ggt gtt agt      469
           Met Val Ala Ile Arg Lys Glu Gln Ser Leu Ser Gly Val Ser
           1               5                   10
   agc gag att aag aag aga gct aag aga aac act cta tcg tcc ctt cct      517
   Ser Glu Ile Lys Lys Arg Ala Lys Arg Asn Thr Leu Ser Ser Leu Pro
   15                  20                  25                  30
   caa gaa acc caa cct ttg agg aaa gtc cgt att att gtg aat gat cct      565
   Gln Glu Thr Gln Pro Leu Arg Lys Val Arg Ile Ile Val Asn Asp Pro
                   35                  40                  45
   tat gct act gat gat tcc tct agt gat gag gaa gag ctt aag gtt cct      613
   Tyr Ala Thr Asp Asp Ser Ser Ser Asp Glu Glu Glu Leu Lys Val Pro
               50                  55                  60
   aag cca agg aaa atg aaa cgt atc gtt cgt gag att aac ttt cct tct      661
   Lys Pro Arg Lys Met Lys Arg Ile Val Arg Glu Ile Asn Phe Pro Ser
           65                  70                  75
   atg gaa gtt tct gaa cag cct tct gag agt tct tct cag gac agt act      709
   Met Glu Val Ser Glu Gln Pro Ser Glu Ser Ser Ser Gln Asp Ser Thr
       80                  85                  90
   aaa act gat ggc aag ata gct gtg tca gct tct cct gct gtt cct agg      757
   Lys Thr Asp Gly Lys Ile Ala Val Ser Ala Ser Pro Ala Val Pro Arg
   95                  100                 105                 110
   aag aag cct gtt ggt gtt agg caa agg aaa tgg ggg aaa tgg gct gct      805
   Lys Lys Pro Val Gly Val Arg Gln Arg Lys Trp Gly Lys Trp Ala Ala
                   115                 120                 125
   gag att aga gat cct att aag aaa act agg act tgg ttg ggt act ttt      853
   Glu Ile Arg Asp Pro Ile Lys Lys Thr Arg Thr Trp Leu Gly Thr Phe
               130                 135                 140
   gat act ctt gaa gaa gct gct aaa gct tat gat gct aag aag ctt gag      901
   Asp Thr Leu Glu Glu Ala Ala Lys Ala Tyr Asp Ala Lys Lys Leu Glu
           145                 150                 155
   ttt gat gct att gtt gct gga aat gtg tcc act act aaa cgt gat gtt      949
   Phe Asp Ala Ile Val Ala Gly Asn Val Ser Thr Thr Lys Arg Asp Val
       160                 165                 170
   tct tca tct gag act agc caa tgc tct cgt tct tca cct gtt gtt cct      997
   Ser Ser Ser Glu Thr Ser Gln Cys Ser Arg Ser Ser Pro Val Val Pro
   175                 180                 185                 190
   gtt gag caa gat gac act tct gca tca gct ctc act tgt gtc aac aac     1045
   Val Glu Gln Asp Asp Thr Ser Ala Ser Ala Leu Thr Cys Val Asn Asn
                   195                 200                 205
   cct gat gac gtc tcg acc gtt gct cca act gct cca act cca aat gtt     1093
   Pro Asp Asp Val Ser Thr Val Ala Pro Thr Ala Pro Thr Pro Asn Val
               210                 215                 220
   cct gct ggt gga aac aag gaa acg ttg ttc gat ttc gac ttt act aat     1141
   Pro Ala Gly Gly Asn Lys Glu Thr Leu Phe Asp Phe Asp Phe Thr Asn
           225                 230                 235
   cta cag atc cct gat ttt ggt ttc ttg gca gag gag caa caa gac cta     1189
   Leu Gln Ile Pro Asp Phe Gly Phe Leu Ala Glu Glu Gln Gln Asp Leu
       240                 245                 250
   gac ttc gat tgt ttc ctc gcg gat gat cag ttt gat gat ttc ggc ttg     1237
   Asp Phe Asp Cys Phe Leu Ala Asp Asp Gln Phe Asp Asp Phe Gly Leu
   255                 260                 265                 270
   ctt gat gac att caa gga ttc gaa gat aac ggt cca agt gcg tta cca     1285
   Leu Asp Asp Ile Gln Gly Phe Glu Asp Asn Gly Pro Ser Ala Leu Pro
                   275                 280                 285
   gat ttc gac ttt gcg gat gtt gaa gat ctt cag cta gct gac tct agt     1333
   Asp Phe Asp Phe Ala Asp Val Glu Asp Leu Gln Leu Ala Asp Ser Ser
               290                 295                 300
   ttc ggt ttc ctt gat caa ctt gct cct atc aac atc tct tgc cca tta     1381
   Phe Gly Phe Leu Asp Gln Leu Ala Pro Ile Asn Ile Ser Cys Pro Leu
           305                 310                 315
   aaa agt ttt gca gct tca tag gatcttgctt agtaatgtta agtgagaaga        1432
   Lys Ser Phe Ala Ala Ser
       320
   gtgttttgtt ttttcgttta tgctttagta atttaagaca tacaaaagtg tgtgttccgg   1492
   attgtagtaa gatcttaaga cataaagccg ggttttgcaa ttaggaatcg agttttaatg   1552
   aagttttagt ttatgtttg                                                1571
<210> 20
   <211> 324
   <212> PRT
   <213> Arabidopsis thaliana
<400> 20
   Met Val Ala Ile Arg Lys Glu Gln Ser Leu Ser Gly Val Ser Ser Glu
   1               5                   10                  15
   Ile Lys Lys Arg Ala Lys Arg Asn Thr Leu Ser Ser Leu Pro Gln Glu
               20                  25                  30
   Thr Gln Pro Leu Arg Lys Val Arg Ile Ile Val Asn Asp Pro Tyr Ala
           35                  40                  45
   Thr Asp Asp Ser Ser Ser Asp Glu Glu Glu Leu Lys Val Pro Lys Pro
       50                  55                  60
   Arg Lys Met Lys Arg Ile Val Arg Glu Ile Asn Phe Pro Ser Met Glu
   65                  70                  75                  80
   Val Ser Glu Gln Pro Ser Glu Ser Ser Ser Gln Asp Ser Thr Lys Thr
                   85                  90                  95
   Asp Gly Lys Ile Ala Val Ser Ala Ser Pro Ala Val Pro Arg Lys Lys
               100                 105                 110
   Pro Val Gly Val Arg Gln Arg Lys Trp Gly Lys Trp Ala Ala Glu Ile
           115                 120                 125
   Arg Asp Pro Ile Lys Lys Thr Arg Thr Trp Leu Gly Thr Phe Asp Thr
       130                 135                 140
   Leu Glu Glu Ala Ala Lys Ala Tyr Asp Ala Lys Lys Leu Glu Phe Asp
   145                 150                 155                 160
   Ala Ile Val Ala Gly Asn Val Ser Thr Thr Lys Arg Asp Val Ser Ser
                   165                 170                 175
   Ser Glu Thr Ser Gln Cys Ser Arg Ser Ser Pro Val Val Pro Val Glu
               180                 185                 190
   Gln Asp Asp Thr Ser Ala Ser Ala Leu Thr Cys Val Asn Asn Pro Asp
           195                 200                 205
   Asp Val Ser Thr Val Ala Pro Thr Ala Pro Thr Pro Asn Val Pro Ala
       210                 215                 220
   Gly Gly Asn Lys Glu Thr Leu Phe Asp Phe Asp Phe Thr Asn Leu Gln
   225 230 235 240
   Ile Pro Asp Phe Gly Phe Leu Ala Glu Glu Gln Gln Asp Leu Asp Phe
   245 250 255
   Asp Cys Phe Leu Ala Asp Asp Gln Phe Asp Asp Phe Gly Leu Leu Asp
   260 265 270
   Asp Ile Gln Gly Phe Glu Asp Asn Gly Pro Ser Ala Leu Pro Asp Phe
   275 280 285
   Asp Phe Ala Asp Val Glu Asp Leu Gln Leu Ala Asp Ser Ser Phe Gly
   290 295 300
   Phe Leu Asp Gln Leu Ala Pro Ile Asn Ile Ser Cys Pro Leu Lys Ser
   305 310 315 320
   Phe Ala Ala Ser
<210> 21
   <211> 1195
   <212> DNA
   <213> Arabidopsis thaliana
<220>
   <221> CDS
   <222> (67)..(1041)
   <223> G883
<400> 21
   ctctctcgtc ttcgtcttct tcttcttcaa cgttcctctc caaaatcctc agaccaagaa       60
   atcatc atg gcc gtc gat cta atg cgt ttc cct aag ata gat gat caa        108
          Met Ala Val Asp Leu Met Arg Phe Pro Lys Ile Asp Asp Gln
          1               5                   10
   acg gct att cag gaa gct gca tcg caa ggt tta caa agt atg gaa cat      156
   Thr Ala Ile Gln Glu Ala Ala Ser Gln Gly Leu Gln Ser Met Glu His
   15                  20                  25                  30
   ctg atc cgt gtc ctc tct aac cgt ccc gaa caa caa cac aac gtt gac      204
   Leu Ile Arg Val Leu Ser Asn Arg Pro Glu Gln Gln His Asn Val Asp
                   35                  40                  45
   tgc tcc gag atc act gac ttc acc gtt tct aaa ttc aaa acc gtc att      252
   Cys Ser Glu Ile Thr Asp Phe Thr Val Ser Lys Phe Lys Thr Val Ile
               50                  55                  60
   tct ctc ctt aac cgt act ggt cac gct cgg ttc aga cgc gga ccg gtt      300
   Ser Leu Leu Asn Arg Thr Gly His Ala Arg Phe Arg Arg Gly Pro Val
           65                  70                  75
   cac tcc act tcc tct gcc gca tct cag aaa cta cag agt cag atc gtt      348
   His Ser Thr Ser Ser Ala Ala Ser Gln Lys Leu Gln Ser Gln Ile Val
       80                  85                  90
   aaa aat act caa cct gag gct ccg ata gtg aga aca act acg aat cac      396
   Lys Asn Thr Gln Pro Glu Ala Pro Ile Val Arg Thr Thr Thr Asn His
   95                  100                 105                 110
   cct caa atc gtt cct cca ccg tct agt gta aca ctc gat ttc tct aaa      444
   Pro Gln Ile Val Pro Pro Pro Ser Ser Val Thr Leu Asp Phe Ser Lys
                   115                 120                 125
   cca agc atc ttc ggc acc aaa gct aag agc gcc gag ctg gaa ttc tcc      492
   Pro Ser Ile Phe Gly Thr Lys Ala Lys Ser Ala Glu Leu Glu Phe Ser
               130                 135                 140
   aaa gaa aac ttc agt gtt tct tta aac tcc tca ttc atg tcg tcg gcg      540
   Lys Glu Asn Phe Ser Val Ser Leu Asn Ser Ser Phe Met Ser Ser Ala
           145                 150                 155
   ata acc gga gac ggc agc gtc tcc aat gga aaa atc ttc ctt gct tct      588
   Ile Thr Gly Asp Gly Ser Val Ser Asn Gly Lys Ile Phe Leu Ala Ser
       160                 165                 170
   gct ccg tcg cag cct gtt aac tct tcc gga aaa cca ccg ttg gct ggt      636
   Ala Pro Ser Gln Pro Val Asn Ser Ser Gly Lys Pro Pro Leu Ala Gly
   175                 180                 185                 190
   cat cct tac aga aag aga tgt ctc gag cat gag cac tca gag agt ttc      684
   His Pro Tyr Arg Lys Arg Cys Leu Glu His Glu His Ser Glu Ser Phe
                   195                 200                 205
   tcc gga aaa gtc tcc ggc tcc gcc tac gga aag tgc cat tgc aag aaa      732
   Ser Gly Lys Val Ser Gly Ser Ala Tyr Gly Lys Cys His Cys Lys Lys
               210                 215                 220
   agg aaa aat cgg atg aag aga acc gtg aga gta ccg gcg ata agt gca      780
   Arg Lys Asn Arg Met Lys Arg Thr Val Arg Val Pro Ala Ile Ser Ala
           225                 230                 235
   aag atc gcc gat att cca ccg gac gaa tat tcg tgg agg aag tac gga      828
   Lys Ile Ala Asp Ile Pro Pro Asp Glu Tyr Ser Trp Arg Lys Tyr Gly
       240                 245                 250
   caa aaa ccg atc aag ggc tca cca cac cca cgt ggt tac tac aag tgc      876
   Gln Lys Pro Ile Lys Gly Ser Pro His Pro Arg Gly Tyr Tyr Lys Cys
   255                 260                 265                 270
   agt aca ttc aga gga tgt cca gcg agg aaa cac gtg gaa cga gca tta      924
   Ser Thr Phe Arg Gly Cys Pro Ala Arg Lys His Val Glu Arg Ala Leu
                   275                 280                 285
   gat gat cca gcg atg ctt att gtg aca tac gaa gga gag cac cgt cat      972
   Asp Asp Pro Ala Met Leu Ile Val Thr Tyr Glu Gly Glu His Arg His
               290                 295                 300
   aac caa tcc gcg atg cag gag aat att tct tct tca ggc att aat gat     1020
   Asn Gln Ser Ala Met Gln Glu Asn Ile Ser Ser Ser Gly Ile Asn Asp
           305                 310                 315
   tta gtg ttt gcc tcg gct tga cttttttttg tactatttgt tttttgattt        1071
   Leu Val Phe Ala Ser Ala
       320
   tttgagtact ttagatggat tgaaatttgt aaattttttt attaagaaat caatttaaat   1131
   agagaaaaat tagtggtggt gcaaaaaaaa aaaaaaaaaa aaaaaaaaaa aaaaaaaaaa   1191
   aaaa                                                                1195
<210> 22
   <211> 324
   <212> PRT
   <213> Arabidopsis thaliana
<400> 22
   Met Ala Val Asp Leu Met Arg Phe Pro Lys Ile Asp Asp Gln Thr Ala
   1               5                   10                  15
   Ile Gln Glu Ala Ala Ser Gln Gly Leu Gln Ser Met Glu His Leu Ile
               20                  25                  30
   Arg Val Leu Ser Asn Arg Pro Glu Gln Gln His Asn Val Asp Cys Ser
           35                  40                  45
   Glu Ile Thr Asp Phe Thr Val Ser Lys Phe Lys Thr Val Ile Ser Leu
       50                  55                  60
   Leu Asn Arg Thr Gly His Ala Arg Phe Arg Arg Gly Pro Val His Ser
   65                  70                  75                  80
   Thr Ser Ser Ala Ala Ser Gln Lys Leu Gln Ser Gln Ile Val Lys Asn
                   85                  90                  95
   Thr Gln Pro Glu Ala Pro Ile Val Arg Thr Thr Thr Asn His Pro Gln
               100                 105                 110
   Ile Val Pro Pro Pro Ser Ser Val Thr Leu Asp Phe Ser Lys Pro Ser
           115                 120                 125
   Ile Phe Gly Thr Lys Ala Lys Ser Ala Glu Leu Glu Phe Ser Lys Glu
       130                 135                 140
   Asn Phe Ser Val Ser Leu Asn Ser Ser Phe Met Ser Ser Ala Ile Thr
   145                 150                 155                 160
   Gly Asp Gly Ser Val Ser Asn Gly Lys Ile Phe Leu Ala Ser Ala Pro
                   165                 170                 175
   Ser Gln Pro Val Asn Ser Ser Gly Lys Pro Pro Leu Ala Gly His Pro
               180                 185                 190
   Tyr Arg Lys Arg Cys Leu Glu His Glu His Ser Glu Ser Phe Ser Gly
           195                 200                 205
   Lys Val Ser Gly Ser Ala Tyr Gly Lys Cys His Cys Lys Lys Arg Lys
       210                 215                 220
   Asn Arg Met Lys Arg Thr Val Arg Val Pro Ala Ile Ser Ala Lys Ile
   225                 230                 235                 240
   Ala Asp Ile Pro Pro Asp Glu Tyr Ser Trp Arg Lys Tyr Gly Gln Lys
                   245                 250                 255
   Pro Ile Lys Gly Ser Pro His Pro Arg Gly Tyr Tyr Lys Cys Ser Thr
               260                 265                 270
   Phe Arg Gly Cys Pro Ala Arg Lys His Val Glu Arg Ala Leu Asp Asp
           275                 280                 285
   Pro Ala Met Leu Ile Val Thr Tyr Glu Gly Glu His Arg His Asn Gln
       290                 295                 300
   Ser Ala Met Gln Glu Asn Ile Ser Ser Ser Gly Ile Asn Asp Leu Val
   305                 310                 315                 320
   Phe Ala Ser Ala
<210> 23
   <211> 1755
   <212> DNA
   <213> Arabidopsis thaliana
<220>
   <221> CDS
   <222> (1)..(1755)
   <223> G938
<400> 23
   atg atg atg ttt aac gag atg gga atg tat gga aac atg gat ttc ttc       48
   Met Met Met Phe Asn Glu Met Gly Met Tyr Gly Asn Met Asp Phe Phe
   1               5                   10                  15
   tct tcc tcc aca tct ctc gat gtg tgt cca tta cca caa gct gaa caa       96
   Ser Ser Ser Thr Ser Leu Asp Val Cys Pro Leu Pro Gln Ala Glu Gln
               20                  25                  30
   gaa cct gta gtt gaa gat gtc gac tac acc gat gat gag atg gat gtg      144
   Glu Pro Val Val Glu Asp Val Asp Tyr Thr Asp Asp Glu Met Asp Val
           35                  40                  45
   gat gag ctt gag aag agg atg tgg aga gac aaa atg cgt ttg aaa cgt      192
   Asp Glu Leu Glu Lys Arg Met Trp Arg Asp Lys Met Arg Leu Lys Arg
       50                  55                  60
   ctc aag gag caa cag agt aag tgt aaa gaa ggc gtc gat ggt tcg aaa      240
   Leu Lys Glu Gln Gln Ser Lys Cys Lys Glu Gly Val Asp Gly Ser Lys
   65                  70                  75                  80
   cag agg cag tcg caa gag caa gct agg agg aag aaa atg tct aga gcc      288
   Gln Arg Gln Ser Gln Glu Gln Ala Arg Arg Lys Lys Met Ser Arg Ala
                   85                  90                  95
   caa gat ggg atc ttg aag tat atg ttg aag atg atg gaa gtt tgt aaa      336
   Gln Asp Gly Ile Leu Lys Tyr Met Leu Lys Met Met Glu Val Cys Lys
               100                 105                 110
   gct caa ggc ttt gtt tat ggt att att cct gag aag ggt aag cct gtg      384
   Ala Gln Gly Phe Val Tyr Gly Ile Ile Pro Glu Lys Gly Lys Pro Val
           115                 120                 125
   act ggt gct tcg gat aat ttg agg gaa tgg tgg aaa gat aag gtt agg      432
   Thr Gly Ala Ser Asp Asn Leu Arg Glu Trp Trp Lys Asp Lys Val Arg
       130                 135                 140
   ttt gat cgt aat ggt cca gct gct att gct aag tat cag tca gag aat      480
   Phe Asp Arg Asn Gly Pro Ala Ala Ile Ala Lys Tyr Gln Ser Glu Asn
   145                 150                 155                 160
   aat att tct gga ggg agt aat gat tgt aac agc ttg gtt ggt cca aca      528
   Asn Ile Ser Gly Gly Ser Asn Asp Cys Asn Ser Leu Val Gly Pro Thr
                   165                 170                 175
   ccg cat acg ctt cag gag ctt cag gac acg act ctt ggt tcg ctt tta      576
   Pro His Thr Leu Gln Glu Leu Gln Asp Thr Thr Leu Gly Ser Leu Leu
               180                 185                 190
   tcg gct ttg atg caa cat tgt gat cca ccg cag aga cgg ttt cct ttg      624
   Ser Ala Leu Met Gln His Cys Asp Pro Pro Gln Arg Arg Phe Pro Leu
           195                 200                 205
   gag aaa gga gtt tct cca cct tgg tgg cct aat ggg aat gaa gag tgg      672
   Glu Lys Gly Val Ser Pro Pro Trp Trp Pro Asn Gly Asn Glu Glu Trp
       210                 215                 220
   tgg cct cag ctt ggt tta cca aat gag caa ggt cct cct cct tat aag      720
   Trp Pro Gln Leu Gly Leu Pro Asn Glu Gln Gly Pro Pro Pro Tyr Lys
   225                 230                 235                 240
   aag cct cat gat ttg aag aaa gct tgg aaa gtc ggt gtt tta act gcg      768
   Lys Pro His Asp Leu Lys Lys Ala Trp Lys Val Gly Val Leu Thr Ala
                   245                 250                 255
   gtg atc aag cat atg tcg ccg gat att gcg aag atc cgt aag ctt gtg      816
   Val Ile Lys His Met Ser Pro Asp Ile Ala Lys Ile Arg Lys Leu Val
               260                 265                 270
   agg caa tca aaa tgc ttg cag gat aag atg acg gcg aaa gag agt gct      864
   Arg Gln Ser Lys Cys Leu Gln Asp Lys Met Thr Ala Lys Glu Ser Ala
           275                 280                 285
   act tgg ctt gcc att att aac caa gaa gag gtt gtg gct cgg gag ctt      912
   Thr Trp Leu Ala Ile Ile Asn Gln Glu Glu Val Val Ala Arg Glu Leu
       290                 295                 300
   tat ccc gag tca tgc cct cct ctt tct tct tct tca tca tta gga agc      960
   Tyr Pro Glu Ser Cys Pro Pro Leu Ser Ser Ser Ser Ser Leu Gly Ser
   305                 310                 315                 320
   ggg tcg ctt ctc att aat gat tgt agc gag tat gac gtt gaa ggt ttc     1008
   Gly Ser Leu Leu Ile Asn Asp Cys Ser Glu Tyr Asp Val Glu Gly Phe
                   325                 330                 335
   gag aag gaa caa cat ggt ttc gat gtg gaa gag cgg aaa cca gag ata     1056
   Glu Lys Glu Gln His Gly Phe Asp Val Glu Glu Arg Lys Pro Glu Ile
               340                 345                 350
   gtg atg atg cat cct cta gca agc ttt ggg gtt gct aaa atg caa cat     1104
   Val Met Met His Pro Leu Ala Ser Phe Gly Val Ala Lys Met Gln His
           355                 360                 365
   ttt ccc ata aag gag gag gtc gcc acc acg gta aac tta gag ttc acg     1152
   Phe Pro Ile Lys Glu Glu Val Ala Thr Thr Val Asn Leu Glu Phe Thr
       370                 375                 380
   aga aag agg aag cag aac aat gat atg aat gtt atg gta atg gac aga     1200
   Arg Lys Arg Lys Gln Asn Asn Asp Met Asn Val Met Val Met Asp Arg
   385                 390                 395                 400
   tca gca ggt tac act tgt gag aat ggt cag tgt cct cac agc aaa atg     1248
   Ser Ala Gly Tyr Thr Cys Glu Asn Gly Gln Cys Pro His Ser Lys Met
                   405                 410                 415
   aat ctt gga ttt caa gac agg agt tca agg gac aac cac cag atg gtt     1296
   Asn Leu Gly Phe Gln Asp Arg Ser Ser Arg Asp Asn His Gln Met Val
               420                 425                 430
   tgt cca tat aga gac aat cgt tta gcg tat gga gca tcc aag ttt cat     1344
   Cys Pro Tyr Arg Asp Asn Arg Leu Ala Tyr Gly Ala Ser Lys Phe His
           435                 440                 445
   atg ggt gga atg aaa cta gta gtt cct cag caa cca gtc caa ccg atc     1392
   Met Gly Gly Met Lys Leu Val Val Pro Gln Gln Pro Val Gln Pro Ile
       450                 455                 460
   gac cta tcg ggc gtt gga gtt ccg gaa aac ggg cag aag atg atc acc     1440
   Asp Leu Ser Gly Val Gly Val Pro Glu Asn Gly Gln Lys Met Ile Thr
   465                 470                 475                 480
   gag ctt atg gcc atg tac gac aga aat gtc caa agc aac caa acg cct     1488
   Glu Leu Met Ala Met Tyr Asp Arg Asn Val Gln Ser Asn Gln Thr Pro
                   485                 490                 495
   cct act ttg atg gaa aac caa agc atg gtc att gat gca aaa gca gct     1536
   Pro Thr Leu Met Glu Asn Gln Ser Met Val Ile Asp Ala Lys Ala Ala
               500                 505                 510
   cag aat cag cag ctg aat ttc aac agt ggc aat caa atg ttt atg caa     1584
   Gln Asn Gln Gln Leu Asn Phe Asn Ser Gly Asn Gln Met Phe Met Gln
           515                 520                 525
   caa ggg acg aac aac ggg gtt aac aat cgg ttc cag atg gtg ttt gat     1632
   Gln Gly Thr Asn Asn Gly Val Asn Asn Arg Phe Gln Met Val Phe Asp
       530                 535                 540
   tcg aca cca ttc gat atg gca gca ttc gat tac aga gat gat tgg caa     1680
   Ser Thr Pro Phe Asp Met Ala Ala Phe Asp Tyr Arg Asp Asp Trp Gln
   545                 550                 555                 560
   acc gga gca atg gaa gga atg ggg aag cag cag cag cag cag cag cag      1728
   Thr Gly Ala Met Glu Gly Met Gly Lys Gln Gln Gln Gln Gln Gln Gln
                   565                 570                 575
   cag caa gat gta tca ata tgg ttc tga                                  1755
   Gln Gln Asp Val Ser Ile Trp Phe
               580
<210> 24
   <211> 584
   <212> PRT
   <213> Arabidopsis thaliana
<400> 24
   Met Met Met Phe Asn Glu Met Gly Met Tyr Gly Asn Met Asp Phe Phe
   1               5                   10                  15
   Ser Ser Ser Thr Ser Leu Asp Val Cys Pro Leu Pro Gln Ala Glu Gln
               20                  25                  30
   Glu Pro Val Val Glu Asp Val Asp Tyr Thr Asp Asp Glu Met Asp Val
           35                  40                  45
   Asp Glu Leu Glu Lys Arg Met Trp Arg Asp Lys Met Arg Leu Lys Arg
       50                  55                  60
   Leu Lys Glu Gln Gln Ser Lys Cys Lys Glu Gly Val Asp Gly Ser Lys
   65                  70                  75                  80
   Gln Arg Gln Ser Gln Glu Gln Ala Arg Arg Lys Lys Met Ser Arg Ala
                   85                  90                  95
   Gln Asp Gly Ile Leu Lys Tyr Met Leu Lys Met Met Glu Val Cys Lys
               100                 105                 110
   Ala Gln Gly Phe Val Tyr Gly Ile Ile Pro Glu Lys Gly Lys Pro Val
           115                 120                 125
   Thr Gly Ala Ser Asp Asn Leu Arg Glu Trp Trp Lys Asp Lys Val Arg
       130                 135                 140
   Phe Asp Arg Asn Gly Pro Ala Ala Ile Ala Lys Tyr Gln Ser Glu Asn
   145                 150                 155                 160
   Asn Ile Ser Gly Gly Ser Asn Asp Cys Asn Ser Leu Val Gly Pro Thr
                   165                 170                 175
   Pro His Thr Leu Gln Glu Leu Gln Asp Thr Thr Leu Gly Ser Leu Leu
               180                 185                 190
   Ser Ala Leu Met Gln His Cys Asp Pro Pro Gln Arg Arg Phe Pro Leu
           195                 200                 205
   Glu Lys Gly Val Ser Pro Pro Trp Trp Pro Asn Gly Asn Glu Glu Trp
       210                 215                 220
   Trp Pro Gln Leu Gly Leu Pro Asn Glu Gln Gly Pro Pro Pro Tyr Lys
   225                 230                 235                 240
   Lys Pro His Asp Leu Lys Lys Ala Trp Lys Val Gly Val Leu Thr Ala
                   245                 250                 255
   Val Ile Lys His Met Ser Pro Asp Ile Ala Lys Ile Arg Lys Leu Val
               260                 265                 270
   Arg Gln Ser Lys Cys Leu Gln Asp Lys Met Thr Ala Lys Glu Ser Ala
           275                 280                 285
   Thr Trp Leu Ala Ile Ile Asn Gln Glu Glu Val Val Ala Arg Glu Leu
       290                 295                 300
   Tyr Pro Glu Ser Cys Pro Pro Leu Ser Ser Ser Ser Ser Leu Gly Ser
   305                 310                 315                 320
   Gly Ser Leu Leu Ile Asn Asp Cys Ser Glu Tyr Asp Val Glu Gly Phe
                   325                 330                 335
   Glu Lys Glu Gln His Gly Phe Asp Val Glu Glu Arg Lys Pro Glu Ile
               340                 345                 350
   Val Met Met His Pro Leu Ala Ser Phe Gly Val Ala Lys Met Gln His
           355                 360                 365
   Phe Pro Ile Lys Glu Glu Val Ala Thr Thr Val Asn Leu Glu Phe Thr
       370                 375                 380
   Arg Lys Arg Lys Gln Asn Asn Asp Met Asn Val Met Val Met Asp Arg
   385                 390                 395                 400
   Ser Ala Gly Tyr Thr Cys Glu Asn Gly Gln Cys Pro His Ser Lys Met
                   405                 410                 415
   Asn Leu Gly Phe Gln Asp Arg Ser Ser Arg Asp Asn His Gln Met Val
               420                 425                 430
   Cys Pro Tyr Arg Asp Asn Arg Leu Ala Tyr Gly Ala Ser Lys Phe His
           435                 440                 445
   Met Gly Gly Met Lys Leu Val Val Pro Gln Gln Pro Val Gln Pro Ile
       450                 455                 460
   Asp Leu Ser Gly Val Gly Val Pro Glu Asn Gly Gln Lys Met Ile Thr
   465                 470                 475                 480
   Glu Leu Met Ala Met Tyr Asp Arg Asn Val Gln Ser Asn Gln Thr Pro
                   485                 490                 495
   Pro Thr Leu Met Glu Asn Gln Ser Met Val Ile Asp Ala Lys Ala Ala
               500                 505                 510
   Gln Asn Gln Gln Leu Asn Phe Asn Ser Gly Asn Gln Met Phe Met Gln
           515                 520                 525
   Gln Gly Thr Asn Asn Gly Val Asn Asn Arg Phe Gln Met Val Phe Asp
       530                 535                     540
   Ser Thr Pro Phe Asp Met Ala Ala Phe Asp Tyr Arg Asp Asp Trp Gln
   545                 550                     555                 560
   Thr Gly Ala Met Glu Gly Met Gly Lys Gln Gln Gln Gln Gln Gln Gln
                   565                     570                     575
   Gln Gln Asp Val Ser Ile Trp Phe
               580
<210> 25
   <211> 1161
   <212> DNA
   <213> Arabidopsis thaliana
<220>
   <221> CDS
   <222> (67)..(1041)
   <223> G1328
<400> 25
   aattcaatca ctatattttt ttaaaaacat ttgacttcat cgatcggtta acaattaatc      60
   aaaaag atg gga cga tca cca tgt tgt gag aag aag aat ggt ctc aag        108
          Met Gly Arg Ser Pro Cys Cys Glu Lys Lys Asn Gly Leu Lys
          1               5                   10
   aaa gga cca tgg act cct gag gag gat caa aag ctc att gat tat atc       156
   Lys Gly Pro Trp Thr Pro Glu Glu Asp Gln Lys Leu Ile Asp Tyr Ile
   15                  20                  25                  30
   aat ata cat ggt tat gga aat tgg aga act ctt ccc aag aat gct ggg       204
   Asn Ile His Gly Tyr Gly Asn Trp Arg Thr Leu Pro Lys Asn Ala Gly
                   35                  40                  45
   tta caa aga tgt ggt aag agt tgt cgt ctc cgg tgg acc aac tat ctc       252
   Leu Gln Arg Cys Gly Lys Ser Cys Arg Leu Arg Trp Thr Asn Tyr Leu
               50                  55                  60
   cga cca gat att aag cgt gga aga ttc tct ttt gaa gaa gaa gaa acc       300
   Arg Pro Asp Ile Lys Arg Gly Arg Phe Ser Phe Glu Glu Glu Glu Thr
           65                  70                  75
   att att caa ctt cac agc atc atg gga aac aag tgg tct gcg att gcg       348
   Ile Ile Gln Leu His Ser Ile Met Gly Asn Lys Trp Ser Ala Ile Ala
       80                  85                  90
   gct cgt ttg cct gga aga aca gac aac gag atc aaa aac tat tgg aac       396
   Ala Arg Leu Pro Gly Arg Thr Asp Asn Glu Ile Lys Asn Tyr Trp Asn
   95                  100                 105                 110
   act cac atc aga aaa aga ctt cta aag atg gga atc gac ccg gtt aca       444
   Thr His Ile Arg Lys Arg Leu Leu Lys Met Gly Ile Asp Pro Val Thr
                   115                 120                 125
   cac act cca cgt ctt gat ctt ctc gat atc tcc tcc att ctc agc tca       492
   His Thr Pro Arg Leu Asp Leu Leu Asp Ile Ser Ser Ile Leu Ser Ser
               130                 135                 140
   tct atc tac aac tct tcg cat cat cat cat cat cat cat caa caa cat       540
   Ser Ile Tyr Asn Ser Ser His His His His His His His Gln Gln His
           145                 150                 155
   atg aac atg tcg agg ctc atg atg agt gat ggt aat cat caa cca ttg       588
   Met Asn Met Ser Arg Leu Met Met Ser Asp Gly Asn His Gln Pro Leu
       160                 165                 170
   gtt aac ccc gag ata ctc aaa ctc gca acc tct ctc ttt tca aac caa        636
   Val Asn Pro Glu Ile Leu Lys Leu Ala Thr Ser Leu Phe Ser Asn Gln
   175                 180                 185                 190
   aac cac ccc aac aac aca cac gag aac aac acg gtt aac caa acc gaa        684
   Asn His Pro Asn Asn Thr His Glu Asn Asn Thr Val Asn Gln Thr Glu
                   195                 200                 205
   gta aac caa tac caa acc ggt tac aac atg cct ggt aat gaa gaa tta        732
   Val Asn Gln Tyr Gln Thr Gly Tyr Asn Met Pro Gly Asn Glu Glu Leu
               210                 215                 220
   caa tct tgg ttc cct atc atg gat caa ttc acg aat ttc caa gac ctc        780
   Gln Ser Trp Phe Pro Ile Met Asp Gln Phe Thr Asn Phe Gln Asp Leu
           225                 230                 235
   atg cca atg aag acg acg gtc caa aat tca ttg tca tac gat gat gat        828
   Met Pro Met Lys Thr Thr Val Gln Asn Ser Leu Ser Tyr Asp Asp Asp
       240                 245                 250
   tgt tcg aag tcc aat ttt gta tta gaa cct tat tac tcc gac ttt gct        876
   Cys Ser Lys Ser Asn Phe Val Leu Glu Pro Tyr Tyr Ser Asp Phe Ala
   255                 260                 265                 270
   tca gtc ttg acc aca cct tct tca agc ccg act ccg tta aac tca agt        924
   Ser Val Leu Thr Thr Pro Ser Ser Ser Pro Thr Pro Leu Asn Ser Ser
                   275                 280                 285
   tcc tca act tac atc aat agt agc act tgc agc acc gag gat gaa aaa        972
   Ser Ser Thr Tyr Ile Asn Ser Ser Thr Cys Ser Thr Glu Asp Glu Lys
               290                 295                 300
   gag agt tat tac agt gat aat atc act aat tat tcg ttt gat gtt aat       1020
   Glu Ser Tyr Tyr Ser Asp Asn Ile Thr Asn Tyr Ser Phe Asp Val Asn
           305                 310                 315
   ggt ttt ctc caa ttc caa taa acaaaacgcc attggaatag agttatgtaa         1071
   Gly Phe Leu Gln Phe Gln
       320
   acatgcaatc attgtatttg ttatatagat tttgttacat atccaaaatc caaaatacta    1131
   tagttttaaa ataaaaaaaa aaaaaaaaaa                                       1161
<210> 26
   <211> 324
   <212> PRT
   <213> Arabidopsis thaliana
<400> 26
   Met Gly Arg Ser Pro Cys Cys Glu Lys Lys Asn Gly Leu Lys Lys Gly
   1               5                   10                  15
   Pro Trp Thr Pro Glu Glu Asp Gln Lys Leu Ile Asp Tyr Ile Asn Ile
               20                  25                  30
   His Gly Tyr Gly Asn Trp Arg Thr Leu Pro Lys Asn Ala Gly Leu Gln
           35                  40                  45
   Arg Cys Gly Lys Ser Cys Arg Leu Arg Trp Thr Asn Tyr Leu Arg Pro
       50                  55                  60
   Asp Ile Lys Arg Gly Arg Phe Ser Phe Glu Glu Glu Glu Thr Ile Ile
   65                  70                  75                  80
   Gln Leu His Ser Ile Met Gly Asn Lys Trp Ser Ala Ile Ala Ala Arg
                   85                  90                  95
   Leu Pro Gly Arg Thr Asp Asn Glu Ile Lys Asn Tyr Trp Asn Thr His
               100                 105                 110
   Ile Arg Lys Arg Leu Leu Lys Met Gly Ile Asp Pro Val Thr His Thr
           115                 120                 125
   Pro Arg Leu Asp Leu Leu Asp Ile Ser Ser Ile Leu Ser Ser Ser Ile
       130                 135                 140
   Tyr Asn Ser Ser His His His His His His His Gln Gln His Met Asn
   145                 150                 155                 160
   Met Ser Arg Leu Met Met Ser Asp Gly Asn His Gln Pro Leu Val Asn
                   165                 170                 175
   Pro Glu Ile Leu Lys Leu Ala Thr Ser Leu Phe Ser Asn Gln Asn His
               180                 185                 190
   Pro Asn Asn Thr His Glu Asn Asn Thr Val Asn Gln Thr Glu Val Asn
           195                 200                 205
   Gln Tyr Gln Thr Gly Tyr Asn Met Pro Gly Asn Glu Glu Leu Gln Ser
       210                 215                 220
   Trp Phe Pro Ile Met Asp Gln Phe Thr Asn Phe Gln Asp Leu Met Pro
   225                 230                 235                 240
   Met Lys Thr Thr Val Gln Asn Ser Leu Ser Tyr Asp Asp Asp Cys Ser
                   245                 250                 255
   Lys Ser Asn Phe Val Leu Glu Pro Tyr Tyr Ser Asp Phe Ala Ser Val
               260                 265                 270
   Leu Thr Thr Pro Ser Ser Ser Pro Thr Pro Leu Asn Ser Ser Ser Ser
           275                 280                 285
   Thr Tyr Ile Asn Ser Ser Thr Cys Ser Thr Glu Asp Glu Lys Glu Ser
       290                 295                 300
   Tyr Tyr Ser Asp Asn Ile Thr Asn Tyr Ser Phe Asp Val Asn Gly Phe
   305                 310                 315                 320
   Leu Gln Phe Gln
<210> 27
   <211> 2162
   <212> DNA
   <213> Arabidopsis thaliana
<220>
   <221> CDS
   <222> (40)..(1809)
   <223> G584
<400> 27
   aaaaagtctt ctcttttata actacgtcag agaactgtt atg tct ccg acg aat         54
                                              Met Ser Pro Thr Asn
                                                     1               5
   gtt caa gta acc gat tac cat ctc aac caa tca aaa acg gat aca aca      102
   Val Gln Val Thr Asp Tyr His Leu Asn Gln Ser Lys Thr Asp Thr Thr
                   10                  15                  20
   aat ctc tgg tca acc gac gac gat gca tcg gta atg gaa gct ttc atc      150
   Asn Leu Trp Ser Thr Asp Asp Asp Ala Ser Val Met Glu Ala Phe Ile
               25                  30                  35
   ggc ggc ggc tcc gat cat tct tct ctt ttt cct cca ctt cct cct cct      198
   Gly Gly Gly Ser Asp His Ser Ser Leu Phe Pro Pro Leu Pro Pro Pro
           40                  45                  50
   cct ctt cct caa gtc aac gaa gat aat ctc cag caa cgt ctc caa gct      246
   Pro Leu Pro Gln Val Asn Glu Asp Asn Leu Gln Gln Arg Leu Gln Ala
       55                  60                  65
   tta atc gaa gga gca aac gag aac tgg act tac gcc gtg ttc tgg caa      294
   Leu Ile Glu Gly Ala Asn Glu Asn Trp Thr Tyr Ala Val Phe Trp Gln
   70                  75                  80                  85
   tca tct cac ggt ttc gcc gga gaa gac aac aac aac aac aac aca gtg      342
   Ser Ser His Gly Phe Ala Gly Glu Asp Asn Asn Asn Asn Asn Thr Val
                   90                  95                  100
   ttg tta ggt tgg gga gat ggt tat tac aaa gga gaa gaa gag aag tct      390
   Leu Leu Gly Trp Gly Asp Gly Tyr Tyr Lys Gly Glu Glu Glu Lys Ser
               105                 110                 115
   aga aag aag aaa tca aat cca gct agt gca gct gaa caa gag cat cgt      438
   Arg Lys Lys Lys Ser Asn Pro Ala Ser Ala Ala Glu Gln Glu His Arg
           120                 125                 130
   aag aga gtg att aga gag ctc aac tct tta atc tcc ggt ggt gta gga      486
   Lys Arg Val Ile Arg Glu Leu Asn Ser Leu Ile Ser Gly Gly Val Gly
       135                 140                 145
   gga gga gat gaa gct gga gat gaa gaa gtt aca gat act gaa tgg ttc      534
   Gly Gly Asp Glu Ala Gly Asp Glu Glu Val Thr Asp Thr Glu Trp Phe
   150                 155                 160                 165
   ttc tta gtt tca atg aca cag agc ttt gtc aag ggt act ggt tta cct      582
   Phe Leu Val Ser Met Thr Gln Ser Phe Val Lys Gly Thr Gly Leu Pro
                   170                 175                 180
   ggt caa gct ttc tca aat tca gac acg att tgg tta tct ggt tct aat      630
   Gly Gln Ala Phe Ser Asn Ser Asp Thr Ile Trp Leu Ser Gly Ser Asn
               185                 190                 195
   gct tta gct gga tca agt tgt gag aga gct cgt caa ggt cag att tat      678
   Ala Leu Ala Gly Ser Ser Cys Glu Arg Ala Arg Gln Gly Gln Ile Tyr
           200                 205                 210
   ggg tta caa aca atg gtg tgt gta gcg aca gag aat ggt gtc gtt gag      726
   Gly Leu Gln Thr Met Val Cys Val Ala Thr Glu Asn Gly Val Val Glu
       215                 220                 225
   ctt ggt tcg tcg gag att att cat caa agt tca gat ctt gtt gat aaa      774
   Leu Gly Ser Ser Glu Ile Ile His Gln Ser Ser Asp Leu Val Asp Lys
   230                 235                 240                 245
   gtt gac acc ttt ttc aat ttt aac aat ggt ggt ggt gaa ttt ggt tct      822
   Val Asp Thr Phe Phe Asn Phe Asn Asn Gly Gly Gly Glu Phe Gly Ser
                   250                 255                 260
   tgg gcg ttt aat ttg aat cca gat caa gga gag aat gat cca ggt ttg      870
   Trp Ala Phe Asn Leu Asn Pro Asp Gln Gly Glu Asn Asp Pro Gly Leu
               265                 270                 275
   tgg att agt gaa cct aat ggt gtt gac tct ggt ctt gta gct gct ccg      918
   Trp Ile Ser Glu Pro Asn Gly Val Asp Ser Gly Leu Val Ala Ala Pro
           280                 285                 290
   gtg atg aat aat ggt gga aat gac tca act tct aat tct gat tct caa      966
   Val Met Asn Asn Gly Gly Asn Asp Ser Thr Ser Asn Ser Asp Ser Gln
       295                 300                 305
   cca att tct aag ctt tgt aat gga agc tct gtt gaa aac cct aac cct      1014
   Pro Ile Ser Lys Leu Cys Asn Gly Ser Ser Val Glu Asn Pro Asn Pro
   310                 315                 320                 325
   aaa gtt ctg aaa tct tgt gaa atg gtg aat ttc aag aat ggg att gag      1062
   Lys Val Leu Lys Ser Cys Glu Met Val Asn Phe Lys Asn Gly Ile Glu
                   330                 335                 340
   aat ggt caa gaa gaa gat agt agt aat aag aag aga tca ccg gtt tcg      1110
   Asn Gly Gln Glu Glu Asp Ser Ser Asn Lys Lys Arg Ser Pro Val Ser
               345                 350                 355
   aat aat gaa gaa ggg atg ctt tct ttt acc tct gtt ctt cca tgt gac      1158
   Asn Asn Glu Glu Gly Met Leu Ser Phe Thr Ser Val Leu Pro Cys Asp
           360                 365                 370
   tcg aat cac tct gat ctt gaa gct tca gtg gct aaa gaa gct gag agt      1206
   Ser Asn His Ser Asp Leu Glu Ala Ser Val Ala Lys Glu Ala Glu Ser
       375                 380                 385
   aac aga gtt gtg gtt gaa ccg gag aag aaa ccg agg aaa cga ggg aga      1254
   Asn Arg Val Val Val Glu Pro Glu Lys Lys Pro Arg Lys Arg Gly Arg
   390                 395                 400                 405
   aaa ccg gcg aat gga aga gaa gag cct ttg aat cat gta gag gca gag      1302
   Lys Pro Ala Asn Gly Arg Glu Glu Pro Leu Asn His Val Glu Ala Glu
                   410                 415                 420
   aga cag aga aga gag aag ttg aat cag aga ttc tat tct tta aga gct      1350
   Arg Gln Arg Arg Glu Lys Leu Asn Gln Arg Phe Tyr Ser Leu Arg Ala
               425                 430                 435
   gtg gtt cct aat gtg tct aag atg gat aaa gct tct cta tta gga gat      1398
   Val Val Pro Asn Val Ser Lys Met Asp Lys Ala Ser Leu Leu Gly Asp
           440                 445                 450
   gct att tcg tat atc agt gag ctt aag tct aag ttg caa aag gct gaa      1446
   Ala Ile Ser Tyr Ile Ser Glu Leu Lys Ser Lys Leu Gln Lys Ala Glu
       455                 460                 465
   tct gat aaa gaa gag ttg cag aag cag att gat gtg atg aat aaa gaa      1494
   Ser Asp Lys Glu Glu Leu Gln Lys Gln Ile Asp Val Met Asn Lys Glu
   470                 475                 480                 485
   gcg gga aat gcg aaa agt tcg gta aaa gat cga aaa tgt ttg aat caa      1542
   Ala Gly Asn Ala Lys Ser Ser Val Lys Asp Arg Lys Cys Leu Asn Gln
                   490                 495                 500
   gaa tcg agt gtg ttg ata gag atg gag gtt gat gtg aag att att ggt      1590
   Glu Ser Ser Val Leu Ile Glu Met Glu Val Asp Val Lys Ile Ile Gly
               505                 510                 515
   tgg gat gca atg ata agg att caa tgt agt aag agg aat cat cct ggt      1638
   Trp Asp Ala Met Ile Arg Ile Gln Cys Ser Lys Arg Asn His Pro Gly
           520                 525                 530
   gct aag ttc atg gaa gca ctt aag gag ttg gat ttg gaa gtg aat cat      1686
   Ala Lys Phe Met Glu Ala Leu Lys Glu Leu Asp Leu Glu Val Asn His
       535                 540                 545
   gcg agt tta tcg gta gtg aat gat ctt atg atc caa caa gcg act gtg      1734
   Ala Ser Leu Ser Val Val Asn Asp Leu Met Ile Gln Gln Ala Thr Val
   550                 555                 560                 565
   aaa atg ggg aat cag ttt ttc acg caa gat caa ctc aag gtt gct cta      1782
   Lys Met Gly Asn Gln Phe Phe Thr Gln Asp Gln Leu Lys Val Ala Leu
                   570                 575                 580
   acg gag aaa gtt gga gaa tgt cca tga attgaagtca gcatctttag            1829
   Thr Glu Lys Val Gly Glu Cys Pro
               585
   ggctaataca ccggagaata ctgcgaaaag tcgaaaacaa cgatcatagt ataagccgcg   1889
   gtaaaaagtg ttaaaccttt cacacaagtt tctctagtga atgtagttgt aaactctatt   1949
   gtgtaagggt aattttgtag tacccacttg ttgctattga atgcttgtta gagaggattc   2009
   ttagtgtagt atatgattag gttggggttt gttgtttcat gagataaata aatgtgtttg   2069
   atcaatggtt aagtctttgg tttgttggtg tatgtatgta aataaggctt ttgttagaaa   2129
   taagacaaat gggactgaag ttggagttta aaa                                2162
<210> 28
   <211> 589
   <212> PRT
   <213> Arabidopsis thaliana
<400> 28
   Met Ser Pro Thr Asn Val Gln Val Thr Asp Tyr His Leu Asn Gln Ser
   1 5 10 15
   Lys Thr Asp Thr Thr Asn Leu Trp Ser Thr Asp Asp Asp Ala Ser Val
   20 25 30
   Met Glu Ala Phe Ile Gly Gly Gly Ser Asp His Ser Ser Leu Phe Pro
   35 40 45
   Pro Leu Pro Pro Pro Pro Leu Pro Gln Val Asn Glu Asp Asn Leu Gln
   50 55 60
   Gln Arg Leu Gln Ala Leu Ile Glu Gly Ala Asn Glu Asn Trp Thr Tyr
   65 70 75 80
   Ala Val Phe Trp Gln Ser Ser His Gly Phe Ala Gly Glu Asp Asn Asn
   85 90 95
   Asn Asn Asn Thr Val Leu Leu Gly Trp Gly Asp Gly Tyr Tyr Lys Gly
   100 105 110
   Glu Glu Glu Lys Ser Arg Lys Lys Lys Ser Asn Pro Ala Ser Ala Ala
   115 120 125
   Glu Gln Glu His Arg Lys Arg Val Ile Arg Glu Leu Asn Ser Leu Ile
   130 135 140
   Ser Gly Gly Val Gly Gly Gly Asp Glu Ala Gly Asp Glu Glu Val Thr
   145 150 155 160
   Asp Thr Glu Trp Phe Phe Leu Val Ser Met Thr Gln Ser Phe Val Lys
   165 170 175
   Gly Thr Gly Leu Pro Gly Gln Ala Phe Ser Asn Ser Asp Thr Ile Trp
   180 185 190
   Leu Ser Gly Ser Asn Ala Leu Ala Gly Ser Ser Cys Glu Arg Ala Arg
   195 200 205
   Gln Gly Gln Ile Tyr Gly Leu Gln Thr Met Val Cys Val Ala Thr Glu
   210 215 220
   Asn Gly Val Val Glu Leu Gly Ser Ser Glu Ile Ile His Gln Ser Ser
   225 230 235 240
   Asp Leu Val Asp Lys Val Asp Thr Phe Phe Asn Phe Asn Asn Gly Gly
   245 250 255
   Gly Glu Phe Gly Ser Trp Ala Phe Asn Leu Asn Pro Asp Gln Gly Glu
   260 265 270
   Asn Asp Pro Gly Leu Trp Ile Ser Glu Pro Asn Gly Val Asp Ser Gly
   275 280 285
   Leu Val Ala Ala Pro Val Met Asn Asn Gly Gly Asn Asp Ser Thr Ser
   290 295 300
   Asn Ser Asp Ser Gln Pro Ile Ser Lys Leu Cys Asn Gly Ser Ser Val
   305 310 315 320
   Glu Asn Pro Asn Pro Lys Val Leu Lys Ser Cys Glu Met Val Asn Phe
   325 330 335
   Lys Asn Gly Ile Glu Asn Gly Gln Glu Glu Asp Ser Ser Asn Lys Lys
   340 345 350
   Arg Ser Pro Val Ser Asn Asn Glu Glu Gly Met Leu Ser Phe Thr Ser
   355 360 365
   Val Leu Pro Cys Asp Ser Asn His Ser Asp Leu Glu Ala Ser Val Ala
   370 375 380
   Lys Glu Ala Glu Ser Asn Arg Val Val Val Glu Pro Glu Lys Lys Pro
   385 390 395 400
   Arg Lys Arg Gly Arg Lys Pro Ala Asn Gly Arg Glu Glu Pro Leu Asn
   405 410 415
   His Val Glu Ala Glu Arg Gln Arg Arg Glu Lys Leu Asn Gln Arg Phe
   420 425 430
   Tyr Ser Leu Arg Ala Val Val Pro Asn Val Ser Lys Met Asp Lys Ala
   435 440 445
   Ser Leu Leu Gly Asp Ala Ile Ser Tyr Ile Ser Glu Leu Lys Ser Lys
   450 455 460
   Leu Gln Lys Ala Glu Ser Asp Lys Glu Glu Leu Gln Lys Gln Ile Asp
   465 470 475 480
   Val Met Asn Lys Glu Ala Gly Asn Ala Lys Ser Ser Val Lys Asp Arg
   485 490 495
   Lys Cys Leu Asn Gln Glu Ser Ser Val Leu Ile Glu Met Glu Val Asp
   500 505 510
   Val Lys Ile Ile Gly Trp Asp Ala Met Ile Arg Ile Gln Cys Ser Lys
   515 520 525
   Arg Asn His Pro Gly Ala Lys Phe Met Glu Ala Leu Lys Glu Leu Asp
       530                 535                     540
   Leu Glu Val Asn His Ala Ser Leu Ser Val Val Asn Asp Leu Met Ile
   545                 550                 555                     560
   Gln Gln Ala Thr Val Lys Met Gly Asn Gln Phe Phe Thr Gln Asp Gln
                   565                 570                     575
   Leu Lys Val Ala Leu Thr Glu Lys Val Gly Glu Cys Pro
               580                 585
<210> 29
   <211> 1056
   <212> DNA
   <213> Arabidopsis thaliana
<220>
   <221> CDS
   <222> (1)..(1056)
   <223> G668
<400> 29
   atg gga aga cca cct tgc tgt gaa aag att gga gtg aag aaa ggg cca      48
   Met Gly Arg Pro Pro Cys Cys Glu Lys Ile Gly Val Lys Lys Gly Pro
   1               5                   10                  15
   tgg aca cca gag gaa gac atc atc ttg gtt tct tac atc caa gaa cat      96
   Trp Thr Pro Glu Glu Asp Ile Ile Leu Val Ser Tyr Ile Gln Glu His
               20                  25                  30
   ggt cct gga aac tgg aga tct gtc cca aca cac aca ggt tta aga tgt     144
   Gly Pro Gly Asn Trp Arg Ser Val Pro Thr His Thr Gly Leu Arg Cys
           35                  40                  45
   agc aag agc tgc aga ttg aga tgg act aat tat ctt cga ccc ggt att     192
   Ser Lys Ser Cys Arg Leu Arg Trp Thr Asn Tyr Leu Arg Pro Gly Ile
       50                  55                  60
   aag cgt gga aat ttt act gag cat gaa gag aag aca att gtt cat ctt     240
   Lys Arg Gly Asn Phe Thr Glu His Glu Glu Lys Thr Ile Val His Leu
   65                  70                  75                  80
   caa gcc ctt tta ggc aac aga tgg gca gcc ata gca tca tac ctt cca     288
   Gln Ala Leu Leu Gly Asn Arg Trp Ala Ala Ile Ala Ser Tyr Leu Pro
                   85                  90                  95
   gaa agg aca gac aat gat ata aag aac tat tgg aac act cac ttg aag     336
   Glu Arg Thr Asp Asn Asp Ile Lys Asn Tyr Trp Asn Thr His Leu Lys
               100                 105                 110
   aag aag ctc aaa aag att aat gaa tct ggt gaa gaa gat aat gat ggt     384
   Lys Lys Leu Lys Lys Ile Asn Glu Ser Gly Glu Glu Asp Asn Asp Gly
           115                 120                 125
   gtc tct tca tca aac act agt tca caa aag aac cat caa agc act aac     432
   Val Ser Ser Ser Asn Thr Ser Ser Gln Lys Asn His Gln Ser Thr Asn
       130                 135                 140
   aaa ggt caa tgg gaa aga aga ctt cag aca gac att aac atg gca aaa     480
   Lys Gly Gln Trp Glu Arg Arg Leu Gln Thr Asp Ile Asn Met Ala Lys
   145                 150                 155                 160
   caa gct ctt tgt gag gcc ttg tct tta gac aaa cca tca tcc act ctt     528
   Gln Ala Leu Cys Glu Ala Leu Ser Leu Asp Lys Pro Ser Ser Thr Leu
                   165                 170                 175
   tca tca tct tca tca tta ccg aca cca gta atc aca caa caa aac atc     576
   Ser Ser Ser Ser Ser Leu Pro Thr Pro Val Ile Thr Gln Gln Asn Ile
               180                 185                 190
   cgt aac ttc tca tca gct ttg ctt gac cgt tgt tat gat cca tcc tct      624
   Arg Asn Phe Ser Ser Ala Leu Leu Asp Arg Cys Tyr Asp Pro Ser Ser
           195                 200                 205
   tct tct tca tct acc aca acc acc act aca agc aac act act aat cca      672
   Ser Ser Ser Ser Thr Thr Thr Thr Thr Thr Ser Asn Thr Thr Asn Pro
       210                 215                 220
   tac cca tca ggg gta tat gcg tca agt gct gag aac atc gcc cgg ttg      720
   Tyr Pro Ser Gly Val Tyr Ala Ser Ser Ala Glu Asn Ile Ala Arg Leu
   225                 230                 235                 240
   ctt caa gat ttc atg aaa gac aca ccc aag gct tta act tta tca tct      768
   Leu Gln Asp Phe Met Lys Asp Thr Pro Lys Ala Leu Thr Leu Ser Ser
                   245                 250                 255
   tca tct ccg gtt tca gag act gga cca ctc act gct gca gtc tcg gaa      816
   Ser Ser Pro Val Ser Glu Thr Gly Pro Leu Thr Ala Ala Val Ser Glu
               260                 265                 270
   gaa ggt gga gaa ggg ttt gaa caa tct ttc ttc agc ttc aat tca atg      864
   Glu Gly Gly Glu Gly Phe Glu Gln Ser Phe Phe Ser Phe Asn Ser Met
           275                 280                 285
   gac gaa act caa aac ttg act cag gag aca agc ttc ttc cat gat caa      912
   Asp Glu Thr Gln Asn Leu Thr Gln Glu Thr Ser Phe Phe His Asp Gln
       290                 295                 300
   gtg atc aaa ccg gaa ata aca atg gac caa gat cat ggt cta ata tca      960
   Val Ile Lys Pro Glu Ile Thr Met Asp Gln Asp His Gly Leu Ile Ser
   305                 310                 315                 320
   caa ggg tct ctg tct ttg ttt gag aaa tgg tta ttt gat gag caa agc     1008
   Gln Gly Ser Leu Ser Leu Phe Glu Lys Trp Leu Phe Asp Glu Gln Ser
                   325                 330                 335
   cac gag atg gtt ggt atg gca cta gca gga caa gaa ggg atg ttc tag     1056
   His Glu Met Val Gly Met Ala Leu Ala Gly Gln Glu Gly Met Phe
               340                 345                 350
<210> 30
   <211> 351
   <212> PRT
   <213> Arabidopsis thaliana
<400> 30
   Met Gly Arg Pro Pro Cys Cys Glu Lys Ile Gly Val Lys Lys Gly Pro
   1               5                   10                  15
   Trp Thr Pro Glu Glu Asp Ile Ile Leu Val Ser Tyr Ile Gln Glu His
               20                  25                  30
   Gly Pro Gly Asn Trp Arg Ser Val Pro Thr His Thr Gly Leu Arg Cys
           35                  40                  45
   Ser Lys Ser Cys Arg Leu Arg Trp Thr Asn Tyr Leu Arg Pro Gly Ile
       50                  55                  60
   Lys Arg Gly Asn Phe Thr Glu His Glu Glu Lys Thr Ile Val His Leu
   65                  70                  75                  80
   Gln Ala Leu Leu Gly Asn Arg Trp Ala Ala Ile Ala Ser Tyr Leu Pro
                   85                  90                  95
   Glu Arg Thr Asp Asn Asp Ile Lys Asn Tyr Trp Asn Thr His Leu Lys
               100                 105                 110
   Lys Lys Leu Lys Lys Ile Asn Glu Ser Gly Glu Glu Asp Asn Asp Gly
           115                 120                 125
   Val Ser Ser Ser Asn Thr Ser Ser Gln Lys Asn His Gln Ser Thr Asn
       130                 135                 140
   Lys Gly Gln Trp Glu Arg Arg Leu Gln Thr Asp Ile Asn Met Ala Lys
   145                 150                 155                 160
   Gln Ala Leu Cys Glu Ala Leu Ser Leu Asp Lys Pro Ser Ser Thr Leu
                   165                 170                 175
   Ser Ser Ser Ser Ser Leu Pro Thr Pro Val Ile Thr Gln Gln Asn Ile
               180                 185                 190
   Arg Asn Phe Ser Ser Ala Leu Leu Asp Arg Cys Tyr Asp Pro Ser Ser
           195                 200                 205
   Ser Ser Ser Ser Thr Thr Thr Thr Thr Thr Ser Asn Thr Thr Asn Pro
       210                 215                 220
   Tyr Pro Ser Gly Val Tyr Ala Ser Ser Ala Glu Asn Ile Ala Arg Leu
   225                 230                 235                 240
   Leu Gln Asp Phe Met Lys Asp Thr Pro Lys Ala Leu Thr Leu Ser Ser
                   245                 250                 255
   Ser Ser Pro Val Ser Glu Thr Gly Pro Leu Thr Ala Ala Val Ser Glu
               260                 265                 270
   Glu Gly Gly Glu Gly Phe Glu Gln Ser Phe Phe Ser Phe Asn Ser Met
           275                 280                 285
   Asp Glu Thr Gln Asn Leu Thr Gln Glu Thr Ser Phe Phe His Asp Gln
       290                 295                 300
   Val Ile Lys Pro Glu Ile Thr Met Asp Gln Asp His Gly Leu Ile Ser
   305                 310                 315                 320
   Gln Gly Ser Leu Ser Leu Phe Glu Lys Trp Leu Phe Asp Glu Gln Ser
                   325                 330                 335
   His Glu Met Val Gly Met Ala Leu Ala Gly Gln Glu Gly Met Phe
               340                 345                 350
<210> 31
   <211> 2526
   <212> DNA
   <213> Arabidopsis thaliana
<220>
   <221> CDS
   <222> (338)..(2275)
   <223> G680
<400> 31
   cagttatctt cttccttctt ctctctgttt tttaaattta tttttagaga attttttttg      60
   ttttgcttcc gatttgatta tttccgggaa cgatgacttc tccggggagt tcccggtgag     120
   atgataagtc agattgcata cttgtctcct ccatggctac tctcaagggt tttggctgcg     180
   gtggattcgt ttggtttctc tagaatctaa agaggttatc acaacggctt tgcaatttga     240
   aaactttcat gtttggggag atcaaagatg gtttcttttt tatactttac ttgttagaga     300
   ggatttgaag cagcgaatag ctgcaaccgg tcctgtt atg gat act aat aca tct     355
                                            Met Asp Thr Asn Thr Ser
                                            1               5
   gga gaa gaa tta tta gct aag gca aga aag cca tat aca ata aca aag      403
   Gly Glu Glu Leu Leu Ala Lys Ala Arg Lys Pro Tyr Thr Ile Thr Lys
               10                  15                  20
   cag cga gag cga tgg act gag gat gag cat gag agg ttt cta gaa gcc      451
   Gln Arg Glu Arg Trp Thr Glu Asp Glu His Glu Arg Phe Leu Glu Ala
           25                  30                  35
   ttg agg ctt tat gga aga gct tgg caa cga att gaa gaa cat att ggg      499
   Leu Arg Leu Tyr Gly Arg Ala Trp Gln Arg Ile Glu Glu His Ile Gly
       40                  45                  50
   aca aag act gct gtt cag atc aga agt cat gca caa aag ttc ttc aca      547
   Thr Lys Thr Ala Val Gln Ile Arg Ser His Ala Gln Lys Phe Phe Thr
   55                  60                  65                  70
   aag ttg gag aaa gag gct gaa gtt aaa ggc atc cct gtt tgc caa gct      595
   Lys Leu Glu Lys Glu Ala Glu Val Lys Gly Ile Pro Val Cys Gln Ala
                   75                  80                  85
   ttg gac ata gaa att ccg cct cct cgt cct aaa cga aaa ccc aat act      643
   Leu Asp Ile Glu Ile Pro Pro Pro Arg Pro Lys Arg Lys Pro Asn Thr
               90                  95                  100
   cct tat cct cga aaa cct ggg aac aac ggt aca tct tcc tct caa gta      691
   Pro Tyr Pro Arg Lys Pro Gly Asn Asn Gly Thr Ser Ser Ser Gln Val
           105                 110                 115
   tca tca gca aaa gat gca aaa ctt gtt tca tcg gcc tct tct tca cag      739
   Ser Ser Ala Lys Asp Ala Lys Leu Val Ser Ser Ala Ser Ser Ser Gln
       120                 125                 130
   ttg aat cag gcg ttc ttg gat ttg gaa aaa atg ccg ttc tct gag aaa      787
   Leu Asn Gln Ala Phe Leu Asp Leu Glu Lys Met Pro Phe Ser Glu Lys
   135                 140                 145                 150
   aca tca act gga aaa gaa aat caa gat gag aat tgc tcg ggt gtt tct      835
   Thr Ser Thr Gly Lys Glu Asn Gln Asp Glu Asn Cys Ser Gly Val Ser
                   155                 160                 165
   act gtg aac aag tat ccc tta cca acg aaa cag gta agt ggc gac att      883
   Thr Val Asn Lys Tyr Pro Leu Pro Thr Lys Gln Val Ser Gly Asp Ile
               170                 175                 180
   gaa aca agt aag acc tca act gtg gac aac gcg gtt caa gat gtt ccc      931
   Glu Thr Ser Lys Thr Ser Thr Val Asp Asn Ala Val Gln Asp Val Pro
           185                 190                 195
   aag aag aac aaa gac aaa gat ggt aac gat ggt act act gtg cac agc      979
   Lys Lys Asn Lys Asp Lys Asp Gly Asn Asp Gly Thr Thr Val His Ser
       200                 205                 210
   atg caa aac tac cct tgg cat ttc cac gca gat att gtg aac ggg aat     1027
   Met Gln Asn Tyr Pro Trp His Phe His Ala Asp Ile Val Asn Gly Asn
   215                 220                 225                 230
   ata gca aaa tgc cct caa aat cat ccc tca ggt atg gta tct caa gac     1075
   Ile Ala Lys Cys Pro Gln Asn His Pro Ser Gly Met Val Ser Gln Asp
                   235                 240                 245
   ttc atg ttt cat cct atg aga gaa gaa act cac ggg cac gca aat ctt      1123
   Phe Met Phe His Pro Met Arg Glu Glu Thr His Gly His Ala Asn Leu
               250                 255                 260
   caa gct aca aca gca tct gct act act aca gct tct cat caa gcg ttt      1171
   Gln Ala Thr Thr Ala Ser Ala Thr Thr Thr Ala Ser His Gln Ala Phe
           265                 270                 275
   cca gct tgt cat tca cag gat gat tac cgt tcg ttt ctc cag ata tca      1219
   Pro Ala Cys His Ser Gln Asp Asp Tyr Arg Ser Phe Leu Gln Ile Ser
       280                 285                 290
   tct act ttc tcc aat ctt att atg tca act ctc cta cag aat cct gca      1267
   Ser Thr Phe Ser Asn Leu Ile Met Ser Thr Leu Leu Gln Asn Pro Ala
   295                 300                 305                 310
   gct cat gct gca gct aca ttc gct gct tcg gtc tgg cct tat gcg agt      1315
   Ala His Ala Ala Ala Thr Phe Ala Ala Ser Val Trp Pro Tyr Ala Ser
                   315                 320                 325
   gtc ggg aat tct ggt gat tca tca acc cca atg agc tct tct cct cca      1363
   Val Gly Asn Ser Gly Asp Ser Ser Thr Pro Met Ser Ser Ser Pro Pro
               330                 335                 340
   agt ata act gcc att gcc gct gct aca gta gct gct gca act gct tgg      1411
   Ser Ile Thr Ala Ile Ala Ala Ala Thr Val Ala Ala Ala Thr Ala Trp
           345                 350                 355
   tgg gct tct cat gga ctt ctt cct gta tgc gct cca gct cca ata aca      1459
   Trp Ala Ser His Gly Leu Leu Pro Val Cys Ala Pro Ala Pro Ile Thr
       360                 365                 370
   tgt gtt cca ttc tca act gtt gca gtt cca act cca gca atg act gaa      1507
   Cys Val Pro Phe Ser Thr Val Ala Val Pro Thr Pro Ala Met Thr Glu
   375                 380                 385                 390
   atg gat acc gtt gaa aat act caa ccg ttt gag aaa caa aac aca gct      1555
   Met Asp Thr Val Glu Asn Thr Gln Pro Phe Glu Lys Gln Asn Thr Ala
                   395                 400                 405
   ctg caa gat caa acc ttg gct tcg aaa tct cca gct tca tca tct gat      1603
   Leu Gln Asp Gln Thr Leu Ala Ser Lys Ser Pro Ala Ser Ser Ser Asp
               410                 415                 420
   gat tca gat gag act gga gta acc aag cta aat gcc gac tca aaa acc      1651
   Asp Ser Asp Glu Thr Gly Val Thr Lys Leu Asn Ala Asp Ser Lys Thr
           425                 430                 435
   aat gat gat aaa att gag gag gtt gtt gtt act gcc gct gtg cat gac      1699
   Asn Asp Asp Lys Ile Glu Glu Val Val Val Thr Ala Ala Val His Asp
       440                 445                 450
   tca aac act gcc cag aag aaa aat ctt gtg gac cgc tca tcg tgt ggc      1747
   Ser Asn Thr Ala Gln Lys Lys Asn Leu Val Asp Arg Ser Ser Cys Gly
   455                 460                 465                 470
   tca aat aca cct tca ggg agt gac gca gaa act gat gca tta gat aaa      1795
   Ser Asn Thr Pro Ser Gly Ser Asp Ala Glu Thr Asp Ala Leu Asp Lys
                   475                 480                 485
   atg gag aaa gat aaa gag gat gtg aag gag aca gat gag aat cag cca      1843
   Met Glu Lys Asp Lys Glu Asp Val Lys Glu Thr Asp Glu Asn Gln Pro
               490                 495                 500
   gat gtt att gag tta aat aac cgt aag att aaa atg aga gac aac aac      1891
   Asp Val Ile Glu Leu Asn Asn Arg Lys Ile Lys Met Arg Asp Asn Asn
           505                 510                 515
   agc aac aac aat gca act act gat tcg tgg aag gaa gtc tcc gaa gag      1939
   Ser Asn Asn Asn Ala Thr Thr Asp Ser Trp Lys Glu Val Ser Glu Glu
       520                 525                 530
   ggt cgt ata gcg ttt cag gct ctc ttt gca aga gaa aga ttg cct caa      1987
   Gly Arg Ile Ala Phe Gln Ala Leu Phe Ala Arg Glu Arg Leu Pro Gln
   535                 540                 545                 550
   agc ttt tcg cct cct caa gtg gca gag aat gtg aat aga aaa caa agt      2035
   Ser Phe Ser Pro Pro Gln Val Ala Glu Asn Val Asn Arg Lys Gln Ser
                   555                 560                 565
   gac acg tca atg cca ttg gct cct aat ttc aaa agc cag gat tct tgt      2083
   Asp Thr Ser Met Pro Leu Ala Pro Asn Phe Lys Ser Gln Asp Ser Cys
               570                 575                 580
   gct gca gac caa gaa gga gta gta atg atc ggt gtt gga aca tgc aag      2131
   Ala Ala Asp Gln Glu Gly Val Val Met Ile Gly Val Gly Thr Cys Lys
           585                 590                 595
   agt ctt aaa acg aga cag aca gga ttt aag cca tac aag aga tgt tca      2179
   Ser Leu Lys Thr Arg Gln Thr Gly Phe Lys Pro Tyr Lys Arg Cys Ser
       600                 605                 610
   atg gaa gtg aaa gag agc caa gtt ggg aac ata aac aat caa agt gat      2227
   Met Glu Val Lys Glu Ser Gln Val Gly Asn Ile Asn Asn Gln Ser Asp
   615                 620                 625                 630
   gaa aaa gtc tgc aaa agg ctt cga ttg gaa gga gaa gct tct aca tga      2275
   Glu Lys Val Cys Lys Arg Leu Arg Leu Glu Gly Glu Ala Ser Thr
                   635                 640                 645
   cagacttgga ggtaaaaaaa aaacatccac atttttatca atatctttaa atctagtgtt    2335
   agtagtttgc ttctccaatc tttatgaaag agacttttaa ttttccttcc gaacatttct    2395
   ttggtcatgt caggttctgt accatattac cccatgtctt gtctcttgtc tctgtttgtg    2455
   tatgctactt gtggtctata tgtcatctgc tactactgtt aattaaccat taagcaatgg    2515
   atttgtcttt a                                                         2526
<210> 32
   <211> 645
   <212> PRT
   <213> Arabidopsis thaliana
<400> 32
   Met Asp Thr Asn Thr Ser Gly Glu Glu Leu Leu Ala Lys Ala Arg Lys
   1               5                   10                  15
   Pro Tyr Thr Ile Thr Lys Gln Arg Glu Arg Trp Thr Glu Asp Glu His
               20                  25                  30
   Glu Arg Phe Leu Glu Ala Leu Arg Leu Tyr Gly Arg Ala Trp Gln Arg
           35                  40                  45
   Ile Glu Glu His Ile Gly Thr Lys Thr Ala Val Gln Ile Arg Ser His
       50                  55                  60
   Ala Gln Lys Phe Phe Thr Lys Leu Glu Lys Glu Ala Glu Val Lys Gly
   65                  70                  75                  80
   Ile Pro Val Cys Gln Ala Leu Asp Ile Glu Ile Pro Pro Pro Arg Pro
                   85                  90                  95
   Lys Arg Lys Pro Asn Thr Pro Tyr Pro Arg Lys Pro Gly Asn Asn Gly
               100                 105                 110
   Thr Ser Ser Ser Gln Val Ser Ser Ala Lys Asp Ala Lys Leu Val Ser
           115                 120                 125
   Ser Ala Ser Ser Ser Gln Leu Asn Gln Ala Phe Leu Asp Leu Glu Lys
   130 135 140
   Met Pro Phe Ser Glu Lys Thr Ser Thr Gly Lys Glu Asn Gln Asp Glu
   145 150 155 160
   Asn Cys Ser Gly Val Ser Thr Val Asn Lys Tyr Pro Leu Pro Thr Lys
   165 170 175
   Gln Val Ser Gly Asp Ile Glu Thr Ser Lys Thr Ser Thr Val Asp Asn
   180 185 190
   Ala Val Gln Asp Val Pro Lys Lys Asn Lys Asp Lys Asp Gly Asn Asp
   195 200 205
   Gly Thr Thr Val His Ser Met Gln Asn Tyr Pro Trp His Phe His Ala
   210 215 220
   Asp Ile Val Asn Gly Asn Ile Ala Lys Cys Pro Gln Asn His Pro Ser
   225 230 235 240
   Gly Met Val Ser Gln Asp Phe Met Phe His Pro Met Arg Glu Glu Thr
   245 250 255
   His Gly His Ala Asn Leu Gln Ala Thr Thr Ala Ser Ala Thr Thr Thr
   260 265 270
   Ala Ser His Gln Ala Phe Pro Ala Cys His Ser Gln Asp Asp Tyr Arg
   275 280 285
   Ser Phe Leu Gln Ile Ser Ser Thr Phe Ser Asn Leu Ile Met Ser Thr
   290 295 300
   Leu Leu Gln Asn Pro Ala Ala His Ala Ala Ala Thr Phe Ala Ala Ser
   305 310 315 320
   Val Trp Pro Tyr Ala Ser Val Gly Asn Ser Gly Asp Ser Ser Thr Pro
   325 330 335
   Met Ser Ser Ser Pro Pro Ser Ile Thr Ala Ile Ala Ala Ala Thr Val
   340 345 350
   Ala Ala Ala Thr Ala Trp Trp Ala Ser His Gly Leu Leu Pro Val Cys
   355 360 365
   Ala Pro Ala Pro Ile Thr Cys Val Pro Phe Ser Thr Val Ala Val Pro
   370 375 380
   Thr Pro Ala Met Thr Glu Met Asp Thr Val Glu Asn Thr Gln Pro Phe
   385 390 395 400
   Glu Lys Gln Asn Thr Ala Leu Gln Asp Gln Thr Leu Ala Ser Lys Ser
   405 410 415
   Pro Ala Ser Ser Ser Asp Asp Ser Asp Glu Thr Gly Val Thr Lys Leu
   420 425 430
   Asn Ala Asp Ser Lys Thr Asn Asp Asp Lys Ile Glu Glu Val Val Val
   435 440 445
   Thr Ala Ala Val His Asp Ser Asn Thr Ala Gln Lys Lys Asn Leu Val
   450 455 460
   Asp Arg Ser Ser Cys Gly Ser Asn Thr Pro Ser Gly Ser Asp Ala Glu
   465 470 475 480
   Thr Asp Ala Leu Asp Lys Met Glu Lys Asp Lys Glu Asp Val Lys Glu
   485 490 495
   Thr Asp Glu Asn Gln Pro Asp Val Ile Glu Leu Asn Asn Arg Lys Ile
   500 505 510
   Lys Met Arg Asp Asn Asn Ser Asn Asn Asn Ala Thr Thr Asp Ser Trp
   515 520 525
   Lys Glu Val Ser Glu Glu Gly Arg Ile Ala Phe Gln Ala Leu Phe Ala
   530 535 540
   Arg Glu Arg Leu Pro Gln Ser Phe Ser Pro Pro Gln Val Ala Glu Asn
   545 550 555 560
   Val Asn Arg Lys Gln Ser Asp Thr Ser Met Pro Leu Ala Pro Asn Phe
   565 570 575
   Lys Ser Gln Asp Ser Cys Ala Ala Asp Gln Glu Gly Val Val Met Ile
   580 585 590
   Gly Val Gly Thr Cys Lys Ser Leu Lys Thr Arg Gln Thr Gly Phe Lys
   595 600 605
   Pro Tyr Lys Arg Cys Ser Met Glu Val Lys Glu Ser Gln Val Gly Asn
   610 615 620
   Ile Asn Asn Gln Ser Asp Glu Lys Val Cys Lys Arg Leu Arg Leu Glu
   625 630 635 640
   Gly Glu Ala Ser Thr
   645
<210> 33
   <211> 228
   <212> DNA
   <213> Arabidopsis thaliana
<220>
   <221> CDS
   <222> (1)..(228)
   <223> G682
<400> 33
   atg gat aac cat cgc agg act aag caa ccc aag acc aac tcc atc gtt        48
   Met Asp Asn His Arg Arg Thr Lys Gln Pro Lys Thr Asn Ser Ile Val
   1               5                   10                  15
   act tct tct tct gaa gaa gtg agt agt ctt gag tgg gaa gtt gtg aac        96
   Thr Ser Ser Ser Glu Glu Val Ser Ser Leu Glu Trp Glu Val Val Asn
               20                  25                  30
   atg agt caa gaa gaa gaa gat ttg gtc tct cga atg cat aag ctt gtc       144
   Met Ser Gln Glu Glu Glu Asp Leu Val Ser Arg Met His Lys Leu Val
           35                  40                  45
   ggt gac agg tgg gaa ctg ata gct ggg agg atc cca gga aga acc gct       192
   Gly Asp Arg Trp Glu Leu Ile Ala Gly Arg Ile Pro Gly Arg Thr Ala
       50                  55                  60
   gga gaa att gag agg ttt tgg gtc atg aaa aat tga                       228
   Gly Glu Ile Glu Arg Phe Trp Val Met Lys Asn
   65                  70                  75
<210> 34
   <211> 75
   <212> PRT
   <213> Arabidopsis thaliana
<400> 34
   Met Asp Asn His Arg Arg Thr Lys Gln Pro Lys Thr Asn Ser Ile Val
   1               5                   10                  15
   Thr Ser Ser Ser Glu Glu Val Ser Ser Leu Glu Trp Glu Val Val Asn
               20                  25                  30
   Met Ser Gln Glu Glu Glu Asp Leu Val Ser Arg Met His Lys Leu Val
           35                  40                  45
   Gly Asp Arg Trp Glu Leu Ile Ala Gly Arg Ile Pro Gly Arg Thr Ala
       50                  55                  60
   Gly Glu Ile Glu Arg Phe Trp Val Met Lys Asn
   65                  70                  75
<210> 35
   <211> 584
   <212> DNA
   <213> Arabidopsis thaliana
<220>
   <221> CDS
   <222> (157)..(441)
   <223> G225
<400> 35
   ctctctctct cactcttttc ttttccgaga acccaacaaa aaaaaagcta ctattaatcc      60
   ttcccctcgt gaggaaatca tttcttcttg tttctcgaga tttattctct ttctctctct     120
   ctttctctgt gtgtttcgtg tcttcagatt agttcg atg ttt cgt tca gac aag       174
                                           Met Phe Arg Ser Asp Lys
                                           1               5
   gcg gaa aaa atg gat aaa cga cga cgg aga cag agc aaa gcc aag gct       222
   Ala Glu Lys Met Asp Lys Arg Arg Arg Arg Gln Ser Lys Ala Lys Ala
               10                  15                  20
   tct tgt tcc gaa gag gtg agt agt atc gaa tgg gaa gct gtg aag atg       270
   Ser Cys Ser Glu Glu Val Ser Ser Ile Glu Trp Glu Ala Val Lys Met
           25                  30                  35
   tca gaa gaa gaa gaa gat ctc att tct cgg atg tat aaa ctc gtt ggc       318
   Ser Glu Glu Glu Glu Asp Leu Ile Ser Arg Met Tyr Lys Leu Val Gly
       40                  45                  50
   gac agg tgg gag ttg atc gcc gga agg atc ccg gga cgg acg ccg gag       366
   Asp Arg Trp Glu Leu Ile Ala Gly Arg Ile Pro Gly Arg Thr Pro Glu
   55                  60                  65                  70
   gag ata gag aga tat tgg ctt atg aaa cac ggc gtc gtt ttt gcc aac       414
   Glu Ile Glu Arg Tyr Trp Leu Met Lys His Gly Val Val Phe Ala Asn
                   75                  80                  85
   aga cga aga gac ttt ttt agg aaa tga ttttttttgt ttggattaaa             461
   Arg Arg Arg Asp Phe Phe Arg Lys
               90
   agaaaatttt cctctcctta attcacaaga caagaaaaaa aggaaatgta cctgtccttg     521
   aattactatt ttggaatgta taattatcta tatatataag aagaaaaaat tgcttaggaa     581
   ttt                                                                   584
<210> 36
   <211> 94
   <212> PRT
   <213> Arabidopsis thaliana
<400> 36
   Met Phe Arg Ser Asp Lys Ala Glu Lys Met Asp Lys Arg Arg Arg Arg
   1               5                   10                  15
   Gln Ser Lys Ala Lys Ala Ser Cys Ser Glu Glu Val Ser Ser Ile Glu
               20                  25                  30
   Trp Glu Ala Val Lys Met Ser Glu Glu Glu Glu Asp Leu Ile Ser Arg
           35                  40                  45
   Met Tyr Lys Leu Val Gly Asp Arg Trp Glu Leu Ile Ala Gly Arg Ile
       50                  55                  60
   Pro Gly Arg Thr Pro Glu Glu Ile Glu Arg Tyr Trp Leu Met Lys His
   65                  70                  75                  80
   Gly Val Val Phe Ala Asn Arg Arg Arg Asp Phe Phe Arg Lys
                   85                  90
<210> 37
   <211> 1369
   <212> DNA
   <213> Arabidopsis thaliana
<220>
   <221> CDS
   <222> (104)..(1174)
   <223> G678
<400> 37
   ggttgtgtcg tcactttact tctctttaaa taatctcttt ctctagtgat tgactttagg     60
   aacaagtgaa gtgagtgatt tttaataatc gccggccgag aga atg gga agg gcg      115
                                                   Met Gly Arg Ala
                                                   1
   ccg tgc tgt gag aag gta gga att aag aag ggg cgc tgg acg gcg gag      163
   Pro Cys Cys Glu Lys Val Gly Ile Lys Lys Gly Arg Trp Thr Ala Glu
   5                   10                  15                  20
   gaa gac cgg act ctc tcc gac tac att cag tcc aac ggc gaa gga tca      211
   Glu Asp Arg Thr Leu Ser Asp Tyr Ile Gln Ser Asn Gly Glu Gly Ser
                   25                  30                  35
   tgg cgt tct ctt ccc aaa aat gcc ggg cta aag aga tgt gga aag agc      259
   Trp Arg Ser Leu Pro Lys Asn Ala Gly Leu Lys Arg Cys Gly Lys Ser
               40                  45                  50
   tgt aga ttg aga tgg ata aac tat ttg aga tca gac atc aag aga gga      307
   Cys Arg Leu Arg Trp Ile Asn Tyr Leu Arg Ser Asp Ile Lys Arg Gly
           55                  60                  65
   aac ata act ccc gaa gaa gag gac gtc att gtt aaa ctg cat tcc act      355
   Asn Ile Thr Pro Glu Glu Glu Asp Val Ile Val Lys Leu His Ser Thr
       70                  75                  80
   ttg gga acc agg tgg tca aca att gcg agc aat cta ccg gga aga aca      403
   Leu Gly Thr Arg Trp Ser Thr Ile Ala Ser Asn Leu Pro Gly Arg Thr
   85                  90                  95                  100
   gac aac gaa ata aaa aac tat tgg aat tct cat ctc agc cgt aaa ctc      451
   Asp Asn Glu Ile Lys Asn Tyr Trp Asn Ser His Leu Ser Arg Lys Leu
                   105                 110                 115
   cac ggt tac ttc aga aaa cca act gtc gcc aat acc gtc gag aat gcg      499
   His Gly Tyr Phe Arg Lys Pro Thr Val Ala Asn Thr Val Glu Asn Ala
               120                 125                 130
   cct ccg cct cct aag cgt aga cct gga aga acc agc aga tcc gcc atg      547
   Pro Pro Pro Pro Lys Arg Arg Pro Gly Arg Thr Ser Arg Ser Ala Met
           135                 140                 145
   aaa ccc aaa ttt atc cta aac cct aaa aac cac aaa acc cct aat tct      595
   Lys Pro Lys Phe Ile Leu Asn Pro Lys Asn His Lys Thr Pro Asn Ser
       150                 155                 160
   ttt aaa gca aac aaa agt gac atc gtt ttg cca act acg aca ata gag      643
   Phe Lys Ala Asn Lys Ser Asp Ile Val Leu Pro Thr Thr Thr Ile Glu
   165                 170                 175                 180
   aat gga gag gga gac aaa gaa gac gca tta atg gtg ttg tca agt agt      691
   Asn Gly Glu Gly Asp Lys Glu Asp Ala Leu Met Val Leu Ser Ser Ser
                   185                 190                 195
   agc tta agt gga gca gag gaa ccc ggt tta gga cca tgt ggt tat gga      739
   Ser Leu Ser Gly Ala Glu Glu Pro Gly Leu Gly Pro Cys Gly Tyr Gly
               200                 205                 210
   gac gat ggc gat tgt aac cca agc att aat ggc gac gat gga gct ttg      787
   Asp Asp Gly Asp Cys Asn Pro Ser Ile Asn Gly Asp Asp Gly Ala Leu
           215                 220                 225
   tgt ctc aat gac gac att ttc gat tct tgt ttt cta ttg gac gac tct      835
   Cys Leu Asn Asp Asp Ile Phe Asp Ser Cys Phe Leu Leu Asp Asp Ser
       230                 235                 240
   cat gct gtc cac gtg tcc tca tgt gag tcg aac aac gta aaa aac tct      883
   His Ala Val His Val Ser Ser Cys Glu Ser Asn Asn Val Lys Asn Ser
   245                 250                 255                 260
   gag cca tat gga ggg atg tca gtt ggg cac aaa aat atc gaa acg atg      931
   Glu Pro Tyr Gly Gly Met Ser Val Gly His Lys Asn Ile Glu Thr Met
                   265                 270                 275
   gct gat gat ttc gtt gac tgg gac ttt gta tgg aga gaa ggt caa acc      979
   Ala Asp Asp Phe Val Asp Trp Asp Phe Val Trp Arg Glu Gly Gln Thr
               280                 285                 290
   ctt tgg gac gaa aaa gag gat ctt gat tcg gtt ttg tcg agg ctg tta     1027
   Leu Trp Asp Glu Lys Glu Asp Leu Asp Ser Val Leu Ser Arg Leu Leu
           295                 300                 305
   gat gga gag gaa atg gaa tct gag atc aga caa agg gac tcc aac gac     1075
   Asp Gly Glu Glu Met Glu Ser Glu Ile Arg Gln Arg Asp Ser Asn Asp
       310                 315                 320
   ttt gga gaa ccg ttg gat att gac gaa gaa aac aag atg gct gct tgg     1123
   Phe Gly Glu Pro Leu Asp Ile Asp Glu Glu Asn Lys Met Ala Ala Trp
   325                 330                 335                 340
   ctt ttt tcc tta aaa att tta ccc cct tcc ttt tcc ctt ttc ccc ctt      1171
   Leu Phe Ser Leu Lys Ile Leu Pro Pro Ser Phe Ser Leu Phe Pro Leu
                   345                 350                 355
   taa tttttaccaa aaccccccct tgccagatcc tgtccgtttt tccattaaac           1224
   ctttttctcc ccctaccttc ctttttttat ttttaatttt ttttttttcc tttttttttc       1284
   ctttcctttt ttaattccga tttttggcgg gttgccaatt aaccaaatta aatccatcct    1344
   taaaaaaaaa aaaaaaaaaa aaaaa                                          1369
<210> 38
   <211> 356
   <212> PRT
   <213> Arabidopsis thaliana
<400> 38
   Met Gly Arg Ala Pro Cys Cys Glu Lys Val Gly Ile Lys Lys Gly Arg
   1               5                   10                  15
   Trp Thr Ala Glu Glu Asp Arg Thr Leu Ser Asp Tyr Ile Gln Ser Asn
               20                  25                  30
   Gly Glu Gly Ser Trp Arg Ser Leu Pro Lys Asn Ala Gly Leu Lys Arg
           35                  40                  45
   Cys Gly Lys Ser Cys Arg Leu Arg Trp Ile Asn Tyr Leu Arg Ser Asp
       50                  55                  60
   Ile Lys Arg Gly Asn Ile Thr Pro Glu Glu Glu Asp Val Ile Val Lys
   65                  70                  75                  80
   Leu His Ser Thr Leu Gly Thr Arg Trp Ser Thr Ile Ala Ser Asn Leu
                   85                  90                  95
   Pro Gly Arg Thr Asp Asn Glu Ile Lys Asn Tyr Trp Asn Ser His Leu
               100                 105                 110
   Ser Arg Lys Leu His Gly Tyr Phe Arg Lys Pro Thr Val Ala Asn Thr
           115                 120                 125
   Val Glu Asn Ala Pro Pro Pro Pro Lys Arg Arg Pro Gly Arg Thr Ser
       130                 135                 140
   Arg Ser Ala Met Lys Pro Lys Phe Ile Leu Asn Pro Lys Asn His Lys
   145                 150                 155                 160
   Thr Pro Asn Ser Phe Lys Ala Asn Lys Ser Asp Ile Val Leu Pro Thr
                   165                 170                 175
   Thr Thr Ile Glu Asn Gly Glu Gly Asp Lys Glu Asp Ala Leu Met Val
               180                 185                 190
   Leu Ser Ser Ser Ser Leu Ser Gly Ala Glu Glu Pro Gly Leu Gly Pro
           195                 200                 205
   Cys Gly Tyr Gly Asp Asp Gly Asp Cys Asn Pro Ser Ile Asn Gly Asp
       210                 215                 220
   Asp Gly Ala Leu Cys Leu Asn Asp Asp Ile Phe Asp Ser Cys Phe Leu
   225 230 235 240
   Leu Asp Asp Ser His Ala Val His Val Ser Ser Cys Glu Ser Asn Asn
   245 250 255
   Val Lys Asn Ser Glu Pro Tyr Gly Gly Met Ser Val Gly His Lys Asn
   260 265 270
   Ile Glu Thr Met Ala Asp Asp Phe Val Asp Trp Asp Phe Val Trp Arg
   275 280 285
   Glu Gly Gln Thr Leu Trp Asp Glu Lys Glu Asp Leu Asp Ser Val Leu
   290 295 300
   Ser Arg Leu Leu Asp Gly Glu Glu Met Glu Ser Glu Ile Arg Gln Arg
   305 310 315 320
   Asp Ser Asn Asp Phe Gly Glu Pro Leu Asp Ile Asp Glu Glu Asn Lys
   325 330 335
   Met Ala Ala Trp Leu Phe Ser Leu Lys Ile Leu Pro Pro Ser Phe Ser
   340 345 350
   Leu Phe Pro Leu
   355
<210> 39
   <211> 1046
   <212> DNA
   <213> Arabidopsis thaliana
<220>
   <221> CDS
   <222> (46)..(867)
   <223> G233
<400> 39
   gaaaaacatt tcaacttctt ttatcagcaa tcacaaatca aagag atg gga aga gct     57
                                                     Met Gly Arg Ala
                                                     1
   cca tgc tgt gag aag atg ggg ttg aag aga gga cca tgg aca cct gaa       105
   Pro Cys Cys Glu Lys Met Gly Leu Lys Arg Gly Pro Trp Thr Pro Glu
   5                   10                  15                  20
   gaa gat caa atc ttg gtc tct ttt atc ctc aac cat gga cat agt aac       153
   Glu Asp Gln Ile Leu Val Ser Phe Ile Leu Asn His Gly His Ser Asn
                   25                  30                  35
   tgg cga gcc ctc cct aag caa gct ggt ctt ttg aga tgt gga aaa agc       201
   Trp Arg Ala Leu Pro Lys Gln Ala Gly Leu Leu Arg Cys Gly Lys Ser
               40                  45                  50
   tgt aga ctt agg tgg atg aac tat tta aag cct gat att aaa cgt ggc       249
   Cys Arg Leu Arg Trp Met Asn Tyr Leu Lys Pro Asp Ile Lys Arg Gly
           55                  60                  65
   aat ttc acc aaa gaa gag gaa gat gct atc atc agc tta cac caa ata       297
   Asn Phe Thr Lys Glu Glu Glu Asp Ala Ile Ile Ser Leu His Gln Ile
       70                  75                  80
   ctt ggc aat aga tgg tca gcg att gca gca aaa ctg cct gga aga acc       345
   Leu Gly Asn Arg Trp Ser Ala Ile Ala Ala Lys Leu Pro Gly Arg Thr
   85                  90                  95                  100
   gat aac gag atc aag aac gta tgg cac act cac ttg aag aag aga ctc      393
   Asp Asn Glu Ile Lys Asn Val Trp His Thr His Leu Lys Lys Arg Leu
                   105                 110                 115
   gaa gat tat caa cca gct aaa cct aag acc agc aac aaa aag aag ggt      441
   Glu Asp Tyr Gln Pro Ala Lys Pro Lys Thr Ser Asn Lys Lys Lys Gly
               120                 125                 130
   act aaa cca aaa tct gaa tcc gta ata acg agc tcg aac agt act aga      489
   Thr Lys Pro Lys Ser Glu Ser Val Ile Thr Ser Ser Asn Ser Thr Arg
           135                 140                 145
   agc gaa tcg gag cta gca gat tca tca aac cct tct gga gaa agc tta      537
   Ser Glu Ser Glu Leu Ala Asp Ser Ser Asn Pro Ser Gly Glu Ser Leu
       150                 155                 160
   ttt tcg aca tcg cct tcg aca agt gag gtt tct tcg atg aca ctc ata      585
   Phe Ser Thr Ser Pro Ser Thr Ser Glu Val Ser Ser Met Thr Leu Ile
   165                 170                 175                 180
   agc cac gac ggc tat agc aac gag att aat atg gat aac aaa ccg gga      633
   Ser His Asp Gly Tyr Ser Asn Glu Ile Asn Met Asp Asn Lys Pro Gly
                   185                 190                 195
   gat atc agt act atc gat caa gaa tgt gtt tct ttc gaa act ttt ggt      681
   Asp Ile Ser Thr Ile Asp Gln Glu Cys Val Ser Phe Glu Thr Phe Gly
               200                 205                 210
   gcg gat atc gat gaa agc ttc tgg aaa gag aca ctg tat agc caa gat      729
   Ala Asp Ile Asp Glu Ser Phe Trp Lys Glu Thr Leu Tyr Ser Gln Asp
           215                 220                 225
   gaa cac aac tac gta tcg aat gac cta gaa gtc gct ggt tta gtt gag      777
   Glu His Asn Tyr Val Ser Asn Asp Leu Glu Val Ala Gly Leu Val Glu
       230                 235                 240
   ata caa caa gag ttt caa aac ttg ggc tcc gct aat aat gag atg att      825
   Ile Gln Gln Glu Phe Gln Asn Leu Gly Ser Ala Asn Asn Glu Met Ile
   245                 250                 255                 260
   ttt gac agt gag atg gaa ctt ctg gtt cga tgt att ggc tag              867
   Phe Asp Ser Glu Met Glu Leu Leu Val Arg Cys Ile Gly
                   265                 270
   aaccggcggg gaacaagatc tcttagccgg gctctagtta acatgtttga ggagtaaagt   927
   gaaatggtgc aaattagtta aggctaagaa attcaaaagc ttttgtttac cgagaaaaaa   987
   acacactcta actcttgatg tgatgtagtt agtgtattaa ttagaggctg cgttttcaa   1046
<210> 40
   <211> 273
   <212> PRT
   <213> Arabidopsis thaliana
<400> 40
   Met Gly Arg Ala Pro Cys Cys Glu Lys Met Gly Leu Lys Arg Gly Pro
   1               5                   10                  15
   Trp Thr Pro Glu Glu Asp Gln Ile Leu Val Ser Phe Ile Leu Asn His
               20                  25                  30
   Gly His Ser Asn Trp Arg Ala Leu Pro Lys Gln Ala Gly Leu Leu Arg
           35                  40                  45
   Cys Gly Lys Ser Cys Arg Leu Arg Trp Met Asn Tyr Leu Lys Pro Asp
       50                  55                  60
   Ile Lys Arg Gly Asn Phe Thr Lys Glu Glu Glu Asp Ala Ile Ile Ser
   65                  70                  75                  80
   Leu His Gln Ile Leu Gly Asn Arg Trp Ser Ala Ile Ala Ala Lys Leu
                   85                  90                  95
   Pro Gly Arg Thr Asp Asn Glu Ile Lys Asn Val Trp His Thr His Leu
               100                 105                 110
   Lys Lys Arg Leu Glu Asp Tyr Gln Pro Ala Lys Pro Lys Thr Ser Asn
           115                 120                 125
   Lys Lys Lys Gly Thr Lys Pro Lys Ser Glu Ser Val Ile Thr Ser Ser
       130                 135                 140
   Asn Ser Thr Arg Ser Glu Ser Glu Leu Ala Asp Ser Ser Asn Pro Ser
   145                 150                 155                 160
   Gly Glu Ser Leu Phe Ser Thr Ser Pro Ser Thr Ser Glu Val Ser Ser
                   165                 170                 175
   Met Thr Leu Ile Ser His Asp Gly Tyr Ser Asn Glu Ile Asn Met Asp
               180                 185                 190
   Asn Lys Pro Gly Asp Ile Ser Thr Ile Asp Gln Glu Cys Val Ser Phe
           195                 200                 205
   Glu Thr Phe Gly Ala Asp Ile Asp Glu Ser Phe Trp Lys Glu Thr Leu
       210                 215                 220
   Tyr Ser Gln Asp Glu His Asn Tyr Val Ser Asn Asp Leu Glu Val Ala
   225                 230                 235                 240
   Gly Leu Val Glu Ile Gln Gln Glu Phe Gln Asn Leu Gly Ser Ala Asn
                   245                 250                 255
   Asn Glu Met Ile Phe Asp Ser Glu Met Glu Leu Leu Val Arg Cys Ile
               260                 265                 270
   Gly
<210> 41
   <211> 1262
   <212> DNA
   <213> Arabidopsis thaliana
<220>
   <221> CDS
   <222> (217)..(957)
   <223> G463
<400> 41
   ctcgagctac gtcaggggtc tctttctgtt tgtttgtttt cttgtttcct tctctctctc      60
   tctttctttc tttgtcttcc tttcccaggt tgtttttttt tgctctctct gccttcttga     120
   ctttcaaaag actctttctt tcttttggat tgattttgga ttctagggct ctctttcttt     180
   tagtgggttt ttgttgttgt tgttgtggtc tctctg atg att act gaa ctt gag      234
                                           Met Ile Thr Glu Leu Glu
                                           1               5
   atg ggg aaa ggt gag agt gag ctt gag ctt ggt cta ggg ctg agt ctt      282
   Met Gly Lys Gly Glu Ser Glu Leu Glu Leu Gly Leu Gly Leu Ser Leu
               10                  15                  20
   ggc ggt gga acg gcg gcc aag att ggt aaa tca ggt ggt ggt ggc gcg      330
   Gly Gly Gly Thr Ala Ala Lys Ile Gly Lys Ser Gly Gly Gly Gly Ala
           25                  30                  35
   tgg gga gag cgt gga agg ctt ttg acg gct aag gat ttt cct tct gtt      378
   Trp Gly Glu Arg Gly Arg Leu Leu Thr Ala Lys Asp Phe Pro Ser Val
       40                  45                  50
   ggt tct aaa cgt gct gct gat tct gct tct cat gct ggt tca tct cct      426
   Gly Ser Lys Arg Ala Ala Asp Ser Ala Ser His Ala Gly Ser Ser Pro
   55                  60                  65                  70
   cct cgt tca agt caa gtt gtt gga tgg cct cct ata ggg tca cac agg      474
   Pro Arg Ser Ser Gln Val Val Gly Trp Pro Pro Ile Gly Ser His Arg
                   75                  80                  85
   atg aac agt ttg gtt aat aac caa gct aca aag tca gca aga gaa gaa      522
   Met Asn Ser Leu Val Asn Asn Gln Ala Thr Lys Ser Ala Arg Glu Glu
               90                  95                  100
   gaa gaa gct ggt aag aag aaa gtg aaa gat gat gaa cct aaa gat gtg      570
   Glu Glu Ala Gly Lys Lys Lys Val Lys Asp Asp Glu Pro Lys Asp Val
           105                 110                 115
   aca aag aaa gtg aat ggg aaa gta caa gtt gga ttt att aag gtg aac      618
   Thr Lys Lys Val Asn Gly Lys Val Gln Val Gly Phe Ile Lys Val Asn
       120                 125                 130
   atg gat gga gtt gct ata gga aga aaa gtg gat ttg aat gct cat tct      666
   Met Asp Gly Val Ala Ile Gly Arg Lys Val Asp Leu Asn Ala His Ser
   135                 140                 145                 150
   tct tac gag aat ttg gcg caa aca ttg gaa gat atg ttc ttt cgc act      714
   Ser Tyr Glu Asn Leu Ala Gln Thr Leu Glu Asp Met Phe Phe Arg Thr
                   155                 160                 165
   aat ccg ggt act gtc ggg tta acc agt cag ttc act aaa ccg ttg agg      762
   Asn Pro Gly Thr Val Gly Leu Thr Ser Gln Phe Thr Lys Pro Leu Arg
               170                 175                 180
   ctt tta gat gga tcg tct gag ttt gta ctt act tat gaa gat aag gaa      810
   Leu Leu Asp Gly Ser Ser Glu Phe Val Leu Thr Tyr Glu Asp Lys Glu
           185                 190                 195
   gga gat tgg atg ctt gtt ggt gat gtt cca tgg aga atg ttc atc aac      858
   Gly Asp Trp Met Leu Val Gly Asp Val Pro Trp Arg Met Phe Ile Asn
       200                 205                 210
   tcg gtg aaa agg cta cgt gtg atg aaa acc tct gaa gct aat gga ctc      906
   Ser Val Lys Arg Leu Arg Val Met Lys Thr Ser Glu Ala Asn Gly Leu
   215                 220                 225                 230
   gct gca cga aat caa gaa cca aac gag aga cag cga aag cag ccg gtt      954
   Ala Ala Arg Asn Gln Glu Pro Asn Glu Arg Gln Arg Lys Gln Pro Val
                   235                 240                 245
   tag atctcttttc gacgttacgg tgttacaggt tttatatttt ggggttttgc          1007
   aagtctgaga tacttctgaa gcaagcataa gctagattga tcttatatcc agtttgtgta   1067
   ttttcttggt tcttataatg gtttttactg gttttcttta gttttttttt ttgctgtctt   1127
   ttaattttcg gttgcgattt cactatatac tatggatgga agagaatgct ctttatatct   1187
   tttactacac tgtaaatatt tgaagcttat ctaatatcgt ttttaagggt taaaaaaccc   1247
   tgacgtagcc tcgag 1262
<210> 42
   <211> 246
   <212> PRT
   <213> Arabidopsis thaliana
<400> 42
   Met Ile Thr Glu Leu Glu Met Gly Lys Gly Glu Ser Glu Leu Glu Leu
   1               5                   10                  15
   Gly Leu Gly Leu Ser Leu Gly Gly Gly Thr Ala Ala Lys Ile Gly Lys
               20                  25                  30
   Ser Gly Gly Gly Gly Ala Trp Gly Glu Arg Gly Arg Leu Leu Thr Ala
           35                  40                  45
   Lys Asp Phe Pro Ser Val Gly Ser Lys Arg Ala Ala Asp Ser Ala Ser
       50                  55                  60
   His Ala Gly Ser Ser Pro Pro Arg Ser Ser Gln Val Val Gly Trp Pro
   65                  70                  75                  80
   Pro Ile Gly Ser His Arg Met Asn Ser Leu Val Asn Asn Gln Ala Thr
                   85                  90                  95
   Lys Ser Ala Arg Glu Glu Glu Glu Ala Gly Lys Lys Lys Val Lys Asp
               100                 105                 110
   Asp Glu Pro Lys Asp Val Thr Lys Lys Val Asn Gly Lys Val Gln Val
           115                 120                 125
   Gly Phe Ile Lys Val Asn Met Asp Gly Val Ala Ile Gly Arg Lys Val
       130                 135                 140
   Asp Leu Asn Ala His Ser Ser Tyr Glu Asn Leu Ala Gln Thr Leu Glu
   145                 150                 155                 160
   Asp Met Phe Phe Arg Thr Asn Pro Gly Thr Val Gly Leu Thr Ser Gln
                   165                 170                 175
   Phe Thr Lys Pro Leu Arg Leu Leu Asp Gly Ser Ser Glu Phe Val Leu
               180                 185                 190
   Thr Tyr Glu Asp Lys Glu Gly Asp Trp Met Leu Val Gly Asp Val Pro
           195                 200                 205
   Trp Arg Met Phe Ile Asn Ser Val Lys Arg Leu Arg Val Met Lys Thr
       210                 215                 220
   Ser Glu Ala Asn Gly Leu Ala Ala Arg Asn Gln Glu Pro Asn Glu Arg
   225                 230                 235                 240
   Gln Arg Lys Gln Pro Val
                   245
<210> 43
   <211> 741
   <212> DNA
   <213> Arabidopsis thaliana
<220>
   <221> CDS
   <222> (1)..(741)
   <223> G2422
<400> 43
   atg ggc gaa tca ccc aaa ggg ttg aga aaa ggt aca tgg act act gaa      48
   Met Gly Glu Ser Pro Lys Gly Leu Arg Lys Gly Thr Trp Thr Thr Glu
   1               5                   10                  15
   gaa gat att ctc ttg agg caa tgc att gat aag tat gga gaa ggc aaa      96
   Glu Asp Ile Leu Leu Arg Gln Cys Ile Asp Lys Tyr Gly Glu Gly Lys
               20                  25                  30
   tgg cat cga gtt cct tta aga act ggt ctc aat cgg tgc cga aag agt      144
   Trp His Arg Val Pro Leu Arg Thr Gly Leu Asn Arg Cys Arg Lys Ser
           35                  40                  45
   tgt aga ctt aga tgg ttg aat tat ttg aag cca agt att aag aga gga      192
   Cys Arg Leu Arg Trp Leu Asn Tyr Leu Lys Pro Ser Ile Lys Arg Gly
       50                  55                  60
   aaa ctc tgc tcc gat gaa gtt gat ctt gtt ctt cgc ctt cat aaa ctt      240
   Lys Leu Cys Ser Asp Glu Val Asp Leu Val Leu Arg Leu His Lys Leu
   65                  70                  75                  80
   cta gga aat agg tgg tcc ttg atc gct ggt aga ttg cct ggt cgg act      288
   Leu Gly Asn Arg Trp Ser Leu Ile Ala Gly Arg Leu Pro Gly Arg Thr
                   85                  90                  95
   gct aat gat gtc aag aat tac tgg aac act cat ttg agt aag aag cac      336
   Ala Asn Asp Val Lys Asn Tyr Trp Asn Thr His Leu Ser Lys Lys His
               100                 105                 110
   gat gaa cga tgc tgt aag acg aag atg ata aac aaa aac att act tct      384
   Asp Glu Arg Cys Cys Lys Thr Lys Met Ile Asn Lys Asn Ile Thr Ser
           115                 120                 125
   cat cct act tca tcg gcc caa aaa atc gat gtt tta aag cct cgg cct      432
   His Pro Thr Ser Ser Ala Gln Lys Ile Asp Val Leu Lys Pro Arg Pro
       130                 135                 140
   cga tcc ttc tcc gat aaa aat agt tgc aac gat gtc aat atc ttg cca      480
   Arg Ser Phe Ser Asp Lys Asn Ser Cys Asn Asp Val Asn Ile Leu Pro
   145                 150                 155                 160
   aaa gtt gac gtt gtt cct tta cat ctt gga ctc aac aac aat tat gtt      528
   Lys Val Asp Val Val Pro Leu His Leu Gly Leu Asn Asn Asn Tyr Val
                   165                 170                 175
   tgt gaa agt agt att aca tgt aac aaa gat gag caa aaa gat aag ctt      576
   Cys Glu Ser Ser Ile Thr Cys Asn Lys Asp Glu Gln Lys Asp Lys Leu
               180                 185                 190
   att aat att aat cta ttg gat gga gat aat atg tgg tgg gaa agt tta      624
   Ile Asn Ile Asn Leu Leu Asp Gly Asp Asn Met Trp Trp Glu Ser Leu
           195                 200                 205
   ctg gag gca gat gtg ttg ggt cca gaa gct acg gaa aca gca aag ggt      672
   Leu Glu Ala Asp Val Leu Gly Pro Glu Ala Thr Glu Thr Ala Lys Gly
       210                 215                 220
   gtg acc tta ccg ctt gac ttt gag caa att tgg gct cgg ttt gat gaa      720
   Val Thr Leu Pro Leu Asp Phe Glu Gln Ile Trp Ala Arg Phe Asp Glu
   225                 230                 235                 240
   gag act tta gaa ctg aat tag                                          741
   Glu Thr Leu Glu Leu Asn
                   245
<210> 44
   <211> 246
   <212> PRT
   <213> Arabidopsis thaliana
<400> 44
   Met Gly Glu Ser Pro Lys Gly Leu Arg Lys Gly Thr Trp Thr Thr Glu
   1               5                   10                  15
   Glu Asp Ile Leu Leu Arg Gln Cys Ile Asp Lys Tyr Gly Glu Gly Lys
               20                  25                  30
   Trp His Arg Val Pro Leu Arg Thr Gly Leu Asn Arg Cys Arg Lys Ser
           35                  40                  45
   Cys Arg Leu Arg Trp Leu Asn Tyr Leu Lys Pro Ser Ile Lys Arg Gly
       50                  55                  60
   Lys Leu Cys Ser Asp Glu Val Asp Leu Val Leu Arg Leu His Lys Leu
   65                  70                  75                  80
   Leu Gly Asn Arg Trp Ser Leu Ile Ala Gly Arg Leu Pro Gly Arg Thr
                   85                  90                  95
   Ala Asn Asp Val Lys Asn Tyr Trp Asn Thr His Leu Ser Lys Lys His
               100                 105                 110
   Asp Glu Arg Cys Cys Lys Thr Lys Met Ile Asn Lys Asn Ile Thr Ser
           115                 120                 125
   His Pro Thr Ser Ser Ala Gln Lys Ile Asp Val Leu Lys Pro Arg Pro
       130                 135                 140
   Arg Ser Phe Ser Asp Lys Asn Ser Cys Asn Asp Val Asn Ile Leu Pro
   145                 150                 155                 160
   Lys Val Asp Val Val Pro Leu His Leu Gly Leu Asn Asn Asn Tyr Val
                   165                 170                 175
   Cys Glu Ser Ser Ile Thr Cys Asn Lys Asp Glu Gln Lys Asp Lys Leu
               180                 185                 190
   Ile Asn Ile Asn Leu Leu Asp Gly Asp Asn Met Trp Trp Glu Ser Leu
           195                 200                 205
   Leu Glu Ala Asp Val Leu Gly Pro Glu Ala Thr Glu Thr Ala Lys Gly
       210                 215                 220
   Val Thr Leu Pro Leu Asp Phe Glu Gln Ile Trp Ala Arg Phe Asp Glu
   225                 230                 235                 240
   Glu Thr Leu Glu Leu Asn
                   245
<210> 45
   <211> 762
   <212> DNA
   <213> Arabidopsis thaliana
<220>
   <221> CDS
   <222> (1)..(630)
   <223> G2421
<400> 45
   atg gag ggt tcg tcc aaa ggg ttg agg aaa ggt gca tgg act gct gaa      48
   Met Glu Gly Ser Ser Lys Gly Leu Arg Lys Gly Ala Trp Thr Ala Glu
   1               5                   10                  15
   gaa gat agt ctc ttg agg cag tgt att ggt aag tat gga gaa ggc aaa      96
   Glu Asp Ser Leu Leu Arg Gln Cys Ile Gly Lys Tyr Gly Glu Gly Lys
               20                  25                  30
   tgg cat caa gtt cct tta aga gct ggg cta aat cgg tgc agg aaa agt     144
   Trp His Gln Val Pro Leu Arg Ala Gly Leu Asn Arg Cys Arg Lys Ser
           35                  40                  45
   tgt aga cta aga tgg tta aac tat ttg aag cca agt atc aag aga gga     192
   Cys Arg Leu Arg Trp Leu Asn Tyr Leu Lys Pro Ser Ile Lys Arg Gly
       50                  55                  60
   aaa ttt agt tct gat gaa gtt gat ctt ctt ctt cgt ctt cat aag ctt     240
   Lys Phe Ser Ser Asp Glu Val Asp Leu Leu Leu Arg Leu His Lys Leu
   65                  70                  75                  80
   cta gga aat agg tgg tcc ttg att gct ggt cga tta cct ggt cgg acc     288
   Leu Gly Asn Arg Trp Ser Leu Ile Ala Gly Arg Leu Pro Gly Arg Thr
                   85                  90                  95
   gct aat gat gtc aag aac tac tgg aac acc cat ctg agt aag aag cat     336
   Ala Asn Asp Val Lys Asn Tyr Trp Asn Thr His Leu Ser Lys Lys His
               100                 105                 110
   gaa ccg tgt tgt aaa act aag ata aaa agg ata aat att ata acc cct     384
   Glu Pro Cys Cys Lys Thr Lys Ile Lys Arg Ile Asn Ile Ile Thr Pro
           115                 120                 125
   cct aat aca ccg gcc caa aaa gtt tgt gaa aat agt atc aca tgt aac     432
   Pro Asn Thr Pro Ala Gln Lys Val Cys Glu Asn Ser Ile Thr Cys Asn
       130                 135                 140
   aaa gat gat gag aaa gat gat ttt gtg gat aat ttt atg gtt gga gat     480
   Lys Asp Asp Glu Lys Asp Asp Phe Val Asp Asn Phe Met Val Gly Asp
   145                 150                 155                 160
   aat ata tgg ttg gag cgt ttg cta gac gag ggc caa gag gta gat gtg     528
   Asn Ile Trp Leu Glu Arg Leu Leu Asp Glu Gly Gln Glu Val Asp Val
                   165                 170                 175
   ctg gtt aca gaa gcg gcg gca aca gaa aag gag ggc act ttg gcg ttt     576
   Leu Val Thr Glu Ala Ala Ala Thr Glu Lys Glu Gly Thr Leu Ala Phe
               180                 185                 190
   gac gtt gag caa ctt tgg aat ttg ttc gat gga gag act gtg atc ttt     624
   Asp Val Glu Gln Leu Trp Asn Leu Phe Asp Gly Glu Thr Val Ile Phe
           195                 200                 205
   gat tag tgtttataaa cgtttgtgtt ctcttgtttg tgaggtttct ctatttaatt      680
   Asp
   tagtatctat tttctaaatt aactaatatc ttatagtatt ttaggcaaac cttatgtttc   740
   cgtttctgtg cggccgctct ag                                            762
<210> 46
   <211> 209
   <212> PRT
   <213> Arabidopsis thaliana
<400> 46
   Met Glu Gly Ser Ser Lys Gly Leu Arg Lys Gly Ala Trp Thr Ala Glu
   1               5                   10                  15
   Glu Asp Ser Leu Leu Arg Gln Cys Ile Gly Lys Tyr Gly Glu Gly Lys
               20                  25                  30
   Trp His Gln Val Pro Leu Arg Ala Gly Leu Asn Arg Cys Arg Lys Ser
           35                  40                  45
   Cys Arg Leu Arg Trp Leu Asn Tyr Leu Lys Pro Ser Ile Lys Arg Gly
       50                  55                  60
   Lys Phe Ser Ser Asp Glu Val Asp Leu Leu Leu Arg Leu His Lys Leu
   65                  70                  75                  80
   Leu Gly Asn Arg Trp Ser Leu Ile Ala Gly Arg Leu Pro Gly Arg Thr
                   85                  90                  95
   Ala Asn Asp Val Lys Asn Tyr Trp Asn Thr His Leu Ser Lys Lys His
               100                 105                 110
   Glu Pro Cys Cys Lys Thr Lys Ile Lys Arg Ile Asn Ile Ile Thr Pro
           115                 120                 125
   Pro Asn Thr Pro Ala Gln Lys Val Cys Glu Asn Ser Ile Thr Cys Asn
       130                 135                 140
   Lys Asp Asp Glu Lys Asp Asp Phe Val Asp Asn Phe Met Val Gly Asp
   145                 150                 155                 160
   Asn Ile Trp Leu Glu Arg Leu Leu Asp Glu Gly Gln Glu Val Asp Val
                   165                 170                 175
   Leu Val Thr Glu Ala Ala Ala Thr Glu Lys Glu Gly Thr Leu Ala Phe
               180                 185                 190
   Asp Val Glu Gln Leu Trp Asn Leu Phe Asp Gly Glu Thr Val Ile Phe
           195                 200                 205
   Asp
<210> 47
   <211> 1665
   <212> DNA
   <213> Arabidopsis thaliana
<220>
   <221> CDS
   <222> (33)..(1376)
   <223> G772
<400> 47
   aaaaaccaaa accggttttt ttttttcttt tt atg ggt cgc gaa tct ctg gct        53
                                       Met Gly Arg Glu Ser Leu Ala
                                       1               5
   gtt gtg tcc tcg ccg cca tcg gcg act gcg ccc agt act gct gtg tcg       101
   Val Val Ser Ser Pro Pro Ser Ala Thr Ala Pro Ser Thr Ala Val Ser
           10                  15                  20
   gct acc tcg ctt gct cct ggc ttt cga ttt cat ccg act gat gag gaa      149
   Ala Thr Ser Leu Ala Pro Gly Phe Arg Phe His Pro Thr Asp Glu Glu
       25                  30                  35
   ctc gtg agc tat tac ttg aag agg aag gtt ctg ggt aaa cct gta cgc      197
   Leu Val Ser Tyr Tyr Leu Lys Arg Lys Val Leu Gly Lys Pro Val Arg
   40                  45                  50                  55
   ttc gat gcg att gga gag gta gat atc tac aag cat gag ccc tgg gat      245
   Phe Asp Ala Ile Gly Glu Val Asp Ile Tyr Lys His Glu Pro Trp Asp
                   60                  65                  70
   tta gca gtg ttt tcg aag ttg aaa act cgg gac caa gaa tgg tac ttc      293
   Leu Ala Val Phe Ser Lys Leu Lys Thr Arg Asp Gln Glu Trp Tyr Phe
               75                  80                  85
   ttc agt gcg tta gat aag aag tac ggt aat ggt gct agg atg aat cga      341
   Phe Ser Ala Leu Asp Lys Lys Tyr Gly Asn Gly Ala Arg Met Asn Arg
           90                  95                  100
   gca act aac aaa ggg tac tgg aaa gca act gga aaa gac aga gaa atc      389
   Ala Thr Asn Lys Gly Tyr Trp Lys Ala Thr Gly Lys Asp Arg Glu Ile
       105                 110                 115
   cgc cgg gat att cag ttg ctc ggt atg aaa aag acg ctt gtt ttc cac      437
   Arg Arg Asp Ile Gln Leu Leu Gly Met Lys Lys Thr Leu Val Phe His
   120                 125                 130                 135
   agc ggg cgt gct cca gac ggc ctt cgg act aat tgg gtc atg cac gag      485
   Ser Gly Arg Ala Pro Asp Gly Leu Arg Thr Asn Trp Val Met His Glu
                   140                 145                 150
   tat cgc ctt gtg gaa tat gaa act gaa act aac gga agc ctg ctg cag      533
   Tyr Arg Leu Val Glu Tyr Glu Thr Glu Thr Asn Gly Ser Leu Leu Gln
               155                 160                 165
   gat gca tat gtg ttg tgc aga gtg ttt cac aag aat aac att ggg cca      581
   Asp Ala Tyr Val Leu Cys Arg Val Phe His Lys Asn Asn Ile Gly Pro
           170                 175                 180
   cca agt ggg aac aga tat gcg cca ttc atg gaa gaa gaa tgg gct gat      629
   Pro Ser Gly Asn Arg Tyr Ala Pro Phe Met Glu Glu Glu Trp Ala Asp
       185                 190                 195
   ggt gga gga gct ctg att cca gga ata gac gtt agg gtc agg gta gag      677
   Gly Gly Gly Ala Leu Ile Pro Gly Ile Asp Val Arg Val Arg Val Glu
   200                 205                 210                 215
   gct cta cca caa gcc aat gga aac aac cag atg gac cag gaa atg cat      725
   Ala Leu Pro Gln Ala Asn Gly Asn Asn Gln Met Asp Gln Glu Met His
                   220                 225                 230
   tca gca agc aag gat ctc att aac atc aac gag cta ccg aga gat gct      773
   Ser Ala Ser Lys Asp Leu Ile Asn Ile Asn Glu Leu Pro Arg Asp Ala
               235                 240                 245
   act cca atg gac atc gaa cct aac caa cag aat cat cat gag agt gcc      821
   Thr Pro Met Asp Ile Glu Pro Asn Gln Gln Asn His His Glu Ser Ala
           250                 255                 260
   ttc aag cca cag gag agt aac aac cat agt ggt tat gaa gaa gat gag      869
   Phe Lys Pro Gln Glu Ser Asn Asn His Ser Gly Tyr Glu Glu Asp Glu
       265                 270                 275
   gac aca ctc aaa cgc gag cac gca gaa gaa gat gag cgt cct cct tct      917
   Asp Thr Leu Lys Arg Glu His Ala Glu Glu Asp Glu Arg Pro Pro Ser
   280                 285                 290                 295
   cta tgc att ctc aac aaa gaa gct cca cta cct ctc ctg caa tac aaa      965
   Leu Cys Ile Leu Asn Lys Glu Ala Pro Leu Pro Leu Leu Gln Tyr Lys
                   300                 305                 310
   cgt aga cgc caa aac gag tcc aac aac aac tca agc agg aac aca cag      1013
   Arg Arg Arg Gln Asn Glu Ser Asn Asn Asn Ser Ser Arg Asn Thr Gln
               315                 320                 325
   gac cat tgt tcg tcc aca ata aca acc gtc gac aat aca acc acc tta      1061
   Asp His Cys Ser Ser Thr Ile Thr Thr Val Asp Asn Thr Thr Thr Leu
           330                 335                 340
   atc tca tca tct gct gct gct gct acc aac act gcc atc tct gca ttg      1109
   Ile Ser Ser Ser Ala Ala Ala Ala Thr Asn Thr Ala Ile Ser Ala Leu
       345                 350                 355
   ctt gag ttc tca ctt atg ggt atc tcc gac aag aaa gaa aac cag cag      1157
   Leu Glu Phe Ser Leu Met Gly Ile Ser Asp Lys Lys Glu Asn Gln Gln
   360                 365                 370                 375
   aaa gag gaa act tct cct cct agt cca att gca tct cct gaa gag aag      1205
   Lys Glu Glu Thr Ser Pro Pro Ser Pro Ile Ala Ser Pro Glu Glu Lys
                   380                 385                 390
   gtt aat gat ctc cag aag gag gtt cac cag atg tct gtt gaa aga gaa      1253
   Val Asn Asp Leu Gln Lys Glu Val His Gln Met Ser Val Glu Arg Glu
               395                 400                 405
   act ttc aag ctt gag atg atg agt gca gag gct atg atc agc att ctc      1301
   Thr Phe Lys Leu Glu Met Met Ser Ala Glu Ala Met Ile Ser Ile Leu
           410                 415                 420
   cag tca aga atc gat gcg ctg cgt cag gag aac gag gaa ctt aag aag      1349
   Gln Ser Arg Ile Asp Ala Leu Arg Gln Glu Asn Glu Glu Leu Lys Lys
       425                 430                 435
   aag aac gcc agt gga caa gct agt taa accaccgcaa catctctcca            1396
   Lys Asn Ala Ser Gly Gln Ala Ser
   440                 445
   ggtgtcttct tcttcttctt cttcttcttt gcctcttagc tgtaatcttc ttaatagtat   1456
   gagctatgga tgtagcttct tcagacggat cagaaacctt atgaatctct gttgtaaaat   1516
   taggataaaa cggaacggag ccaaccaact aggtcttttt attttatcct tttttacttt   1576
   ggatgtttct gcatcttttg ggaacatttt caggctgatc cattgtcgta tattatcatc   1636
   tatctatcta gtcttttcag acaaaaaaa                                     1665
<210> 48
   <211> 447
   <212> PRT
   <213> Arabidopsis thaliana
<400> 48
   Met Gly Arg Glu Ser Leu Ala Val Val Ser Ser Pro Pro Ser Ala Thr
   1               5                   10                  15
   Ala Pro Ser Thr Ala Val Ser Ala Thr Ser Leu Ala Pro Gly Phe Arg
               20                  25                  30
   Phe His Pro Thr Asp Glu Glu Leu Val Ser Tyr Tyr Leu Lys Arg Lys
           35                  40                  45
   Val Leu Gly Lys Pro Val Arg Phe Asp Ala Ile Gly Glu Val Asp Ile
       50                  55                  60
   Tyr Lys His Glu Pro Trp Asp Leu Ala Val Phe Ser Lys Leu Lys Thr
   65                  70                  75                  80
   Arg Asp Gln Glu Trp Tyr Phe Phe Ser Ala Leu Asp Lys Lys Tyr Gly
   85 90 95
   Asn Gly Ala Arg Met Asn Arg Ala Thr Asn Lys Gly Tyr Trp Lys Ala
   100 105 110
   Thr Gly Lys Asp Arg Glu Ile Arg Arg Asp Ile Gln Leu Leu Gly Met
   115 120 125
   Lys Lys Thr Leu Val Phe His Ser Gly Arg Ala Pro Asp Gly Leu Arg
   130 135 140
   Thr Asn Trp Val Met His Glu Tyr Arg Leu Val Glu Tyr Glu Thr Glu
   145 150 155 160
   Thr Asn Gly Ser Leu Leu Gln Asp Ala Tyr Val Leu Cys Arg Val Phe
   165 170 175
   His Lys Asn Asn Ile Gly Pro Pro Ser Gly Asn Arg Tyr Ala Pro Phe
   180 185 190
   Met Glu Glu Glu Trp Ala Asp Gly Gly Gly Ala Leu Ile Pro Gly Ile
   195 200 205
   Asp Val Arg Val Arg Val Glu Ala Leu Pro Gln Ala Asn Gly Asn Asn
   210 215 220
   Gln Met Asp Gln Glu Met His Ser Ala Ser Lys Asp Leu Ile Asn Ile
   225 230 235 240
   Asn Glu Leu Pro Arg Asp Ala Thr Pro Met Asp Ile Glu Pro Asn Gln
   245 250 255
   Gln Asn His His Glu Ser Ala Phe Lys Pro Gln Glu Ser Asn Asn His
   260 265 270
   Ser Gly Tyr Glu Glu Asp Glu Asp Thr Leu Lys Arg Glu His Ala Glu
   275 280 285
   Glu Asp Glu Arg Pro Pro Ser Leu Cys Ile Leu Asn Lys Glu Ala Pro
   290 295 300
   Leu Pro Leu Leu Gln Tyr Lys Arg Arg Arg Gln Asn Glu Ser Asn Asn
   305 310 315 320
   Asn Ser Ser Arg Asn Thr Gln Asp His Cys Ser Ser Thr Ile Thr Thr
   325 330 335
   Val Asp Asn Thr Thr Thr Leu Ile Ser Ser Ser Ala Ala Ala Ala Thr
   340 345 350
   Asn Thr Ala Ile Ser Ala Leu Leu Glu Phe Ser Leu Met Gly Ile Ser
   355 360 365
   Asp Lys Lys Glu Asn Gln Gln Lys Glu Glu Thr Ser Pro Pro Ser Pro
   370 375 380
   Ile Ala Ser Pro Glu Glu Lys Val Asn Asp Leu Gln Lys Glu Val His
   385 390 395 400
   Gln Met Ser Val Glu Arg Glu Thr Phe Lys Leu Glu Met Met Ser Ala
   405 410 415
   Glu Ala Met Ile Ser Ile Leu Gln Ser Arg Ile Asp Ala Leu Arg Gln
   420 425 430
   Glu Asn Glu Glu Leu Lys Lys Lys Asn Ala Ser Gly Gln Ala Ser
   435 440 445
<210> 49
   <211> 1198
   <212> DNA
   <213> Arabidopsis thaliana
<220>
   <221> CDS
   <222> (56)..(1021)
   <223> G866
<400> 49
   aaaaaaaact tgcacatctt ctcagatctt caagtttctc ctctggtttc tcatc atg      58
                                                                Met
                                                                1
   acc gtt gat att atg cgt tta cct aag atg gaa gat caa acg gct ata      106
   Thr Val Asp Ile Met Arg Leu Pro Lys Met Glu Asp Gln Thr Ala Ile
               5                   10                  15
   caa gaa gct gca tca caa ggc tta aaa agc atg gaa cac ttg att cgt      154
   Gln Glu Ala Ala Ser Gln Gly Leu Lys Ser Met Glu His Leu Ile Arg
           20                  25                  30
   gtc ctc tct aac cgt ccc gaa gaa cgt aac gtt gat tgc tct gag atc      202
   Val Leu Ser Asn Arg Pro Glu Glu Arg Asn Val Asp Cys Ser Glu Ile
       35                  40                  45
   act gat ttc aca gtt tct aag ttc aag aaa gtt atc tct ctt ctt aac      250
   Thr Asp Phe Thr Val Ser Lys Phe Lys Lys Val Ile Ser Leu Leu Asn
   50                  55                  60                  65
   cgt tcc ggt cac gcc cgg ttt aga cgt ggt ccg gtt cat tcc cct cct      298
   Arg Ser Gly His Ala Arg Phe Arg Arg Gly Pro Val His Ser Pro Pro
                   70                  75                  80
   tcc tcc tcc gtt cct cca ccg gtg aaa gtg aca act ccg gct ccc act      346
   Ser Ser Ser Val Pro Pro Pro Val Lys Val Thr Thr Pro Ala Pro Thr
               85                  90                  95
   cag atc tct gct cca gca ccg gtt agc ttc gtt cag gca aat caa caa      394
   Gln Ile Ser Ala Pro Ala Pro Val Ser Phe Val Gln Ala Asn Gln Gln
           100                 105                 110
   agc gtg acg tta gat ttc act aga ccg agc gtt ttt ggc gct aaa acc      442
   Ser Val Thr Leu Asp Phe Thr Arg Pro Ser Val Phe Gly Ala Lys Thr
       115                 120                 125
   aag agc tcg gag gtt gtt gag ttt gct aaa gag agc ttt agc gta tct      490
   Lys Ser Ser Glu Val Val Glu Phe Ala Lys Glu Ser Phe Ser Val Ser
   130                 135                 140                 145
   tct aac tct tct ttc atg tct tct gcg atc acc ggt gat gga agt gtc      538
   Ser Asn Ser Ser Phe Met Ser Ser Ala Ile Thr Gly Asp Gly Ser Val
                   150                 155                 160
   tct aaa ggc tct tcg atc ttt ctt gct ccg gct cca gcg gtg cca gtg      586
   Ser Lys Gly Ser Ser Ile Phe Leu Ala Pro Ala Pro Ala Val Pro Val
               165                 170                 175
   act tcc tcc ggg aaa ccg ccg ctt tct ggt ctt cct tac agg aag aga      634
   Thr Ser Ser Gly Lys Pro Pro Leu Ser Gly Leu Pro Tyr Arg Lys Arg
           180                 185                 190
   tgc ttt gaa cat gac cac tct gaa ggc ttt tcc ggc aag atc tct ggc      682
   Cys Phe Glu His Asp His Ser Glu Gly Phe Ser Gly Lys Ile Ser Gly
       195                 200                 205
   tcc ggc aac ggc aag tgc cat tgc aag aaa agc cga aaa aat cgg atg      730
   Ser Gly Asn Gly Lys Cys His Cys Lys Lys Ser Arg Lys Asn Arg Met
   210                 215                 220                 225
   aag aga acc gtg aga gta ccg gcg gta agt gca aag atc gcc gat ata      778
   Lys Arg Thr Val Arg Val Pro Ala Val Ser Ala Lys Ile Ala Asp Ile
                   230                 235                 240
   cca cca gac gaa tat tca tgg aga aag tat gga caa aaa ccg atc aaa      826
   Pro Pro Asp Glu Tyr Ser Trp Arg Lys Tyr Gly Gln Lys Pro Ile Lys
               245                 250                 255
   ggc tca cca cat cca cgg ggt tat tac aag tgt agt aca ttt aga gga      874
   Gly Ser Pro His Pro Arg Gly Tyr Tyr Lys Cys Ser Thr Phe Arg Gly
           260                 265                 270
   tgt cca gcg agg aaa cac gtg gaa aga gct ttg gat gat tca acg atg      922
   Cys Pro Ala Arg Lys His Val Glu Arg Ala Leu Asp Asp Ser Thr Met
       275                 280                 285
   ttg att gtg acg tac gaa gga gag cac cgt cat cac cag tcc acg atg      970
   Leu Ile Val Thr Tyr Glu Gly Glu His Arg His His Gln Ser Thr Met
   290                 295                 300                 305
   cag gag cat gta act cct agc gtg agt ggt ttg gtg ttt ggt tcg gct     1018
   Gln Glu His Val Thr Pro Ser Val Ser Gly Leu Val Phe Gly Ser Ala
                   310                 315                 320
   tga agaattaatt agtttggtag ttttgtaata ttttgagaaa tagaggggtt         1071
   ggttttgtaa ttttttttct ataacaaaat tagttttaga ttttttttta gtagtctttt  1131
   gaatggattt taatcttact accgagaaag aaaaaattct tactacattt tcaaaaaaaa  1191
   aaaaaaa                                                            1198
<210> 50
   <211> 321
   <212> PRT
   <213> Arabidopsis thaliana
<400> 50
   Met Thr Val Asp Ile Met Arg Leu Pro Lys Met Glu Asp Gln Thr Ala
   1               5                   10                  15
   Ile Gln Glu Ala Ala Ser Gln Gly Leu Lys Ser Met Glu His Leu Ile
               20                  25                  30
   Arg Val Leu Ser Asn Arg Pro Glu Glu Arg Asn Val Asp Cys Ser Glu
           35                  40                  45
   Ile Thr Asp Phe Thr Val Ser Lys Phe Lys Lys Val Ile Ser Leu Leu
       50                  55                  60
   Asn Arg Ser Gly His Ala Arg Phe Arg Arg Gly Pro Val His Ser Pro
   65                  70                  75                  80
   Pro Ser Ser Ser Val Pro Pro Pro Val Lys Val Thr Thr Pro Ala Pro
                   85                  90                  95
   Thr Gln Ile Ser Ala Pro Ala Pro Val Ser Phe Val Gln Ala Asn Gln
               100                 105                 110
   Gln Ser Val Thr Leu Asp Phe Thr Arg Pro Ser Val Phe Gly Ala Lys
           115                 120                 125
   Thr Lys Ser Ser Glu Val Val Glu Phe Ala Lys Glu Ser Phe Ser Val
       130                 135                 140
   Ser Ser Asn Ser Ser Phe Met Ser Ser Ala Ile Thr Gly Asp Gly Ser
   145                 150                 155                 160
   Val Ser Lys Gly Ser Ser Ile Phe Leu Ala Pro Ala Pro Ala Val Pro
                   165                 170                 175
   Val Thr Ser Ser Gly Lys Pro Pro Leu Ser Gly Leu Pro Tyr Arg Lys
               180                 185                 190
   Arg Cys Phe Glu His Asp His Ser Glu Gly Phe Ser Gly Lys Ile Ser
           195                 200                 205
   Gly Ser Gly Asn Gly Lys Cys His Cys Lys Lys Ser Arg Lys Asn Arg
       210                 215                 220
   Met Lys Arg Thr Val Arg Val Pro Ala Val Ser Ala Lys Ile Ala Asp
   225                 230                 235                 240
   Ile Pro Pro Asp Glu Tyr Ser Trp Arg Lys Tyr Gly Gln Lys Pro Ile
                   245                 250                 255
   Lys Gly Ser Pro His Pro Arg Gly Tyr Tyr Lys Cys Ser Thr Phe Arg
               260                 265                 270
   Gly Cys Pro Ala Arg Lys His Val Glu Arg Ala Leu Asp Asp Ser Thr
           275                 280                 285
   Met Leu Ile Val Thr Tyr Glu Gly Glu His Arg His His Gln Ser Thr
       290                 295                 300
   Met Gln Glu His Val Thr Pro Ser Val Ser Gly Leu Val Phe Gly Ser
   305                 310                 315                 320
   Ala
<210> 51
   <211> 2310
   <212> DNA
   <213> Arabidopsis thaliana
<220>
   <221> CDS
   <222> (179)..(2065)
   <223> G941
<400> 51
   tcttcttctt cttcctcttc ctcatctcgt atctctaact tttgtcgaag ttcttttgat 60
   gaaactaggg tttattatct tctccttctt tttcccatca ccatagaaaa ggcagagacc    120
   tttttcttca tcatttttat tctccttctt cttctgctgt tcatttctcc aggttaca      178
   atg atg ttt aat gag atg gga atg tgt gga aac atg gat ttc ttc tct      226
   Met Met Phe Asn Glu Met Gly Met Cys Gly Asn Met Asp Phe Phe Ser
   1               5                   10                  15
   tct gga tca ctt ggt gaa gtt gat ttc tgt cct gtt cca caa gct gag      274
   Ser Gly Ser Leu Gly Glu Val Asp Phe Cys Pro Val Pro Gln Ala Glu
               20                  25                  30
   cct gat tcc att gtt gaa gat gac tat act gat gat gag att gat gtt      322
   Pro Asp Ser Ile Val Glu Asp Asp Tyr Thr Asp Asp Glu Ile Asp Val
           35                  40                  45
   gat gaa ttg gag agg agg atg tgg aga gac aaa atg cgg ctt aaa cgt      370
   Asp Glu Leu Glu Arg Arg Met Trp Arg Asp Lys Met Arg Leu Lys Arg
       50                  55                  60
   ctc aag gag cag gat aag ggt aaa gaa ggt gtt gat gct gct aaa cag      418
   Leu Lys Glu Gln Asp Lys Gly Lys Glu Gly Val Asp Ala Ala Lys Gln
   65                  70                  75                  80
   agg cag tct caa gag caa gct agg agg aag aaa atg tct aga gct caa      466
   Arg Gln Ser Gln Glu Gln Ala Arg Arg Lys Lys Met Ser Arg Ala Gln
                   85                  90                  95
   gat ggg atc ttg aag tat atg ttg aag atg atg gaa gtt tgt aaa gct      514
   Asp Gly Ile Leu Lys Tyr Met Leu Lys Met Met Glu Val Cys Lys Ala
               100                 105                 110
   caa ggc ttt gtt tat ggg att att ccg gag aat ggg aag cct gtg act      562
   Gln Gly Phe Val Tyr Gly Ile Ile Pro Glu Asn Gly Lys Pro Val Thr
           115                 120                 125
   ggt gct tct gat aat tta agg gag tgg tgg aaa gat aag gtt agg ttt      610
   Gly Ala Ser Asp Asn Leu Arg Glu Trp Trp Lys Asp Lys Val Arg Phe
       130                 135                 140
   gat cgt aat ggt cct gcg gct att acc aag tat caa gcg gag aat aat      658
   Asp Arg Asn Gly Pro Ala Ala Ile Thr Lys Tyr Gln Ala Glu Asn Asn
   145                 150                 155                 160
   atc ccg ggg att cat gaa ggt aat aac ccg att gga ccg act cct cat      706
   Ile Pro Gly Ile His Glu Gly Asn Asn Pro Ile Gly Pro Thr Pro His
                   165                 170                 175
   acc ttg caa gag ctt caa gac acg act ctt gga tcg ctt ttg tct gcg      754
   Thr Leu Gln Glu Leu Gln Asp Thr Thr Leu Gly Ser Leu Leu Ser Ala
               180                 185                 190
   ttg atg caa cac tgt gat cct cct cag aga cgt ttt cct ttg gag aaa      802
   Leu Met Gln His Cys Asp Pro Pro Gln Arg Arg Phe Pro Leu Glu Lys
           195                 200                 205
   gga gtt cct cct ccg tgg tgg cct aat ggg aaa gag gat tgg tgg cct      850
   Gly Val Pro Pro Pro Trp Trp Pro Asn Gly Lys Glu Asp Trp Trp Pro
       210                 215                 220
   caa ctt ggt ttg cct aaa gat caa ggt cct gca cct tac aag aag cct      898
   Gln Leu Gly Leu Pro Lys Asp Gln Gly Pro Ala Pro Tyr Lys Lys Pro
   225                 230                 235                 240
   cat gat ttg aag aag gcg tgg aaa gtc ggc gtt ttg act gcg gtt atc      946
   His Asp Leu Lys Lys Ala Trp Lys Val Gly Val Leu Thr Ala Val Ile
                   245                 250                 255
   aag cat atg ttt cct gat att gct aag atc cgt aag ctc gtg agg caa      994
   Lys His Met Phe Pro Asp Ile Ala Lys Ile Arg Lys Leu Val Arg Gln
               260                 265                 270
   tct aaa tgt ttg cag gat aag atg act gct aaa gag agt gct acc tgg     1042
   Ser Lys Cys Leu Gln Asp Lys Met Thr Ala Lys Glu Ser Ala Thr Trp
           275                 280                 285
   ctt gct att att aac caa gaa gag tcc ttg gct aga gag ctt tat ccc      1090
   Leu Ala Ile Ile Asn Gln Glu Glu Ser Leu Ala Arg Glu Leu Tyr Pro
       290                 295                 300
   gag tca tgt cca cct ctt tct ctg tct ggt gga agt tgc tcg ctt ctg      1138
   Glu Ser Cys Pro Pro Leu Ser Leu Ser Gly Gly Ser Cys Ser Leu Leu
   305                 310                 315                 320
   atg aat gat tgc agt caa tac gat gtt gaa ggt ttc gag aag gag tct      1186
   Met Asn Asp Cys Ser Gln Tyr Asp Val Glu Gly Phe Glu Lys Glu Ser
                   325                 330                 335
   cac tat gaa gtg gaa gag ctc aag cca gaa aaa gtt atg aat tct tca      1234
   His Tyr Glu Val Glu Glu Leu Lys Pro Glu Lys Val Met Asn Ser Ser
               340                 345                 350
   aac ttt ggg atg gtt gct aaa atg cat gac ttt cct gtc aaa gaa gaa      1282
   Asn Phe Gly Met Val Ala Lys Met His Asp Phe Pro Val Lys Glu Glu
           355                 360                 365
   gtc cca gca gga aac tcg gaa ttc atg aga aag aga aag cca aac aga      1330
   Val Pro Ala Gly Asn Ser Glu Phe Met Arg Lys Arg Lys Pro Asn Arg
       370                 375                 380
   gat ctg aac act att atg gac aga acc gtt ttc acc tgc gag aat ctt      1378
   Asp Leu Asn Thr Ile Met Asp Arg Thr Val Phe Thr Cys Glu Asn Leu
   385                 390                 395                 400
   ggg tgt gcg cac agc gaa atc agc cgg gga ttt ctg gat agg aat tcg      1426
   Gly Cys Ala His Ser Glu Ile Ser Arg Gly Phe Leu Asp Arg Asn Ser
                   405                 410                 415
   aga gac aac cat caa ctg gca tgt cca cat cga gac agt cgc tta ccg      1474
   Arg Asp Asn His Gln Leu Ala Cys Pro His Arg Asp Ser Arg Leu Pro
               420                 425                 430
   tat gga gca gca cca tcc agg ttt cat gtc aat gaa gtt aag cct gta      1522
   Tyr Gly Ala Ala Pro Ser Arg Phe His Val Asn Glu Val Lys Pro Val
           435                 440                 445
   gtt gga ttt cct cag cca agg cca gtg aac tca gta gcc caa cca att      1570
   Val Gly Phe Pro Gln Pro Arg Pro Val Asn Ser Val Ala Gln Pro Ile
       450                 455                 460
   gac tta acg ggt ata gtt cct gaa gat gga cag aag atg atc tca gag      1618
   Asp Leu Thr Gly Ile Val Pro Glu Asp Gly Gln Lys Met Ile Ser Glu
   465                 470                 475                 480
   ctc atg tcc atg tac gac aga aat gtc cag agc aac caa acc tct atg      1666
   Leu Met Ser Met Tyr Asp Arg Asn Val Gln Ser Asn Gln Thr Ser Met
                   485                 490                 495
   gtc atg gaa aat caa agc gtg tca ctg ctt caa ccc aca gtc cat aac      1714
   Val Met Glu Asn Gln Ser Val Ser Leu Leu Gln Pro Thr Val His Asn
               500                 505                 510
   cat caa gaa cat ctc cag ttc cca gga aac atg gtg gaa gga agt ttc      1762
   His Gln Glu His Leu Gln Phe Pro Gly Asn Met Val Glu Gly Ser Phe
           515                 520                 525
   ttt gaa gac ttg aac atc cca aac aga gca aac aac aac aac agc agc      1810
   Phe Glu Asp Leu Asn Ile Pro Asn Arg Ala Asn Asn Asn Asn Ser Ser
       530                 535                 540
   aac aat caa acg ttt ttt caa ggg aac aac aac aac aac aat gtg ttt      1858
   Asn Asn Gln Thr Phe Phe Gln Gly Asn Asn Asn Asn Asn Asn Val Phe
   545                 550                 555                 560
   aag ttc gac act gca gat cac aac aac ttt gaa gct gca cat aac aac      1906
   Lys Phe Asp Thr Ala Asp His Asn Asn Phe Glu Ala Ala His Asn Asn
                   565                 570                 575
   aac aat aac agt agc ggc aac agg ttc cag ctt gtg ttt gat tcc aca      1954
   Asn Asn Asn Ser Ser Gly Asn Arg Phe Gln Leu Val Phe Asp Ser Thr
               580                 585                 590
   ccg ttc gac atg gcg tca ttc gat tac aga gat gat atg tcg atg cca     2002
   Pro Phe Asp Met Ala Ser Phe Asp Tyr Arg Asp Asp Met Ser Met Pro
           595                 600                 605
   gga gta gta gga acg atg gat gga atg cag cag aag cag caa gat gta     2050
   Gly Val Val Gly Thr Met Asp Gly Met Gln Gln Lys Gln Gln Asp Val
       610                 615                 620
   tcc ata tgg ttc taa agtcttggta gtagatttca tcttctctta tttttatctt     2105
   Ser Ile Trp Phe
   625
   ttgtgttctt acattcactc aaccatgtaa tattttttcc tgggtctctc tgtctctatc   2165
   gcttgttatg atgtgtctgt aagagtctct aaaaactctc tgttactgtg tgtctttgtc   2225
   tcggcttggt gaatctctct gtcatcatca gcttttagtt acacacccga cttggggatg   2285
   aacgaacact aaatgtaagt tttca                                         2310
<210> 52
   <211> 628
   <212> PRT
   <213> Arabidopsis thaliana
<400> 52
   Met Met Phe Asn Glu Met Gly Met Cys Gly Asn Met Asp Phe Phe Ser
   1               5                   10                  15
   Ser Gly Ser Leu Gly Glu Val Asp Phe Cys Pro Val Pro Gln Ala Glu
               20                  25                  30
   Pro Asp Ser Ile Val Glu Asp Asp Tyr Thr Asp Asp Glu Ile Asp Val
           35                  40                  45
   Asp Glu Leu Glu Arg Arg Met Trp Arg Asp Lys Met Arg Leu Lys Arg
       50                  55                  60
   Leu Lys Glu Gln Asp Lys Gly Lys Glu Gly Val Asp Ala Ala Lys Gln
   65                  70                  75                  80
   Arg Gln Ser Gln Glu Gln Ala Arg Arg Lys Lys Met Ser Arg Ala Gln
                   85                  90                  95
   Asp Gly Ile Leu Lys Tyr Met Leu Lys Met Met Glu Val Cys Lys Ala
               100                 105                 110
   Gln Gly Phe Val Tyr Gly Ile Ile Pro Glu Asn Gly Lys Pro Val Thr
           115                 120                 125
   Gly Ala Ser Asp Asn Leu Arg Glu Trp Trp Lys Asp Lys Val Arg Phe
       130                 135                 140
   Asp Arg Asn Gly Pro Ala Ala Ile Thr Lys Tyr Gln Ala Glu Asn Asn
   145                 150                 155                 160
   Ile Pro Gly Ile His Glu Gly Asn Asn Pro Ile Gly Pro Thr Pro His
                   165                 170                 175
   Thr Leu Gln Glu Leu Gln Asp Thr Thr Leu Gly Ser Leu Leu Ser Ala
               180                 185                 190
   Leu Met Gln His Cys Asp Pro Pro Gln Arg Arg Phe Pro Leu Glu Lys
           195                 200                 205
   Gly Val Pro Pro Pro Trp Trp Pro Asn Gly Lys Glu Asp Trp Trp Pro
       210                 215                 220
   Gln Leu Gly Leu Pro Lys Asp Gln Gly Pro Ala Pro Tyr Lys Lys Pro
   225                 230                 235                 240
   His Asp Leu Lys Lys Ala Trp Lys Val Gly Val Leu Thr Ala Val Ile
                   245                 250                 255
   Lys His Met Phe Pro Asp Ile Ala Lys Ile Arg Lys Leu Val Arg Gln
               260                 265                 270
   Ser Lys Cys Leu Gln Asp Lys Met Thr Ala Lys Glu Ser Ala Thr Trp
           275                 280                 285
   Leu Ala Ile Ile Asn Gln Glu Glu Ser Leu Ala Arg Glu Leu Tyr Pro
       290                 295                 300
   Glu Ser Cys Pro Pro Leu Ser Leu Ser Gly Gly Ser Cys Ser Leu Leu
   305                 310                 315                 320
   Met Asn Asp Cys Ser Gln Tyr Asp Val Glu Gly Phe Glu Lys Glu Ser
                   325                 330                 335
   His Tyr Glu Val Glu Glu Leu Lys Pro Glu Lys Val Met Asn Ser Ser
               340                 345                 350
   Asn Phe Gly Met Val Ala Lys Met His Asp Phe Pro Val Lys Glu Glu
           355                 360                 365
   Val Pro Ala Gly Asn Ser Glu Phe Met Arg Lys Arg Lys Pro Asn Arg
       370                 375                 380
   Asp Leu Asn Thr Ile Met Asp Arg Thr Val Phe Thr Cys Glu Asn Leu
   385                 390                 395                 400
   Gly Cys Ala His Ser Glu Ile Ser Arg Gly Phe Leu Asp Arg Asn Ser
                   405                 410                 415
   Arg Asp Asn His Gln Leu Ala Cys Pro His Arg Asp Ser Arg Leu Pro
               420                 425                 430
   Tyr Gly Ala Ala Pro Ser Arg Phe His Val Asn Glu Val Lys Pro Val
           435                 440                 445
   Val Gly Phe Pro Gln Pro Arg Pro Val Asn Ser Val Ala Gln Pro Ile
       450                 455                 460
   Asp Leu Thr Gly Ile Val Pro Glu Asp Gly Gln Lys Met Ile Ser Glu
   465                 470                 475                 480
   Leu Met Ser Met Tyr Asp Arg Asn Val Gln Ser Asn Gln Thr Ser Met
   485 490 495
   Val Met Glu Asn Gln Ser Val Ser Leu Leu Gln Pro Thr Val His Asn
   500 505 510
   His Gln Glu His Leu Gln Phe Pro Gly Asn Met Val Glu Gly Ser Phe
   515 520 525
   Phe Glu Asp Leu Asn Ile Pro Asn Arg Ala Asn Asn Asn Asn Ser Ser
   530 535 540
   Asn Asn Gln Thr Phe Phe Gln Gly Asn Asn Asn Asn Asn Asn Val Phe
   545 550 555 560
   Lys Phe Asp Thr Ala Asp His Asn Asn Phe Glu Ala Ala His Asn Asn
   565 570 575
   Asn Asn Asn Ser Ser Gly Asn Arg Phe Gln Leu Val Phe Asp Ser Thr
   580 585 590
   Pro Phe Asp Met Ala Ser Phe Asp Tyr Arg Asp Asp Met Ser Met Pro
   595 600 605
   Gly Val Val Gly Thr Met Asp Gly Met Gln Gln Lys Gln Gln Asp Val
   610 615 620
   Ser Ile Trp Phe
   625
<210> 53
   <211> 1089
   <212> DNA
   <213> Arabidopsis thaliana
<220>
   <221> CDS
   <222> (1)..(1089)
   <223> G198
<400> 53
   atg gca agg tca cct tgt tgc gag aag aac gga ctc aag aaa ggg cca      48
   Met Ala Arg Ser Pro Cys Cys Glu Lys Asn Gly Leu Lys Lys Gly Pro
   1               5                   10                  15
   tgg aca tct gaa gaa gac cag aag ctt gtt gac tat atc cag aaa cat      96
   Trp Thr Ser Glu Glu Asp Gln Lys Leu Val Asp Tyr Ile Gln Lys His
               20                  25                  30
   ggt tat ggt aac tgg aga acc ctc ccc aaa aat gcc ggt acg tgt ttg     144
   Gly Tyr Gly Asn Trp Arg Thr Leu Pro Lys Asn Ala Gly Thr Cys Leu
           35                  40                  45
   caa aga tgt ggc aaa agt tgt agg tta agg tgg act aat tat ctc cga     192
   Gln Arg Cys Gly Lys Ser Cys Arg Leu Arg Trp Thr Asn Tyr Leu Arg
       50                  55                  60
   cca gat ata aaa cga gga aga ttc tct ttt gag gaa gaa gaa gcc att     240
   Pro Asp Ile Lys Arg Gly Arg Phe Ser Phe Glu Glu Glu Glu Ala Ile
   65                  70                  75                  80
   att cag ctt cat agc ttc tta gga aac aag tgg tct gcg att gcg gcg     288
   Ile Gln Leu His Ser Phe Leu Gly Asn Lys Trp Ser Ala Ile Ala Ala
                    85                  90                  95
   cgt ttg cca gga aga aca gat aat gag atc aag aac ttt tgg aac act      336
   Arg Leu Pro Gly Arg Thr Asp Asn Glu Ile Lys Asn Phe Trp Asn Thr
               100                 105                 110
   cat ata aga aag aag cta ctt aga atg ggg att gat cca gtg act cac      384
   His Ile Arg Lys Lys Leu Leu Arg Met Gly Ile Asp Pro Val Thr His
           115                 120                 125
   agt cca cga ctc gat ctc ctc gat atc tca tcc atc tta gct tca tct      432
   Ser Pro Arg Leu Asp Leu Leu Asp Ile Ser Ser Ile Leu Ala Ser Ser
       130                 135                 140
   cta tac aat tca tct tca cat cac atg aac atg tca aga ctc atg atg      480
   Leu Tyr Asn Ser Ser Ser His His Met Asn Met Ser Arg Leu Met Met
   145                 150                 155                 160
   gat act aat cgt cgt cat cag caa caa cat cca ttg gtt aac ccc gag      528
   Asp Thr Asn Arg Arg His Gln Gln Gln His Pro Leu Val Asn Pro Glu
                   165                 170                 175
   ata ctc aag ctt gcg acc tct ata ttc tct caa aac caa aac caa aac      576
   Ile Leu Lys Leu Ala Thr Ser Ile Phe Ser Gln Asn Gln Asn Gln Asn
               180                 185                 190
   cac aac caa aat caa aac caa aac caa aac ctc gtg gtg gat cat gag      624
   His Asn Gln Asn Gln Asn Gln Asn Gln Asn Leu Val Val Asp His Glu
           195                 200                 205
   aag caa aca gtt tat cat cat cat gat gtt aac caa acc gga gta aac      672
   Lys Gln Thr Val Tyr His His His Asp Val Asn Gln Thr Gly Val Asn
       210                 215                 220
   caa tac caa acc gac caa tat ttc gag aac gcg att act caa gaa ctc      720
   Gln Tyr Gln Thr Asp Gln Tyr Phe Glu Asn Ala Ile Thr Gln Glu Leu
   225                 230                 235                 240
   caa tct tcc atg cca cca ttc ccc aat gaa gct cat cag ttt aac gac      768
   Gln Ser Ser Met Pro Pro Phe Pro Asn Glu Ala His Gln Phe Asn Asp
                   245                 250                 255
   atg gat cat cac ttc aat ggt ttt gga gaa caa aat ctt gtt tca act      816
   Met Asp His His Phe Asn Gly Phe Gly Glu Gln Asn Leu Val Ser Thr
               260                 265                 270
   tct act acg tca gtc caa gat tgc tat aat ccg tca ttc aac gat tat      864
   Ser Thr Thr Ser Val Gln Asp Cys Tyr Asn Pro Ser Phe Asn Asp Tyr
           275                 280                 285
   tca agt tca aat ttt gtc tta gat cat tct tat tcg gat cag agc ttc      912
   Ser Ser Ser Asn Phe Val Leu Asp His Ser Tyr Ser Asp Gln Ser Phe
       290                 295                 300
   aac ttc gca aat tcg gtc tta aac acg cca tcc tcg agc ccg agc ccg      960
   Asn Phe Ala Asn Ser Val Leu Asn Thr Pro Ser Ser Ser Pro Ser Pro
   305                 310                 315                 320
   act acg tta aac tcg agt tac atc aat agt agc agt tgc agc act gag     1008
   Thr Thr Leu Asn Ser Ser Tyr Ile Asn Ser Ser Ser Cys Ser Thr Glu
                   325                 330                 335
   gat gaa ata gaa agc tat tgc agt aat ctc atg aag ttt gat att ccc     1056
   Asp Glu Ile Glu Ser Tyr Cys Ser Asn Leu Met Lys Phe Asp Ile Pro
               340                 345                 350
   gat ttc ttg gac gtt aat ggt ttt att ata taa                         1089
   Asp Phe Leu Asp Val Asn Gly Phe Ile Ile
           355                 360
<210> 54
   <211> 362
   <212> PRT
   <213> Arabidopsis thaliana
<400> 54
   Met Ala Arg Ser Pro Cys Cys Glu Lys Asn Gly Leu Lys Lys Gly Pro
   1 5 10 15
   Trp Thr Ser Glu Glu Asp Gln Lys Leu Val Asp Tyr Ile Gln Lys His
   20 25 30
   Gly Tyr Gly Asn Trp Arg Thr Leu Pro Lys Asn Ala Gly Thr Cys Leu
   35 40 45
   Gln Arg Cys Gly Lys Ser Cys Arg Leu Arg Trp Thr Asn Tyr Leu Arg
   50 55 60
   Pro Asp Ile Lys Arg Gly Arg Phe Ser Phe Glu Glu Glu Glu Ala Ile
   65 70 75 80
   Ile Gln Leu His Ser Phe Leu Gly Asn Lys Trp Ser Ala Ile Ala Ala
   85 90 95
   Arg Leu Pro Gly Arg Thr Asp Asn Glu Ile Lys Asn Phe Trp Asn Thr
   100 105 110
   His Ile Arg Lys Lys Leu Leu Arg Met Gly Ile Asp Pro Val Thr His
   115 120 125
   Ser Pro Arg Leu Asp Leu Leu Asp Ile Ser Ser Ile Leu Ala Ser Ser
   130 135 140
   Leu Tyr Asn Ser Ser Ser His His Met Asn Met Ser Arg Leu Met Met
   145 150 155 160
   Asp Thr Asn Arg Arg His Gln Gln Gln His Pro Leu Val Asn Pro Glu
   165 170 175
   Ile Leu Lys Leu Ala Thr Ser Ile Phe Ser Gln Asn Gln Asn Gln Asn
   180 185 190
   His Asn Gln Asn Gln Asn Gln Asn Gln Asn Leu Val Val Asp His Glu
   195 200 205
   Lys Gln Thr Val Tyr His His His Asp Val Asn Gln Thr Gly Val Asn
   210 215 220
   Gln Tyr Gln Thr Asp Gln Tyr Phe Glu Asn Ala Ile Thr Gln Glu Leu
   225 230 235 240
   Gln Ser Ser Met Pro Pro Phe Pro Asn Glu Ala His Gln Phe Asn Asp
   245 250 255
   Met Asp His His Phe Asn Gly Phe Gly Glu Gln Asn Leu Val Ser Thr
   260 265 270
   Ser Thr Thr Ser Val Gln Asp Cys Tyr Asn Pro Ser Phe Asn Asp Tyr
   275 280 285
   Ser Ser Ser Asn Phe Val Leu Asp His Ser Tyr Ser Asp Gln Ser Phe
       290                 295                 300
   Asn Phe Ala Asn Ser Val Leu Asn Thr Pro Ser Ser Ser Pro Ser Pro
   305                 310                 315                 320
   Thr Thr Leu Asn Ser Ser Tyr Ile Asn Ser Ser Ser Cys Ser Thr Glu
                   325                 330                 335
   Asp Glu Ile Glu Ser Tyr Cys Ser Asn Leu Met Lys Phe Asp Ile Pro
               340                 345                 350
   Asp Phe Leu Asp Val Asn Gly Phe Ile Ile
           355                 360

## Description

### FIELD OF THE INVENTION

This invention relates to the field of plant biology. More particularly, the present invention pertains to compositions and methods for phenotypically modifying a plant.

### BACKGROUND OF THE INVENTION

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

The present invention provides novel transcription factors useful for modifying a plant's phenotype in desirable ways, such as modifying the characteristics of a plant's seed.

WO98/07842 relates to methods of modulating seed mass and other traits in plants by producing transgenic plants comprising a recombinant expression cassette containing and ADC nucleic acid linked to a plant promoter.

### SUMMARY OF THE INVENTION

The present invention provides the use of a nucleotide sequence for producing a transgenic plant wherein said nucleotide sequence encodes the transcription factor polypeptide of SEQ ID NO:4 or a homologue thereof having at least 90% amino acid identity to SEQ ID NO:4 wherein over-expression of said polypeptide provides the trait of increased seed protein.

In another aspect, the invention provides a method for producing transgenic plant having the trait of increased seed protein, said method comprising: producing a transgenic plant having an expression vector or cassette comprising a nucleotide sequence which encodes the transcription factor polypeptide of SEQ ID NO:4 or a homologue thereof having at least 90% amino acid identity to SEQ ID NO:4 wherein over-expression of said polypeptide provides the trait of increased seed protein; and selecting a plant having said trait of increased seed protein caused by over-expression of said polypeptide.

In a further aspect, the invention provides a transgenic plant, or progeny thereof, wherein said plant comprises an expression vector or cassette for over-expression of the transcription factor polypeptide of SEQ ID NO:4 or a homologue thereof having at least 90% amino acid identity to SEQ ID NO:4, wherein over-expression of said polypeptide provides the trait of increased seed protein. , plant tissue or plant cells

The invention also provides a plant part, plant tissue or plant cells obtained from the above-defined transgenic plant, said plant part tissue or cells comprising the expression vector or cassette as defined above.

Further embodiments of the invention are set out in the accompanying claims.

The plant may be a soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, or vegetable brassicas plant.

Described herein is a cloning or expression vector comprising the isolated or recombinant polynucleotide described above and cells comprising the cloning or expression vector.

Described herein is a composition produced by incubating a polynucleotide of SEQ ID NO:4 with a nuclease, a restriction enzyme, a polymerase; a polymerase and a primer; a cloning vector, or with a cell.

Furthermore, the invention relates to a method for producing a plant having improved seed traits, as set out in the claims. The method comprises altering the expression of an isolated or recombinant polynucleotide of SEQ ID NO:3 or altering the expression or activity of a polypeptide of SEQ ID NO:4 in a plant to produce a modified plant, and selecting the modified plant for modified seed traits.

Described herein is a method of identifying a factor that is modulated by or interacts with a polypeptide encoded by SEQ ID NO:3. The method comprises expressing a polypeptide encoded by the polynucleotide in a plant; and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

In yet another aspect, described herein is a method of identifying a molecule that modulates activity or expression of a polynucleotide or polypeptide of interest. The method comprises placing the molecule in contact with a plant comprising the polynucleotide or polypeptide encoded by the polynucleotide of SEQ ID NO:3 and monitoring one or more of the expression level of the polynucleotide in the plant, the expression level of the polypeptide in the plant, and modulation of an activity of the polypeptide in the plant.

Described herein is an integrated system, computer or computer readable medium comprising one or more character strings corresponding to a polynucleotide of or to a polypeptide encoded by the polynucleotide. The integrated system, computer or computer readable medium may comprise a link between one or more sequence strings to a modified plant seed trait.

Described herein is a method for identifying a sequence similar or homologous to one or more polynucleotides of SEQ ID NO:3, or one or more polypeptides encoded by the polynucleotides. The method comprises providing a sequence database; and, querying the sequence database with one or more target sequences corresponding to the one or more polynucleotides or to the one or more polypeptides to identify one or more sequence members of the database that display sequence similarity or homology to one or more of the one or more target sequences.

The method may further comprise of linking the one or more of the polynucleotides of SEQ ID NO:3, or encoded polypeptides, to a modified plant seed characteristics phenotype.

### BRIEF DESCRIPTION OF THE DRAWINGS

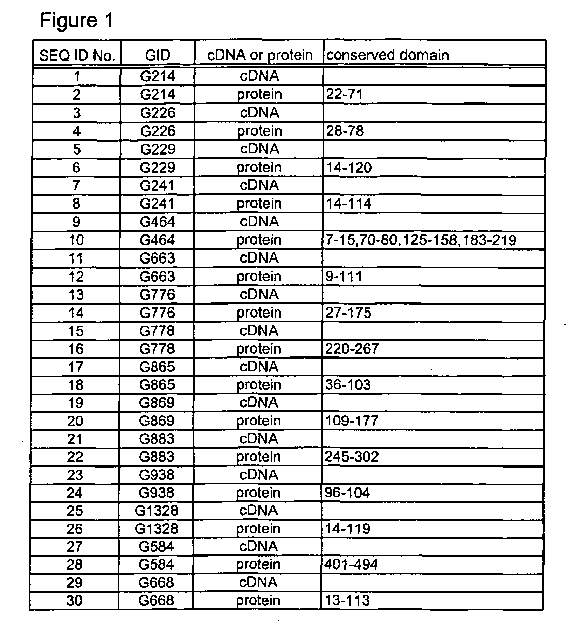

Figure 1 provides a table of exemplary polynucleotide and polypeptide sequences The table includes from left to right for each sequence: the SEQ ID No., the internal code reference number (GID), whether the sequence is a polynucleotide or polypeptide sequence, and identification of any conserved domains for the polypeptide sequences.

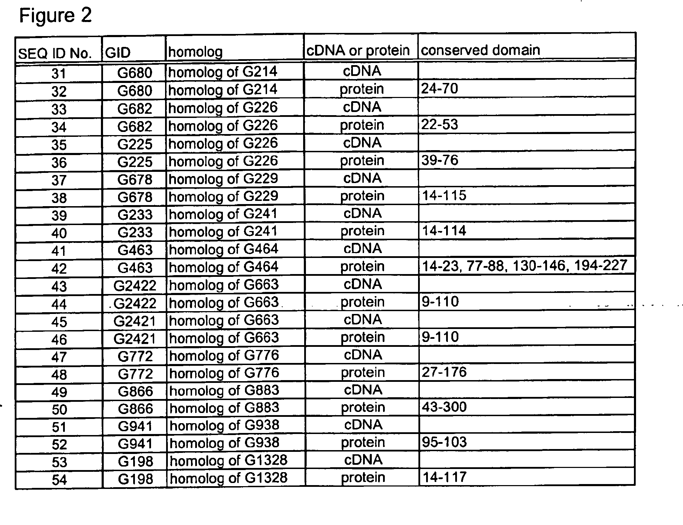

Figure 2 provides a table of exemplary sequences that are homologous to other sequences provided in the Sequence Listing and that are derived from *Arabidopsis thaliana.* The table includes from left to right: the SEQ ID No., the internal code reference number (GID), identification of the homologous sequence, whether the sequence is a polynucleotide or polypeptide sequence, and identification of any conserved domains for the polypeptide sequences.

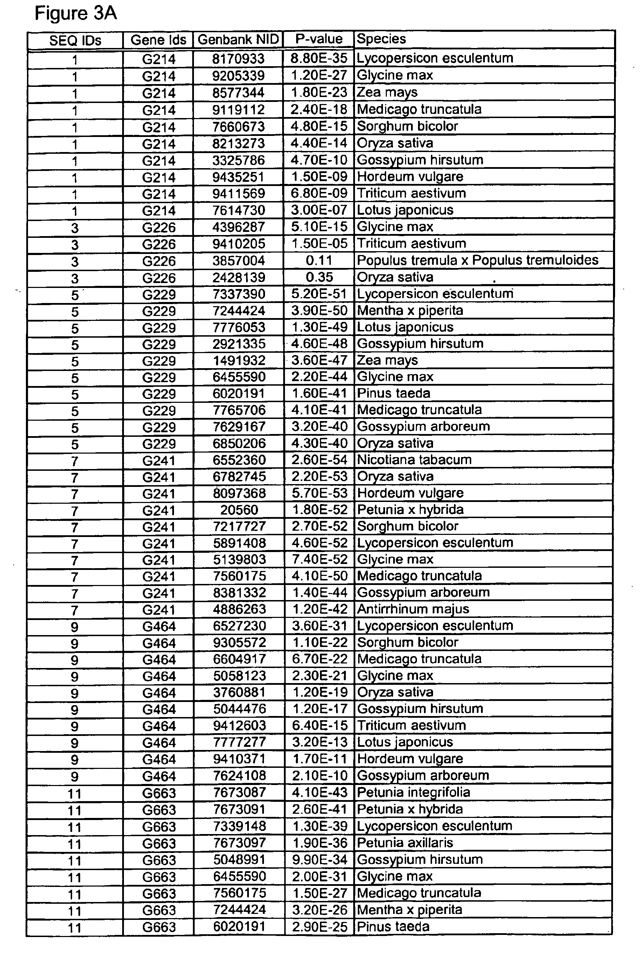

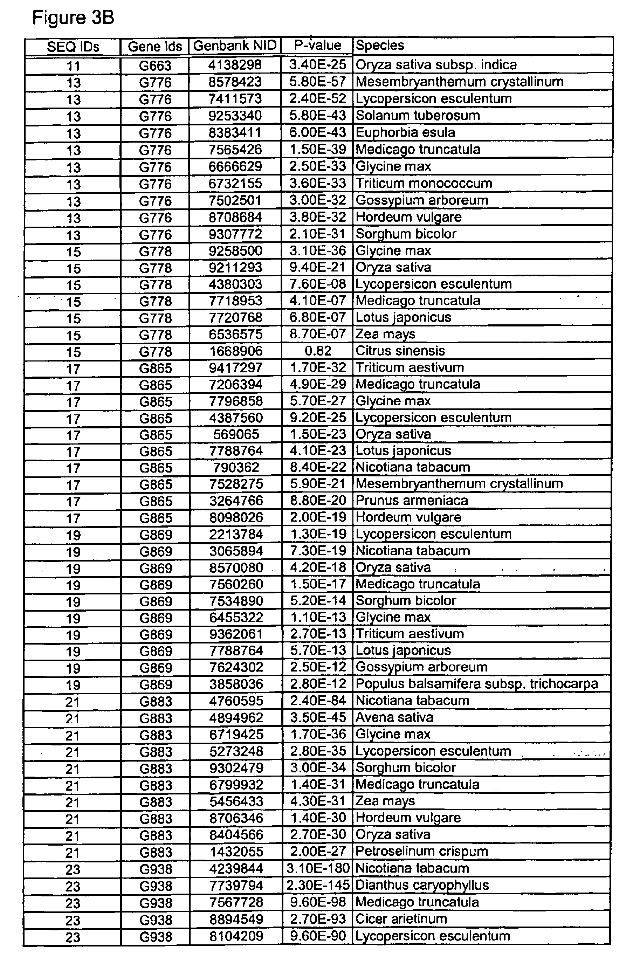

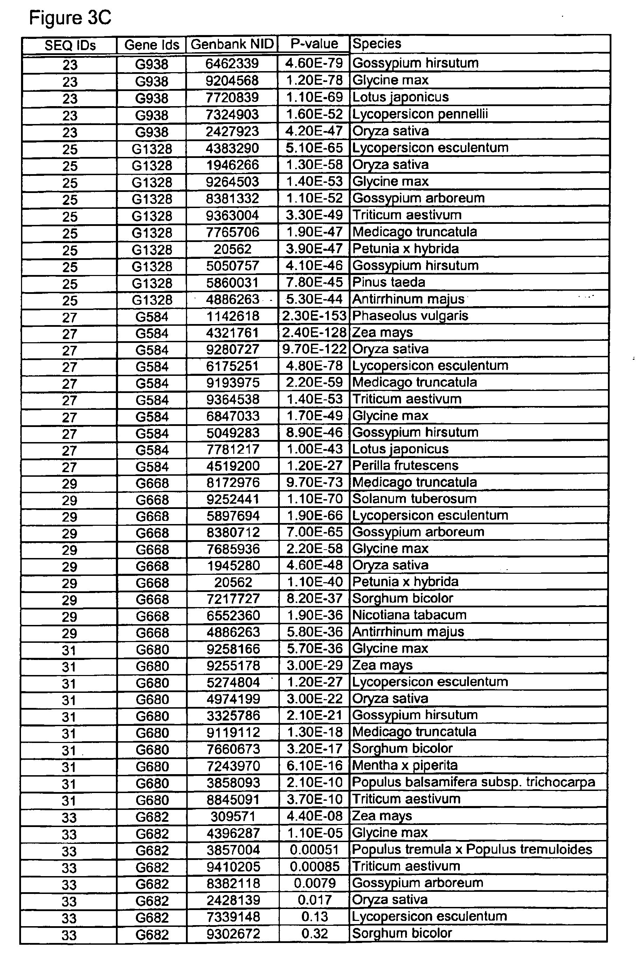

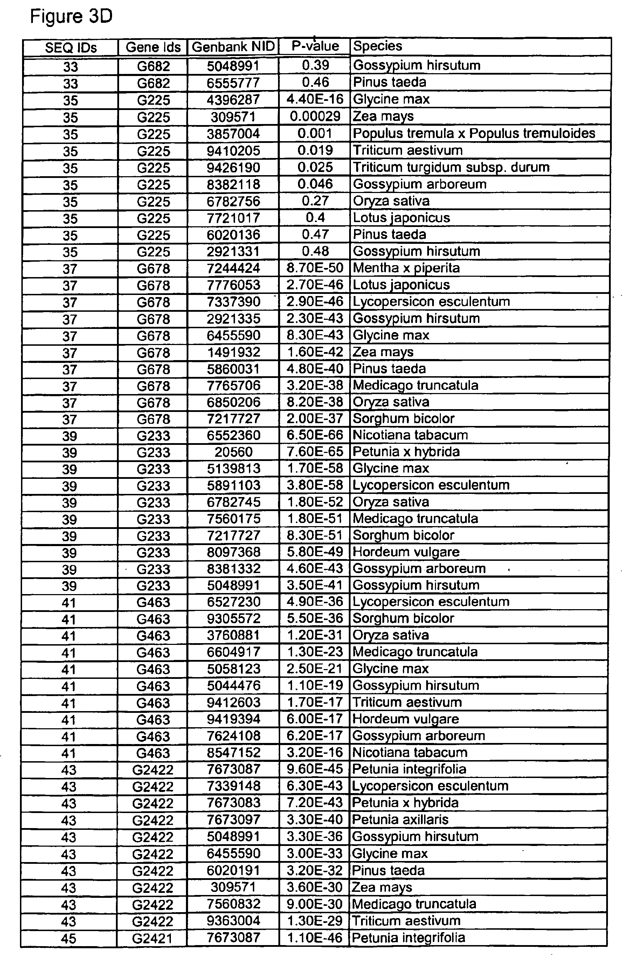

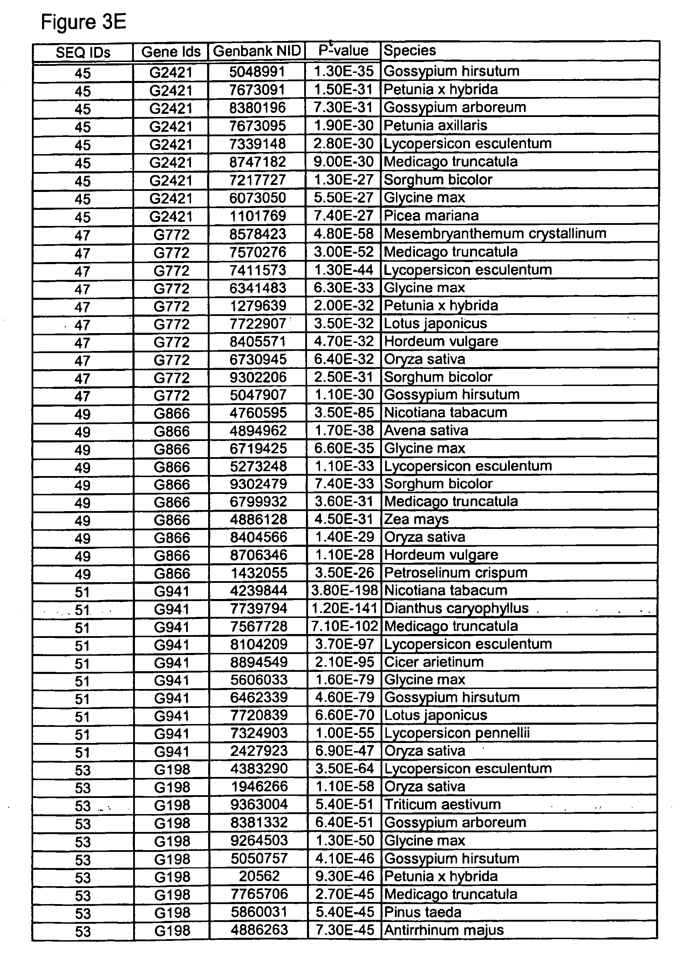

Figure 3 provides a table of exemplary sequences that are homologous to the sequences provided in Figures 1 and 2 and that are derived from plants other than *Arabidopsis thaliana.* The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

The present disclosure relates to polynucleotides and polypeptides, e.g. for modifying phenotypes of plants.

In particular the polynucleotides or polypeptides disclosed herein are useful for modiying traits associated with a plant's seed characteristics when the expression levels of the polynucleotides or expression levels or activity levels of the polypeptides are altered. Specifically, the polynucleotides and polypeptides are useful for modifying the nutritional content or composition of seeds: such as to modify the protein or oil content of seeds, to modify insoluble sugar content or composition, such as by altering the levels of arabinose, fucose, galactose, mannose, rhamnose or xylose or the like; modify prenyl lipid content or composition, such as by altering the levels of lutein, beta-carotene, xanthophyll-1, xanthophyll-2, chlorophylls A or B, or alpha-, delta- or gamma-tocopherol or the like; modify fatty acid content or composition, such as by altering the levels of the fatty acids 16:0 (palmitic acid), 16:1 (palmitoleic acid), 18:0 (stearic acid), 18:1 (oleic acid), 18:2 (linoleic acid), 20:0, 18:3 (linolenic acid), 20:1 (eicosenoic acid), 20:2 and 22:1 (erucic acid); modify wax composition or content, such as by altering the levels of C29, C31, or C33 alkanes; modify sterol composition or content, such as by altering the levels of brassicasterol, campesterol, stigmasterol, sitosterol or stigmastanol or the like, or modify glucosinolate composition or content.

Other seed characteristics that may be modified include traits relating to a seed's germination characteristics; shelf-life; drydown characteristics; size; stress responses, such as to heat, cold, salt or osmotic shock; other nutritional content, such as vitamins, minerals, or flavonoids; seedling vigor; pest resistance, or seed coat quality, resistance to pathogens, germination rate, resistance to heavy metals and toxins. Yet another desirable phenotype is a change in the overall gene expression pattern of the seed.

The polynucleotides disclosed herein plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) J. Biol. Chem. 379:633-646); the MYB transcription factor family (Martin and Paz-Ares (1997) Trends Genet. 13:67-73); the MADS domain transcription factor family (Riechmann and Meyerowitz (1997) J. Biol. Chem. 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Duboule (1994) Guidebook to the Homeobox Genes, Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides described herein, the polynucleotides and polypeptides have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed (such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES

The disclosure relates to among other things, transcription factors (TFs), and transcription factor homologue polypeptides, and isolated or recombinant polynucleotides encoding the polypeptides. These polypeptides and polynucleotides may be employed to modify a plant's seed characteristics.

Exemplary polynucleotides encoding the polypeptides were identified in the *Arabidopsis thaliana* GenBank database usmg publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides were ectopically expressed in overexpressor or knockout plants and changes in the seed characteristics of the plants were observed. Therefore, the polynucleotides and polypeptides can be employed to improve the seed characteristics of plants.

### Making polynucleotides

The polynucleotides include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 2000) ("Ausubel").

Alternatively, polynucleotides disclosed herein can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* e.g., Ausubel, Sambrook and Berger, *all supra.*

Alternatively, polynucleotides and oligonucleotides disclosed herein can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### HOMOLOGOUS SEQUENCES

Sequences homologous, i.e., that share significant sequence identity or similarity, to those provided in the sequence Listing, derived from *Arabidopsis thaliana* of from other plants of choice are also disclosed. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 31% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more % sequence identity to the listed sequences. At the nucleotide level, the sequences will typically-share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x 25x, 35x, 50x or more. The particular signal will depend on the label used in the relevant assay, e.g., a fluorescent label, a colorimetric label, a radio active label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E. coli)* and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologuepolypeptides . Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table may be used.

In addition to silent variations, other conservative variations that alter one, or a few amino acids in the encoded polypeptide, can be made without altering the function of the polypeptide, these conservative variants are, likewise, described herein.

For example, substitutions, deletions and insertions introduced into the sequences provided in the Sequence Listing are also envisioned. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser;Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES MUTATION/ FORCED EVOLUTION

In addition to generating silent or conservative substitutions as noted, above, the present disclosure optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra,* provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra.* Further details on chemical and enzymatic modifications can be found herein. These modification methods can be used to modify any given sequence, or to modify any sequence produced by the various mutation and artificial evolution modification methods noted herein.

Accordingly, modification of any given nucleic acid by mutation, evolution, chemical or enzymatic modification, or other available method is provided by this disclosure as well as the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for S. *cerevisiae* and mammals are TAA and TGA, respectively. The preferred stop codon for monocotyledonous plants is TGA, whereas insects and *E*. *coli* prefer to use TAA as the stop codon.

The polynucleotide sequences of the present disclosure can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides described herein can be combined with domains derived from other transcription factors or synthetic domains to modify the biological activity of a transcription factor. For instance, a DNA binding domain derived from a transcription factor of the invention can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences described herein incorporated into recombinant DNA (or RNA) molecules that direct expression of polypeptides in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

The present disclosure includes recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and Ausubel, *supra.* Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens,* as well as those disclosed by Herrera-Estrella et al. (1983) Nature 303: 209, Bevan (1984) Nucl Acid Res. 12: 8711-8721, Klee (1985) Bio/Technoloey 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequences (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see,* e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide described herein in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plan Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI,* Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present also relates to host cells which are transduced with vectors disclosure, and the production of polypeptides described herein (including fragments > thereof) by recombinant techniques. Host cells are genetically engineered (i.e, nucleic acids are introduced, e.g., transduced, transformed or transfected) with the vectors, which may be, for example, a cloning vector or an expression vector comprising the relevant nucleic acids herein. The vector is optionally a plasmid, a viral particle, a phage, a naked nucleic acids, *etc.* The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens,* and a portion is stably integrated into the plant genome (Horsch et al. (1984) Science 233:496-498; Fraley et al. (1983) Proc. Natl. Acad. Sci. USA 80, 4803).

The cell can include a nucleic acid which encodes a polypeptide, wherein the cells expresses a polypeptide described herein. The cell can also include vector sequences, or the like. Furthermore, cells and transgenic plants which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins can be designed with signal sequences which direct secretion of the mature polypeptides through a prokaryotic or eukaryotic cell membrane.

### Modified Amino Acids

Polypeptides may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor provided by the present disclosure can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor of the invention to modify a phenotype as desired. For example, the transcription factors can be employed to identify one or more downstream gene with which is subject to a regulatory effect of the transcription factor. In one approach, a transcription factor or transcription factor homologue of the disclosure is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, said transcription factor can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and WO 97/00271), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available e.g. using any of a number of well known robotic systems that have also been developed for solution phase chemistries useful in assay systems. These systems include automated workstations including an automated synthesis apparatus and robotic systems utilizing robotic arms. Any of the above devices are suitable for use with the present invention, e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides described herein.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

In an embodiment, This disclosure provides a method for identifying compositions that modulate the activity or expression of a polynucleotide or polypeptide described herein. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide described herein.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted supra.

Subsequences of the polynucleotides described herein, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra.*

In addition, described herein is an isolated or recombinant polypeptide including a subsequence of at least about 15 contiguous amino acids encoded by the recombinant or isolated polynucleotides of the disclosure. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides described herein are favorably employed to produce transgenic plants with various traits, or characteristics, that have been modified in a desirable manner, e.g., to improve the seed characteristics of a plant. For example, alteration of expression levels or patterns (e.g., spatial or temporal expression patterns) of one or more of the transcription factors (or transcription factor homologues) *described herein*, as compared with the levels of the same protein found in a wild type plant, can be used to modify a plant's traits. An illustrative example of trait modification, improved seed characteristics, by altering expression levels of a particular transcription factor is described further in the Examples and the Sequence Listing.

### Antisense and Cosuppression Approaches

In addition to expression of the nucleic acids of the disclosure as gene replacement or plant phenotype modification nucleic acids, the nucleic acids are also useful for sense and anti-sense suppression of expression, e.g., to down-regulate expression of a nucleic acid described herein e.g., as a further mechanism for modulating plant phenotype. That is, the nucleic acids of the disclosure, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA *ofAgrobacterium tumefaciens.* After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this disclosure can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcription machinery in a plant can be modified so as to increase transcription levels of a polynucleotide described herein (*See,* e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides of the disclosure and/or expressing the polypeptides of the disclosure can be produced by a variety of well established techniques as described above. Following construction of a vector, most typically an expression cassette, including a polynucleotide, e.g., encoding a transcription factor or transcription factor homologue, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modified trait can be any of those traits described above. Additionally, to confirm that the modified trait is due to changes in expression levels or activity of the polypeptide or polynucleotide disclosed herein can be determined by analyzing mRNA expression using Northern blots, RT-PCR or microarrays, or protein expression using immunoblots or Western blots or gel shift assays.

### INTEGRATED SYSTEMS-SEQUENCE IDENTITY

Additionally, the present disclosure provides be an integrated system, computer or computer readable medium that comprises an instruction set for determining the identity of one or more sequences in a database. In addition, the instruction set can be used to generate of identify sequences that meet any specified criteria. Furthermore, the instruction set may be used to associate or link certain functional benefits, such improved seed characteristics, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra.*

A variety of methods of determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra).* These initial neighborhood word hits act as seeds for initiating searches to find longer HSPs containing them. The world bits are then extended in hnth directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff (1989) Proc. Natl. Acad. Sci. USA 89:10915).

In addition to calculating percent sequence identity, the BLAST algorithm also performs a statistical analysis of the similarity between two sequences *(see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, the disclosure provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon™ cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon™ Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SaLI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WTTH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl *of Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2-4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation *of Agrobacterium tumefaciens* with plasmid vectors containing the gene, single *Agrobacterium* colonies were identified, propagated, and used to *transform Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg s B-5 vitamins (Sigma), 50% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 pb to each others, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization.

### EXAMPLE VII. IDENTIFICATION OF SEED CHARACTERISTICS PHENOTYPE IN OVEREXPRESSOR OR GENE KNOCKOUT PLANTS

Experiments were performed to identify those transformants or knockouts that exhibited an improved seed characteristics. For such studies, the transformants were observed by eye or biochemical assays were performed.

Among the biochemicals that were assayed were insoluble sugars, such as arabinose, fucose, galactose,.mannose, rhamnose or xylose or the like; prenyl lipids, such as lutein, beta-carotene, xanthophyll-1, xanthophyll-2, chlorophylls A or B, or alpha-, delta- or gamma-tocopherol or the like; fatty acids, such as 16:0 (palmitic acid), 16:1 (palmitoleic acid), 18:0 (stearic acid), 18:1 (oleic acid), 18:2 (linoleic acid), 20:0 , 18:3 (linolenic acid), 20:1 (eicosenoic acid), 20:2, 22:1 (erucic acid) or the like; waxes, such as by altering the levels of C29, C31, or C33 alkanes; sterols, such as brassicasterol, campesterol, stigmasterol, sitosterol or stigmastanol or the like, glucosinolates, protein or oil levels

Fatty acids were measured using two methods depending on whether the tissue was from leaves or seeds. For leaves, lipids were extracted and esterified with hot methanolic H2SO4 and partitioned into hexane from methanolic brine. For seed fatty acids, seeds were pulverized and extracted in methanol:heptane:toluene:2,2-dimethoxypropane:H2SO4 (39:34:20:5:2) for 90 minutes at 80°C. After cooling to room temperature the upper phase, containing the seed fatty acid esters, was subjected to GC analysis. Fatty acid esters from both seed and leaf tissues were analyzed with a Supelco SP-2330 column.

Glucosinolates were purified from seeds or leaves by first heating the tissue at 95°C for 10 minutes. Preheated ethanol:water (50:50) is and after heating at 95°C for a further 10 minutes, the extraction solvent is applied to a DEAE Sephadex column which had been previously equilibrated with 0.5 M pyridine acetate. Desulfoglucosinolates were eluted with 300 ul water and analyzed by reverse phase HPLC monitoring at 226 nm.

For wax alkanes, samples were extracted using an identical method as fatty acids and extracts were analyzed on a HP 5890 GC coupled with a 5973 MSD. Samples were chromatographed on a J&W DB35 mass spectrometer (J&W Scientific).

To measure prenyl lipids levels, seeds or leaves were pulverized with 1 to 2% pyrogallol as an antioxidant. For seeds, extracted samples were filtered and a portion removed for tocopherol and carotenoid/chlorophyll analysis by HPLC. The remaining material was saponified for sterol determination. For leaves, an aliquot was removed and diluted with methanol and chlorophyll A, chlorophyll B, and total carotenoids measured by spectrophotometry by determining absorbance at 665.2 nm, 652.5 nm, and 470 nm. An aliquot was removed for tocopherol and carotenoid/chlorophyll composition by HPLC using a Waters uBondapak C18 column (4.6 mm x 150 mm). The remaining methanolic solution was saponified with 10% KOH at 80°C for one hour. The samples were cooled and diluted with a mixture of methanol and water. A solution of 2% methylene chloride in hexane was mixed in and the samples were centrifuged. The aqueous methanol phase was again re-extracted 2% methylene chloride in hexane and, after centrifugation, the two upper phases were combined and evaporated. 2% methylene chloride in hexane was added to the tubes and the samples were then extracted with one ml of water. The upper phase was removed, dried, and resuspended in 400 ul of 2% methylene chloride in hexane and analyzed by gas chromatography using a 50 m DB-5ms (0.25 mm ID, 0.25 µm phase, J&W Scientific).

Insoluble sugar levels were measured by the method essentially described by Reiter et al., Plant Journal 12:335-345. This method analyzes the neutral sugar composition of cell wall polymers found in *Arabidopsis* leaves. Soluble sugars were separated from sugar polymers by extracting leaves with hot 70% ethanol. The remaining residue containing the insoluble polysaccharides was then acid hydrolyzed with allose added as an internal standard. Sugar monomers generated by the hydrolysis were then reduced to the corresponding alditols by treatment with NaBH4, then were acetylated to generate the volatile alditol acetates which were then analyzed by GC-FID. Identity of the peaks was determined by comparing the retention times of known sugars converted to the corresponding alditol acetates with the retention times of peaks from wild-type plant extracts. Alditol acetates were analyzed on a Supelco SP-2330 capillary column (30 m x 250 um x 0.2 um) using a temperature program beginning at 180° C for 2 minutes followed by an increase to 220° C in 4 minutes. After holding at 220° C for 10 minutes, the oven temperature is increased to 240° C in 2 minutes and held at this temperature for 10 minutes and brought back to room temperature.

To identify plants with alterations in total seed oil or protein content, 150mg of seeds from T2 progeny plants were subjected to analysis by Near Infrared Reflectance (NIR) using a Foss NirSystems Model 6500 with a spinning cup transport system.

Table 3 shows the phenotypes observed for particular overexpressor or knockout plants and provides the SEQ ID No., the internal reference code (GID), whether a knockout or overexpressor plant was analyzed and the observed phenotype.

**Table 3**

| GIDs | Knockout (KO) or overexpressor (OE) | Phenotype observed |
|---|---|---|
| G214 | OE | Up to 111% increase in seed lutein |
| G226 | OE | Up to 17% increase in seed protein content |
| G229 | OE | Up to 11% increase in seed oil, 13% decrease in seed protein |
| G241 | OE | Up to 13% decrease in seed oil |
| G464 | OE | Up to 12% decrease in seed oil, 25% increase in seed protein |
| G663 | OE | Up to 16% decrease in seed oil, 14% increase in seed protein |
| G776 | OE | Up to 31% alteration in some seed fatty acids, including ........ |
| G778 | OE | Up to 32% increase in seed 18:1 fatty acid |
| G865 | OE | Up to 39% increase seed protein; 23% increase in seed oil |
| G869 | OE | Up to 25% alteration in some seed fatty acids |
| G883 | OE | Up to 47% decrease in seed lutein |
| G938 | OE | Up to 115% increase in some seed fatty acids |
| G1328 | OE | Up to 43% decrease in seed lutein |
| G584 | OE | Larger seeds |
| G668 | OE | Reduced seed color |

For a particular overexpressor that shows a less beneficial seed characteristic, it may be more useful to select a plant with a decreased expression of the particular transcription factor. For a particular knockout that shows a less beneficial seed characteristic, it may be more useful to select a plant with an increased expression of the particular transcription factor.

### EXAMPLE VIII. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919).

Identified *Arabidopsis* homologous sequences are provided in Figure 2 and included in the Sequence Listing. The percent sequence identity among these sequences is as low as 47% sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 *(Arabidopsis thaliana).* These sequences were compared to sequences representing genes of SEQ IDs Nos. 1-54 on 9/26/2000 using the Washington University TBLASTX algorithm (version 2.0a19MP). For each gene of SEQ IDs Nos. 1-54, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 3.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 1: 38%-89%; SEQ ID No. 3: 50%-69%; SEQ ID No.5: 68%-93%; SEQ ID No. 7: 69%-84%; SEQ ID No. 9: 34%-60%; SEQ ID No. 11: 52%-81%; SEQ ID No. 13: 48%-81%; SEQ ID No. 15:37%-80%; SEQ ID No. 17: 48%-83%; SEQ ID No. 19: 31%-68%; SEQ ID No. 21: 47%-90%; SEQ ID No. 23: 57%-88%; SEQ ID No. 25: 39%-79%; SEQ ID No. 27: 35%-84%; SEQ ID No. 29: 54%-89%; SEQ ID No. 31: 42%-88%; SEQ ID No. 33:41%-75%; SEQ ID No. 35: 34%-67%; SEQ ID No. 37: 72%-86%; SEQ ID No. 39: 39%-84%; SEQ ID No. 41:40%-58%; SEQ ID No. 43: 44%-82%; SEQ ID No. 45: 54%-68%; SEQ ID No. 47: 48%-64%; SEQ ID No. 49: 46%-88%; SEQ ID No. 51: 52%-92%; and SEQ ID No. 53: 48%-80%.

The polynucleotides and polypeptides in the Sequence Listing and the identified homologous sequences may be stored in a computer system and have associated or linked with the sequences a function, such as that the polynucleotides and polypeptides are useful for modifying the seed characteristics of a plant.

## Claims

1. Use of a nucleotide sequence for producing a transgenic plant wherein said nucleotide sequence encodes the transcription factor polypeptide of SEQ ID NO:4 or a homologue thereof having at least 90% amino acid identity to SEQ ID NO:4 wherein over-expression of said polypeptide provides the trait of increased seed protein.

2. Use of claim 1 wherein said increase in said seed protein is at least a 10% increase in seed protein.

3. Use of any one of the preceding claims wherein said nucleotide sequence which encodes a polypeptide is operably linked to a constitutive, inducible or tissue-active promoter.

4. Use of any one of the preceding claims wherein said polypeptide comprises SEQ ID NO:4.

5. Use of any one of the preceding claims wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

6. A method for producing transgenic plant having the trait of increased seed protein, said method comprising:
producing a transgenic plant having an expression vector or cassette comprising a nucleotide sequence which encodes the transcription factor polypeptide of SEQ ID NO:4 or a homologue thereof having at least 90% amino acid identity to SEQ ID NO:4 wherein over-expression of said polypeptide provides the trait of increased seed protein; and
selecting a plant having said trait of increased seed protein caused by over-expression of said polypeptide.

7. The method of claim 6 wherein said selecting is selecting for the trait of at least a 10% increase in said seed protein.

8. The method of claim 6 or 7 wherein said nucleotide sequence which encodes a polypeptide is operably linked to a constitutive, inducible or tissue-active promoter.

9. The method of any one of claims 6 to 8, wherein said polypeptide comprises SEQ ID NO:4.

10. The method of any one of claims 6 to 9 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

11. A transgenic plant, or progeny thereof, wherein said plant comprises an expression vector or cassette encoding the transcription factor polypeptide of SEQ 10 NO:4 or a homologue thereof having at least 90% amino acid identity to SEQ ID NO:4, wherein over-expression of said polypeptide provides the trait of increased seed protein.

12. The transgenic plant of claim 11 wherein said polypeptide comprises SEQ ID NO:4.

13. The transgenic plant of claim 11 or 12 wherein said plant is soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.

14. A seed of the transgenic plant of claim 11, 12 or 13 which contains an expression vector or cassette encoding the transcription factor polypeptide of SEQ ID NO:4 or a homologue thereof having at least 90% amino acid identity to SEQ ID NO:4 wherein over-expression of said polypeptide provides the trait of increased seed protein.

15. A plant part, plant tissue or plant cells obtained from the transgenic plant of claim 11, 12 or 13, said plant part, tissue or cells comprising the expression vector or cassette as defined in claim 11.

## Patentansprüche

1. Verwendung einer Nucleotidsequenz zur Herstellung einer transgenen Pflanze, wobei die Nucleotidsequenz für das Transkriptionsfaktorpolypeptid der Seq.-ID Nr. 4 oder ein Homolog davon mit zumindest 90 % Aminosäureidentität mit Seq.-ID Nr. 4 kodiert, wobei eine Überexpression dieses Polypeptids das Merkmal erhöhten Samenproteingehalts bereitstellt.

2. Verwendung nach Anspruch 1, wobei die Erhöhung des Samenproteingehalts eine zumindest 10%ige Erhöhung des Samenproteingehalts ist.

3. Verwendung nach einem der vorangegangenen Ansprüche, wobei die Nucleotidsequenz, die für ein Polypeptid kodiert, operabel an einen konstitutiven, induzierbaren oder gewebeaktiven Promotor gebunden ist.

4. Verwendung nach einem der vorangegangenen Ansprüche, wobei das Polypeptid Seq.-ID Nr. 4 umfasst.

5. Verwendung nach einem der vorangegangenen Ansprüche, wobei die Pflanze Sojabohne, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Weintraube, Honigmelone, Kopfsalat, Mango, Melone, Zwiebel, Papaya, Erbse, Paprika, Ananas, Spinat, Kürbis, Zuckermais, Tabak, Tomate, Wassermelone, ein Obstrosengewächs oder Gemüsekohl ist.

6. Verfahren zur Herstellung einer transgenen Pflanze mit dem Merkmal erhöhten Samenproteingehalts, wobei das Verfahren Folgendes umfasst:
Herstellen einer transgenen Pflanze mit einem Expressionsvektor oder einer Expressionskassette, die eine Nucleotidsequenz umfasst, die für das Transkriptionsfaktorpolypeptid der Seq.-ID Nr. 4 oder ein Homolog davon mit zumindest 90 % Aminosäureidentität mit Seq.-ID Nr. 4 kodiert, wobei eine Überexpression des Polypeptids das Merkmal erhöhten Samenproteingehalts bereitstellt; und
Selektieren eine Pflanze mit dem Merkmal erhöhten Samenproteingehalts, das durch Überexpression des Polypeptids bewirkt wurde.

7. Verfahren nach Anspruch 6, wobei das Selektieren das Selektieren bezüglich des Merkmals einer zumindest 10%igen Steigerung des Samenproteingehalts ist.

8. Verfahren nach Anspruch 6 oder 7, wobei die Nucleotidsequenz, die für ein Polypeptid kodiert, operabel an einen konstitutiven, induzierbaren oder gewebeaktiven Promotor gebunden ist.

9. Verfahren nach einem der Ansprüche 6 bis 8, wobei das Polypeptid die Seq.-ID Nr. 4 umfasst.

10. Verfahren nach einem der Ansprüche 6 bis 9, wobei die Pflanze Sojabohne, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Weintraube, Honigmelone, Kopfsalat, Mango, Melone, Zwiebel, Papaya, Erbse, Paprika, Ananas, Spinat, Kürbis, Zuckermais, Tabak, Tomate, Wassermelone, ein Obstrosengewächs oder Gemüsekohl ist.

11. Transgene Pflanze oder Nachkommenschaft davon, wobei die Pflanze einen Expressionsvektor oder eine Expressionskassette umfasst, der/die für das Transkriptionsfaktorpolypeptid der Seq.-ID Nr. 4 oder ein Homolog davon mit zumindest 90 % Aminosäureidentität mit Seq.-ID Nr. 4 kodiert, wobei eine Überexpression des Polypeptids das Merkmal erhöhten Samenproteingehalts bereitstellt.

12. Transgene Pflanze nach Anspruch 11, wobei das Polypeptid Seq.-ID Nr. 4 umfasst.

13. Transgene Pflanze nach Anspruch 11 oder 12, wobei die Pflanze Sojabohne, Weizen, Mais, Kartoffel, Baumwolle, Reis, Raps, Sonnenblume, Luzerne, Zuckerrohr, Rasen, Banane, Brombeere, Heidelbeere, Erdbeere, Himbeere, Kantalupe, Karotte, Blumenkohl, Kaffee, Gurke, Aubergine, Weintraube, Honigmelone, Kopfsalat, Mango, Melone, Zwiebel, Papaya, Erbse, Paprika, Ananas, Spinat, Kürbis, Zuckermais, Tabak, Tomate, Wassermelone, ein Obstrosengewächs oder Gemüsekohl ist.

14. Samen einer transgenen Pflanze nach Anspruch 11, 12 oder 13, der einen Expressionsvektor oder eine Expressionskassette enthält, der/die für das Transkriptionsfaktorpolypeptid der Seq.-ID Nr. 4 oder ein Homolog davon mit zumindest 90 % Aminosäureidentität mit Seq.-ID Nr. 4 kodiert, wobei eine Überexpression des Polypeptids das Merkmal erhöhten Samenproteingehalts bereitstellt.

15. Pflanzenteil, Pflanzengewebe oder Pflanzenzellen, die von einer transgenen Pflanze nach Anspruch 11, 12 oder 13 erhalten wurden, wobei der Teil, das Gewebe oder die Zellen der Pflanze einen Expressionsvektor oder eine Expressionskassette nach Anspruch 11 umfassen.

## Revendications

1. Utilisation d'une séquence de nucléotides pour la production d'une plante transgénique, où ladite séquence de nucléotides code pour le polypeptide facteur de transcription de SEQ ID NO: 4 ou un homologue de celui-ci présentant au moins 90 % d'identité d'acides aminés avec SEQ ID NO: 4, où une surexpression dudit polypeptide entraîne la caractéristique d'augmentation des protéines de graines.

2. Utilisation selon la revendication 1, dans laquelle ladite augmentation desdites protéines de graines est une augmentation des protéines de graines d'au moins 10 %.

3. Utilisation selon l'une quelconque des revendications précédentes, dans laquelle ladite séquence de nucléotides qui code pour un polypeptide est en liaison fonctionnelle avec un promoteur constitutif, inductible ou actif dans un tissu.

4. Utilisation selon l'une quelconque des revendications précédentes, dans laquelle ledit polypeptide comprend SEQ ID NO: 4.

5. Utilisation selon l'une quelconque des revendications précédentes, dans laquelle ladite plante est le soja, le blé, le maïs, la pomme de terre, le coton, le riz, le colza, le tournesol, la luzerne, la canne à sucre, le gazon, la banane, la mûre, la myrtille, la fraise, la framboise, le melon cantaloup, la carotte, le chou-fleur, le café, le concombre, l'aubergine, le raisin, le melon miel, la laitue, la mangue, le melon, l'oignon, la papaye, le pois, le poivre, l'ananas, l'épinard, la courge, le maïs sucré, le tabac, la tomate, la pastèque, les fruits rouges, et des légumes du genre Brassica.

6. Procédé de production d'une plante transgénique présentant la caractéristique d'augmentation des protéines de graines, ledit procédé consistant à:
produire une plante transgénique comportant un vecteur ou une cassette d'expression comprenant une séquence de nucléotides qui code pour le polypeptide facteur de transcription de SEQ ID NO: 4 ou un homologue de celui-ci présentant au moins 90 % d'identité d'acides aminés avec SEQ ID NO: 4, où une surexpression dudit polypeptide entraîne la caractéristique d'augmentation des protéines de graines; et
sélectionner une plante présentant ladite caractéristique d'augmentation des protéines de graines provoquée par une surexpression dudit polypeptide.

7. Procédé selon la revendication 6, dans lequel ladite sélection permet de sélectionner la caractéristique d'augmentation desdites protéines de graines d'au moins 10 %.

8. Procédé selon la revendication 6 ou 7, dans lequel ladite séquence de nucléotides qui code pour un polypeptide est en liaison fonctionnelle avec un promoteur constitutif, inductible ou actif dans un tissu.

9. Procédé selon l'une quelconque des revendications 6 à 8, dans lequel ledit polypeptide comprend SEQ ID NO: 4.

10. Procédé selon l'une quelconque des revendications 6 à 9, dans lequel ladite plante est le soja, le blé, le maïs, la pomme de terre, le coton, le riz, le colza, le tournesol, la luzerne, la canne à sucre, le gazon, la banane, la mûre, la myrtille, la fraise, la framboise, le melon cantaloup, la carotte, le chou-fleur, le café, le concombre, l'aubergine, le raisin, le melon miel, la laitue, la mangue, le melon, l'oignon, la papaye, le pois, le poivre, l'ananas, l'épinard, la courge, le maïs sucré, le tabac, la tomate, la pastèque, les fruits rouges, et des légumes du genre Brassica.

11. Plante transgénique, ou descendance de celle-ci, où ladite plante comprend un vecteur ou une cassette d'expression codant pour le polypeptide facteur de transcription de SEQ ID NO: 4 ou un homologue de celui-ci présentant au moins 90 % d'identité d'acides aminés avec SEQ ID NO: 4, où une surexpression dudit polypeptide entraîne la caractéristique d'augmentation des protéines de graines.

12. Plante transgénique selon la revendication 11, où ledit polypeptide comprend SEQ ID NO: 4.

13. Plante transgénique selon la revendication 11 ou 12, où ladite plante est le soja, le blé, le maïs, la pomme de terre, le coton, le riz, le colza, le tournesol, la luzerne, la canne à sucre, le gazon, la banane, la mûre, la myrtille, la fraise, la framboise, le melon cantaloup, la carotte, le chou-fleur, le café, le concombre, l'aubergine, le raisin, le melon miel, la laitue, la mangue, le melon, l'oignon, la papaye, le pois, le poivre, l'ananas, l'épinard, la courge, le maïs sucré, le tabac, la tomate, la pastèque, les fruits rouges, et des légumes du genre Brassica.

14. Graine de la plante transgénique selon la revendication 11, 12 ou 13, qui contient un vecteur ou une cassette d'expression codant pour le polypeptide facteur de transcription de SEQ ID NO: 4 ou un homologue de celui-ci présentant au moins 90 % d'identité d'acides aminés avec SEQ ID NO: 4, où une surexpression dudit polypeptide entraîne la caractéristique d'augmentation des protéines de graines.

15. Partie de plante, tissu végétal ou cellules végétales obtenu(es) à partir de la plante transgénique selon la revendication 11, 12 ou 13, ladite partie de plante, tissu végétal ou cellules végétales comprenant le vecteur ou la cassette d'expression tel/telle que défini((e) dans la revendication 11.
